(19) **Europäisches Patentamt / European Patent Office / Office européen des brevets**

(11) **EP 3 735 580 B1**

(12) # EUROPÄISCHE PATENTSCHRIFT

(45) Veröffentlichungstag und Bekanntmachung des Hinweises auf die Patenterteilung:
**27.04.2022 Patentblatt 2022/17**

(21) Anmeldenummer: **19700107.6**

(22) Anmeldetag: **07.01.2019**

(51) Internationale Patentklassifikation (IPC):
**G01N 25/18** (2006.01)  **G01F 1/688** (2006.01)
**G01N 33/497** (2006.01)

(52) Gemeinsame Patentklassifikation (CPC):
**G01N 25/18; G01N 33/497**

(86) Internationale Anmeldenummer:
**PCT/EP2019/050261**

(87) Internationale Veröffentlichungsnummer:
**WO 2019/135002 (11.07.2019 Gazette 2019/28)**

(54) **AUSWERTEANORDNUNG FÜR EINEN THERMISCHEN GASSENSOR, VERFAHREN UND COMPUTERPROGRAMME**

EVALUATION ARRANGEMENT FOR A THERMAL GAS SENSOR, METHOD AND COMPUTER PROGRAM PRODUCT

SYSTÈME D'ÉVALUATION POUR UN CAPTEUR DE GAZ THERMIQUE, PROCÉDÉ ET PROGRAMMES INFORMATIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.01.2018 EP 18150496**

(43) Veröffentlichungstag der Anmeldung:
**11.11.2020 Patentblatt 2020/46**

(60) Teilanmeldung:
**21190705.0 / 3 926 335**

(73) Patentinhaber:
• **Hahn-Schickard-Gesellschaft für angewandte Forschung e.V.**
  **78052 Villingen-Schwenningen (DE)**
• **GS Elektromedizinische Geräte G. Stemple GmbH**
  **86916 Kaufering (DE)**

(72) Erfinder:
• **HEDRICH, Frank**
  **78052 Villingen-Schwenningen (DE)**
• **EHRBRECHT, Bernd**
  **78089 Unterkirnach (DE)**
• **KATTINGER, Gerhard**
  **78112 St. Georgen (DE)**
• **KLICHE, Kurt**
  **73240 Wendlingen (DE)**

(74) Vertreter: **Burger, Markus**
  **Schoppe, Zimmermann, Stöckeler Zinkler, Schenk & Partner mbB Patentanwälte**
  **Radlkoferstraße 2**
  **81373 München (DE)**

(56) Entgegenhaltungen:
**EP-A1- 2 175 246    EP-A1- 2 348 292**
**EP-A1- 2 869 041    EP-A1- 2 887 057**
**EP-B1- 1 840 535    WO-A1-2019/034570**
**DE-A1-102011 075 519    DE-A1-102013 102 398**
**US-A1- 2008 044 939**

• **BADARLIS ANASTASIOS ET AL: "Measurement of Gas Thermal Properties Using the Parametric Reduced-Order Modeling Approach", IEEE SENSORS JOURNAL, IEEE SERVICE CENTER, NEW YORK, NY, US, Bd. 16, Nr. 12, 1. Juni 2016 (2016-06-01), Seiten 4704-4714, XP011610625, ISSN: 1530-437X, DOI: 10.1109/JSEN.2016.2558820 [gefunden am 2016-05-17]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- SANTUCCI A ET AL: "Data-acquisition system for measurement of thermal diffusivity and propagation properties of thermal waves by a non-steady-state method", REVIEW OF SCIENTIFIC INSTRUMENTS, AIP, MELVILLE, NY, US, Bd. 57, Nr. 8, 1. August 1986 (1986-08-01) , Seiten 1627-1632, XP001327224, ISSN: 0034-6748
- KURT KLICHE ET AL: "Sensor for Thermal Gas Analysis Based on Micromachined Silicon-Microwires", IEEE SENSORS JOURNAL, IEEE SERVICE CENTER, NEW YORK, NY, US, Bd. 13, Nr. 7, 1. Juli 2013 (2013-07-01), Seiten 2626-2635, XP011513069, ISSN: 1530-437X, DOI: 10.1109/JSEN.2013.2252008
- BILLAT S ET AL: "Convection-based micromachined inclinometer using SOI technology", PROCEEDINGS OF THE IEEE 14TH. ANNUAL INTERNATIONAL CONFERENCE ON MICRO ELECTRO MECHANICAL SYSTEMS. MEMS 2001. INTERLAKEN, SWITZERLAND, JAN. 21 - 25, 2001; [IEEE INTERNATIONAL MICRO ELECTRO MECHANICAL SYSTEMS CONFERENCE], NEW YORK, NY : IEEE, US, 25 January 2001 (2001-01-25), pages 159-161, XP032403321, DOI: 10.1109/MEMSYS.2001.906504 ISBN: 978-0-7803-5998-7
- KLICHE K ET AL: "Sensor for gas analysis based on thermal conductivity, specific heat capacity and thermal diffusivity", IEEE 24TH INTERNATIONAL CONFERENCE ON MICRO ELECTRO MECHANICAL SYSTEMS (MEMS 2011), IEEE, US, 23 January 2011 (2011-01-23), pages 1189-1192, XP031982631, DOI: 10.1109/MEMSYS.2011.5734644 ISBN: 978-1-4244-9632-7

**Beschreibung**

**Technisches Gebiet**

Ausführungsbeispiele gemäß der Erfindung beziehen sich auf Auswerteanordnungen für einen thermischen Gassensor, Verfahren und Computerprogramme.

**Hintergrund der Erfindung**

Gase können derzeit mit unterschiedlichsten Sensoren auf ihre Eigenschaften hin analysiert werden. Am Markt existieren heute verschiedene Systeme zur Patientenbeatmung. Diese werden unterschieden nach Einsatz im klinischen und im Home Care-Bereich (z. B. Systeme der Firmen Heinen+Löwenstein, Dräger und Stephan Medizintechnik). Die Systeme dieser Anbieter beinhalten nur in ihren Top-Varianten alle notwendigen Messeinrichtungen zur Bestimmung von Druck, Atemfluss und Atemgasanalyse. Dazu müssen mehrere Geräte kombiniert werden, die überwiegend patientenfern messen.

In Anbetracht dessen besteht ein Bedarf nach einem Konzept, das einen besseren Kompromiss zwischen einer Reduzierung eines Bauraums und einer Reduzierung eines Systemgewichts eines Gas-Mess-Systems ermöglicht und eine genaue Strömungsmessung sowie eine schnelle Gasanalyse bereitstellt.

Diese Aufgabe wird durch die unabhängigen Patentansprüche gelöst.

Erfindungsgemäße Weiterbildungen sind in den Unteransprüchen definiert.

**Zusammenfassung der Erfindung**

Ein Ausführungsbeispiel betrifft eine Auswerteanordnung für einen thermischen Gassensor mit zumindest einem Heizer (z. B. einem Heizelement) und zumindest einem Detektor (z. B. eine Thermokettenstruktur bzw. "Thermopile-Struktur", temperaturveränderliche Widerstände oder Thermistoren). Die Auswerteanordnung ist z. B. ausgelegt, um eine Information über eine Amplitude eines Detektorsignals eines ersten Detektors (z. B. D1.Uss) und eine Information über eine erste Phasendifferenz zwischen einem Heizer-Signal und dem Detektorsignal des ersten Detektors (beispielsweise (D1-Hz).phi) zu erhalten. Die Auswerteanordnung kann ferner ausgelegt sein, um als Zwischengröße abhängig von der Information über die Amplituden des Detektorsignals (z. B. D1.Uss und/oder D2.Uss) und abhängig von der Information über die erste Phasendifferenz ein Kombinationssignal zu bilden, das Amplitudeninformationen und Phaseninformationen zusammenfassen kann. Die Auswerteanordnung ist des Weiteren ausgelegt, um z. B. eine Information über eine Gaskonzentration oder eine Information über eine Temperaturleitfähigkeit eines Fluids (beispielsweise eines Gases oder Gasgemisches) basierend auf dem Kombinationssignal zu bestimmen (beispielsweise ohne im weiteren Fortgang der Berechnungen, die in das Kombinationssignal einfließenden Einzelinformationen noch einmal separat zu betrachten).

Gemäß einem Ausführungsbeispiel kann der Heizer und der zumindest eine Detektor zyklisch getauscht werden, um evtl. (Gleichlauf-)Fehler zu minimieren. In anderen Worten kann zu einem ersten Zeitpunkt der Detektor als Heizer wirken und der Heizer als Detektor und zu einem zweiten Zeitpunkt kann der Detektor als Detektor dienen und der Heizer als Heizer.

Gemäß einem Ausführungsbeispiel umfasst die Auswerteanordnung zwei Detektoren, wobei diese beiden Detektoren einen gleichen Abstand zu dem Heizer aufweisen. In diesem Fall kann in das Kombinationssignal beispielsweise eine Summe der Information über die Amplituden der beiden Detektorsignale (z. B. D1.Uss und D2.Uss) von den beiden Detektoren und eine Summe der Information über die beiden Phasendifferenzen ((D1-Hz).phi und (D2-Hz).phi) der beiden Detektoren einfließen.

Ein Ausführungsbeispiel betrifft eine Auswerteanordnung für einen thermischen Gassensor mit zumindest einem Heizer (z. B. einem Heizelement) und zwei in unterschiedlichen Abständen von dem Heizer angeordneten Detektoren (z. B. eine erste Thermoelement-Struktur und eine zweite Thermoelement-Struktur oder temperaturveränderliche Widerstände oder Thermistoren), wobei die Auswerteanordnung ausgelegt ist, um eine Information über eine Amplitude eines Detektorsignals eines ersten Detektors (z. B. D1.Uss), eine Information über eine Amplitude eines Detektorsignals eines zweiten Detektors (z. B. D2.Uss), eine Information über eine erste Phasendifferenz zwischen einem Heizer-Signal und dem Detektorsignal des ersten Detektors (beispielsweise (D1-Hz).phi), und eine Information über eine zweite Phasendifferenz zwischen dem Heizer-Signal und dem Detektorsignal des zweiten Detektors (beispielsweise (D2-Hz).phi) zu erhalten. Die Auswerteanordnung kann ausgelegt sein, um als Zwischengröße abhängig von der Information über die Amplituden der Detektorsignale (z. B. D1.Uss des ersten Detektors und D2.Uss des zweiten Detektors) und abhängig von der Information über die erste Phasendifferenz und abhängig von der Information über die zweite Phasendifferenz ein Kombinationssignal zu bilden, das Amplitudeninformationen und Phaseninformationen zusammenfassen kann. Die Auswerteanordnung ist des Weiteren ausgelegt, um eine Information über eine Gaskonzentration oder eine Information über eine Temperaturleitfähigkeit eines Fluids (beispielsweise eines Gases oder Gasgemisches) basierend auf dem Kombinationssignal zu bestimmen (beispielsweise ohne im weiteren Fortgang der Berechnungen, die in das Kombinationssignal einfließenden Einzelinformationen noch einmal separat zu betrachten).

Gemäß einem Ausführungsbeispiel kann in dem thermischen Gassensor zwischen dem zumindest einem Heizer und den in unterschiedlichen Abständen oder in gleichen Abständen (z. B. symmetrisch) von dem Heizer angeordneten Detektoren Gas angeordnet sein, das mit der Auswerteanordnung in Zusammenarbeit mit dem thermischen Gassensor analysiert werden kann. Hierfür wird beispielsweise von dem zumindest einen Heizer Wärme zu dem ersten Detektor und dem zweiten Detektor über das dazwischen befindliche Gas bzw. Gasgemisch transportiert. Dabei kann ein von dem ersten Detektor detektiertes Detektorsignal bzw. ein von dem zweiten Detektor detektiertes Detektorsignal die von dem Heizer zu dem jeweiligen Detektor transportierte Wärme anzeigen. Variiert eine Heizsignalamplitude (z. B. Heizer-Amplitude) des zumindest einen Heizers (z. B. periodische Anregung des Heizers), so kann auch von den zwei Detektoren eine zum Heizer korrespondierende variierende Amplitude detektiert werden. Das Detektorsignal des ersten Detektors bzw. des zweiten Detektors kann an die Auswerteanordnung gesendet werden. Somit kann die Auswerteanordnung aus dem Detektorsignal des ersten Detektors und aus dem Detektorsignal des zweiten Detektors die jeweilige Informationen über die Amplitude erhalten sowie Informationen über die erste Phasendifferenz zwischen dem Heizer-Signal und dem Detektorsignal des ersten Detektors und die Information über die zweite Phasendifferenz zwischen dem Heizer-Signal und dem Detektorsignal des zweiten Detektors erhalten. Hierfür kann die Auswerteanordnung beispielsweise von dem thermischen Gassensor zusätzlich zu den Detektorsignalen des ersten Detektors bzw. des zweiten Detektors das Heizer-Signal erhalten. Alternativ kann die Auswerteanordnung beispielsweise von dem thermischen Gassensor direkt die Information über die Amplitude des Detektorsignals des ersten Detektors, die Information über die Amplitude des Detektorsignals des zweiten Detektors, die Information über die erste Phasendifferenz und die Information über die zweite Phasendifferenz erhalten.

Dieses Ausführungsbeispiel der Auswerteanordnung basiert auf der Erkenntnis, dass das Kombinationssignal, basierend auf der Information über die Amplituden der Detektorsignale und abhängig von der Information über die erste Phasendifferenz und der Information über die zweite Phasendifferenz, ein sehr stabiles Signal darstellt, das von der Auswerteanordnung sehr schnell weiterverarbeitet werden kann, um beispielsweise eine Gaskonzentration oder eine Information über eine Temperaturleitfähigkeit des Fluids zu bestimmen. Somit erlaubt die Auswerteanordnung eine schnelle Gasanalyse.

Gemäß einem Ausführungsbeispiel kann die Auswerteanordnung ausgelegt sein, um eine Information über eine Heizer-Amplitude zu erhalten. Die Auswerteanordnung kann zudem ausgelegt sein, um eine Linearkombination der Information über die Heizer-Amplitude, der Information über Amplituden der Detektorsignale, der Information über die erste Phasendifferenz und der Information über die zweite Phasendifferenz zu bilden, um das Kombinationssignal (sigX) zu bestimmen. Die Information über die Heizer-Amplitude kann beispielsweise eine Information über eine Heizleistung sein. Hierin kann die Information über eine Heizer-Amplitude auch als Hz.Uss bezeichnet werden. Die Auswerteanordnung kann beispielsweise die Information über die Heizer-Amplitude direkt von dem thermischen Gassensor erhalten oder beispielsweise aus einem von dem thermischen Gassensor an die Auswerteanordnung gesendeten Heizer-Signal erhalten. Die Linearkombination kann beispielsweise einen ersten Term, der eine erste Linearkombination der Information über die Heizer-Amplitude und der Information über Amplituden der Detektorsignale aufweist, und einen zweiten Term aufweisen, der eine zweite Linearkombination der Information über die erste Phasendifferenz und der Information über die zweite Phasendifferenz aufweist. Dabei können beispielsweise der erste Term und der zweite Term mit unterschiedlichen Konstanten in der Linearkombination gewichtet werden, um das Kombinationssignal zu bestimmen. Dadurch, dass die Auswerteanordnung die Heizer-Amplitude in der Bestimmung des Kombinationssignals berücksichtigt, kann das Heizer-Signal (z. B. ein vom Heizer ausgesendetes Signal von periodischen Temperaturwellen) mit dem Detektorsignal des ersten Detektors bzw. des zweiten Detektors (z. B. ein empfangenes Signal von periodischen Temperaturwellen) verglichen werden, wodurch ein Wärmeübertrag vom Heizer über das zu analysierende Gas zu den zwei Detektoren sehr genau bestimmt werden kann. Somit wird eine sehr genaue und schnelle Gasanalyse mit der Auswerteanordnung ermöglicht.

Gemäß einem Ausführungsbeispiel kann die Auswerteanordnung ausgelegt sein, um das Kombinationssignal sigX gemäß

$$sigX = sigUss * Ka + sigPhi * Kp$$

zu erhalten. Hierbei kann sigUss eine Amplitudeninformation oder ein Amplituden-Signal sein, das von der Information über die Amplitude des Detektorsignals des ersten Detektors und von der Information über die Amplitude des Detektorsignals des zweiten Detektors abhängig sein kann. Der Term sigPhi kann eine Phaseninformation oder ein addiertes Phasensignal sein, das von der Information über die erste Phasendifferenz und von der Information über die zweite Phasendifferenz abhängig sein kann und die Faktoren Ka und Kp können Konstanten sein. Dabei können die Konstanten Ka und Kp separat die Amplitudeninformation bzw. die Phaseninformation gewichten, damit die Auswerteanordnung das Kombinationssignal sigX erhalten kann. Die Amplitudeninformation sigUss kann eine Linearkombination der Information über die Amplitude des Detektorsignals des ersten Detektors und der Information über die Amplitude des Detektorsignals des zweiten Detektors sein. Die Phaseninformation sigPhi kann eine Linearkombination der Information über die erste Phasendifferenz und der Information über die zweite Phasendifferenz sein. Die Konstanten Ka und Kp können beispielsweise Umrechnungsfaktoren darstellen. Gemäß einem Ausführungsbeispiel handelt es sich bei Ka und Kp um Gewichtungsfaktoren für ein optimiertes Kombinationssignal, wobei die Faktoren Ka und Kp einheitslose Größen darstellen können (Für ein embedded System wie es hier z. B. genutzt wird, das z.B. eine CO2-Konzentration liefert, ist das z. B. nicht notwendig). Gemäß einem Ausführungsbeispiel liefert das embedded System z. B. für die Amplitude AD-digits und PhasenInformation werden z. B. aus der Timing-Unit des embedded Systems, die die Zeiten bis zum Kippen des Komparators z. B. misst, ermittelt. Eine Umrechnungen auf Amplitude und Zeit/Winkel ist somit z. B. durch die technischen Daten einer Schaltung und dem embeded System ($\mu$Controller) der Auswerteanordnung vorgegeben. Die Faktoren sind z. B. so gewählt, dass über den Messbereich der CO2-Kalibrierung beide Signal-Anteile (Amplitude und Phase) zu etwa gleichen Anteilen in das Kombinationssignal SigX eingehen, so dass z. B. die größte Messauflösung in SigX erhalten wird. Die Faktoren Ka und Kp werden z. B. empirisch ermittelt, um z. B. das beste Signal für SigX zu erhalten. Die Konstanten Ka und Kp können beispielsweise abhängig von einer Konzentration, einer Temperatur oder einem Druck eines zu analysierenden Gases sein. Somit kann die Amplitudeninformation sigUss auf die Phaseninformation sigPhi abgestimmt sein. Es wird ermöglicht, dass die Auswerteanordnung mit dem Kombinationssignal sowohl Amplitudeninformationen als auch Phaseninformationen in einem weiterverarbeiten kann, wodurch beispielsweise die Auswerteanordnung das von dem Gassensor detektierte Gas sehr schnell, effizient und genau analysieren kann.

Gemäß einem Ausführungsbeispiel ist die Auswerteanordnung ausgelegt, um die Amplitudeninformation sigUss gemäß sigUss=2*Hz.Uss-(D1.Uss+D2.Uss) zu erhalten. Der Term Hz.Uss kann eine Information über eine Heizer-Amplitude sein, der Term D1.Uss kann die Information über die Amplitude des Detektorsignals des ersten Detektors sein und D2.Uss kann die Information über die Amplitude des Detektorsignals des zweiten Detektors sein. Somit kann die Amplitudeninformation sigUss ein relatives Amplituden-Signal darstellen. In anderen Worten kann die Amplitudeninformation eine Differenz aus der doppelten Heizer-Amplitude und einer Summe der Information über die Amplitude des Detektorsignals des ersten Detektors und der Information über die Amplitude des Detektorsignals des zweiten Detektors sein. Durch diese spezielle Berechnung der Amplitudeninformation sigUss kann erreicht werden, dass die Amplitudeninformation sigUss im Wesentlichen von einer Wärmeübertragung durch das Fluid abhängt und unbekannte Wärmeübergänge beispielsweise von dem zumindest einen Heizer in das zu analysierende Gas und von dem zu analysierenden Gas in den ersten bzw. zweiten Detektor nicht oder nur kaum berücksichtigt werden. Somit wird das Kombinationssignal sigX, das von der Amplitudeninformation sigUss abhängen kann, z. B. gar nicht oder nur kaum von unbekannten Wärmeübergängen beeinflusst, wodurch die Auswerteanordnung sehr genau Eigenschaften des zu analysierenden Gases, wie z. B die Information über die Gaskonzentration oder die Information über die Temperaturleitfähigkeit des Fluids, bestimmen kann.

Gemäß einem Ausführungsbeispiel kann die Auswerteanordnung ausgelegt sein, um ein Polynom (beispielsweise ersten Grades, beispielsweise A.y(sigX)) des Kombinationssignals zu berechnen, um die Information über die Gaskonzentration oder die Information über die Temperaturleitfähigkeit des Fluids zu erhalten. Durch die Polynombildung der Auswerteanordnung des Kombinationssignals kann eine Drift-Korrektur des Kombinationssignals durchgeführt werden. So können beispielsweise Konzentrations-Drift, Druck-Drift und Temperatur-Drift durch die Polynombildung korrigiert werden. So kann die Auswerteanordnung beispielsweise drei Polynome des Kombinationssignals berechnen, wobei ein erstes Polynom einen Zusammenhang zwischen der Gaskonzentration und dem Kombinationssignal darstellen kann, ein zweites Polynom, einen Zusammenhang zwischen Druck zu Signal-Verschiebung (Druck-Drift des Kombinationssignals) darstellen kann und ein drittes Polynom, einen Zusammenhang zwischen einer Temperatur zu Druck-Verschiebung darstellen kann. Somit können durch dieses Merkmal mögliche Ungenauigkeiten korrigiert werden und somit kann die Auswerteanordnung ausgebildet sein, um sehr genau mit einer Reduzierung von möglichen Fehlern das Fluid zu analysieren.

Gemäß einem Ausführungsbeispiel kann die Auswerteanordnung ausgelegt sein, um ein Polynom des Kombinationssignals mit einem Korrekturterm zu multiplizieren, um die Information über die Gaskonzentration oder die Information über die Temperaturleitfähigkeit zu erhalten. Der Korrekturterm kann von dem Kombinationssignal, einer Information über einen Druck (p) und einer Information über eine Temperatur (T) abhängig sein. Somit kann der Korrekturterm beispielsweise eine Druck- und Temperaturabhängigkeit des Kombinationssignals kompensieren. So kann beispielsweise ein Polynom, das einen Zusammenhang zwischen einer Gaskonzentration und dem Kombinationssignal darstellt, mit dem Korrekturterm um eine Druck- und Temperatur-Drift korrigiert werden. Somit kann durch die Multiplikation des Korrekturterms mit dem Polynom des Kombinationssignals eine Reduzierung von möglichen Fehlereinflüssen von der Auswerteanordnung durchgeführt werden, wodurch die Auswerteanordnung ausgebildet sein kann, um sehr genau Informationen über die Gaskonzentration oder Informationen über die Temperaturleitfähigkeit zu erhalten. Hierdurch können beispielsweise druck- und temperaturabhängige Fehler, entstanden beispielsweise bei der Detektion eines Detektorsignals durch den ersten Detektor bzw. den zweiten Detektor des Gassensors, minimiert werden.

Gemäß einem Ausführungsbeispiel kann die Auswerteanordnung ausgelegt sein, um eine Berechnung gemäß

$$C = pol(sigX) \cdot \left(1 - \left[\frac{f(p)}{sigX - const1}\right] \cdot \left(1 - \left[\frac{f(T)}{p - const2}\right]\right)\right)$$

durchzuführen, um eine Information C über die Gaskonzentration zu erhalten. Der Term sigX kann das Kombinationssignal sein, der Term pol(sigX) kann ein Polynom des Kombinationssignals sigX sein, f(p) kann eine Funktion des Drucks p sein (bzw. eines gemessenen Drucks p in einer Umgebung des thermischen Gassensors), const1 kann eine erste Konstante sein, f(T) kann eine Funktion der Temperatur T sein (bzw. einer gemessenen Temperatur T in einer Umgebung des thermischen Gassensors) und const2 kann eine zweite Konstante sein. Die Funktion f(p) kann beispielsweise ein Polynom sein, das einen Zusammenhang zwischen einem Druck zu einer Signal-Verschiebung darstellen kann und f(T) kann ein Polynom sein, das einen Zusammenhang zwischen einer Temperatur zu einer Signal-Verschiebung darstellen kann. Mit diesem Merkmal kann das Polynom des Kombinationssignals um druck- bzw. temperaturabhängige Fehler, hervorgerufen durch den Gassensor, korrigiert bzw. reduziert werden, wodurch eine sehr genaue Gasanalyse durch die Auswerteanordnung ermöglicht wird.

Gemäß einem Ausführungsbeispiel kann die Auswerteanordnung ausgelegt sein, um eine Berechnung gemäß

$$C[vol\%] = A.y(sigX) \cdot \left(1 - \left[\frac{B.y(p) - B.ref}{sigX - B.ref}\right] \cdot \left(1 - \left[\frac{C.y(T) - C.ref}{p - C.ref}\right]\right)\right)$$

durchzuführen, um die Information C über die Gaskonzentration zu erhalten. Der Term sigX kann das Kombinationssignal sein, A.y(sigX) kann ein Polynom (beispielsweise erster Ordnung) des Kombinationssignals sigX sein, B.y(p) kann eine Funktion des Drucks p sein (bzw. eines gemessenen Drucks p in einer Umgebung des thermischen Gassensors), B.ref kann eine Konstante sein, C.y(T) kann eine Funktion der Temperatur T sein (bzw. einer gemessenen Temperatur T in einer Umgebung des thermischen Gassensors) und C.ref kann eine zweite Konstante sein. Hierbei ist B.y(p) beispielsweise eine Polynomfunktion (beispielsweise zweiter Ordnung), die einen Zusammenhang zwischen dem Druck p zu einer Signal-Verschiebung (z. B. des Kombinationssignals sigX) darstellen kann. Die Funktion C.y(T) ist beispielsweise eine Polynomfunktion (beispielsweise zweiter Ordnung), die z. B. einen Zusammenhang zwischen der Temperatur T zu einer Druck-Verschiebung darstellen kann. Somit ist die Auswerteanordnung ausgelegt, um sehr genau beispielsweise eine Information über die Gaskonzentration eines zu analysierenden Fluids zu bestimmen, da die Funktion B.y(p) des Drucks p und die Funktion C.y(T) der Temperatur T einen Korrekturterm bilden können, der das Kombinationssignal sigX korrigieren kann.

Gemäß einem Ausführungsbeispiel kann die Auswerteanordnung ausgelegt sein, um einen Druck und/oder eine Temperatur in einer Umgebung des thermischen Gassensors bei der Bestimmung der Information über die Gaskonzentration zu berücksichtigen. Hierfür kann die Auswerteanordnung beispielsweise Informationen über den Druck und/oder die Temperatur in der Umgebung des thermischen Gassensors erhalten. Der Druck in der Umgebung des thermischen Gassensors wird beispielsweise durch einen Drucksensor und die Temperatur in der Umgebung des thermischen Gassensors wir beispielsweise durch einen Temperatursensor bestimmt und an die Auswerteanordnung zu übermittelt. Der Drucksensor und/oder der Temperatursensor kann in der Umgebung des thermischen Gassensors angeordnet sein. Somit wird ermöglicht, dass die Auswerteanordnung abhängig von dem Druck und/oder der Temperatur Korrekturen durchführt, und somit sehr genau das Fluid analysieren kann und somit sehr genau Informationen beispielsweise über die Gaskonzentration bzw. eine Temperaturleitfähigkeit des Fluids erhalten kann.

Gemäß einem Ausführungsbeispiel kann die Auswerteanordnung ausgelegt sein, um bei der Bestimmung der Information über die Gaskonzentration als Eingangsgrößen einer Drift-Korrektur das Kombinationssignal, eine Information über die Temperatur in einer Umgebung des thermischen Gassensors und eine Information über einen Druck in einer Umgebung des thermischen Gassensors zu verwenden, und um die Information über die Gaskonzentration als Ergebnis der Drift-Korrektur zu erhalten. Die Drift-Korrektur erhält außer den drei genannten Eingangsvariablen beispielsweise keine weiteren Variablen mehr, sondern verwendet beispielsweise nur noch (z. B. zusätzlich) vorher erhaltene - beispielsweise im Rahmen einer Kalibration bestimmte - Konstanten. Somit kann die Auswerteanordnung ausgebildet sein, um mögliche Fehler in der Berechnung der Gaskonzentration, hervorgerufen durch Drift, herauszurechnen und somit eine Drift-Korrektur durchzuführen. Die Drift kann bei unterschiedlichen Temperaturen und Drücken entstehen und so eine Bestimmung der Gaskonzentration verfälschen, was mit diesem Merkmal verhindert werden kann bzw. unterdrückt werden kann. Somit wird ermöglicht mit der Auswerteanordnung sehr genau Informationen über die Gaskonzentration zu bestimmen.

Gemäß einem Ausführungsbeispiel ist die Auswerteanordnung ausgelegt, um das Kombinationssignal, bzw. ein weiteres Kombinationssignal, basierend auf einem Quotienten zwischen einer Amplitudeninformation, die von der Information über die Amplitude des Detektorsignals zumindest des ersten Detektors und optional auch von der Information über die Amplitude des Detektorsignals des zweiten Detektors abhängig ist, und einer Phaseninformation, die von der Information über die erste Phasendifferenz und optional von der Information über die zweite Phasendifferenz abhängig ist, zu erhalten. Ferner kann die Auswerteanordnung ausgelegt sein, um abhängig von dem Kombinationssignal eine Information über eine Konzentration einen Gases, z. B. eines dritten Gases eines Gasgemisches, zu bestimmen. In anderen Worten handelt es sich bei dem Quotienten um ein Verhältnis zwischen der Information über die Amplitude und der Phaseninformation. Gemäß einem Ausführungsbeispiel verschiebt das dritte Gas dieses Verhältnis, wodurch die Auswerteanordnung ausgelegt sein kann, um basierend auf diesem Verhältnis auf die Konzentration der dritten Gaskomponente zu schließen.

Gemäß einem Ausführungsbeispiel ist die Auswerteanordnung ausgelegt, um das Kombinationssignal sigV gemäß

$$sigV=sigUss*Kav/(sigPhi*Kpv)$$

zu erhalten. In der Formel kann sigUss eine Amplitudeninformation sein, die von der Information über die Amplitude des Detektorsignals des ersten Detektors und optional von der Information über die Amplitude des Detektorsignals des zweiten Detektors abhängig ist. Ferner kann sigPhi eine Phaseninformation sein, die von der Information über die erste Phasendifferenz und optional von der Information über die zweite Phasendifferenz abhängig ist. Des Weiteren stellen Kav und Kpv Konstanten dar. Das Kombinationssignal sigV stellt z. B. das Verhältnis zwischen der Information über die Amplitude und der Phaseninformation dar. In anderen Worten ist die Auswerteanordnung ausgelegt, um mittels des Verhältnisses sigV aus Amplituden- und Phasen-Signal einen weiteren physikalischen Gasparameter zu ermitteln, mit dem mittels der Auswerteanordnung z. B. durch Korrelation auf die unbekannte Konzentration eines bekannten dritten Gases in dem zu analysierenden Gasgemisch geschlossen werden kann. Kav und Kpv sind neue Gewichtungsfaktoren, die Änderungen im Verhältnis der Amplituden und Phasen verstärken.

Gemäß einem Ausführungsbeispiel ist die Auswerteanordnung ausgelegt, um eine Information darüber zu erhalten, wieviel Wärme von dem Heizer während einer Heizperiode abgeführt wird, und um abhängig von der Information darüber, wieviel Energie von dem Heizer während der Heizperiode abgeführt wird, eine Information über eine Konzentration eines Gases, z. B. eines dritten Gases eines Gasgemisches, zu bestimmen. Unter einer Heizperiode kann ein Zeitabschnitt zwischen einem ersten Nulldurchgang einer Heizspannung bis zu einem zweiten Nulldurchgang der Heizspannung verstanden werden. Alternativ kann unter der Heizperiode auch ein Zeitabschnitt ab einem ersten Zeitpunkt, an dem die Heizspannung von Null Volt zu größer oder kleiner Null Volt wechselt, bis zu einem zweiten Zeitpunkt, an dem die Heizspannung von größer oder kleiner Null Volt zu Null Volt wechselt, verstanden werden. Das Heizsignal kann z. B. ein Sinussignal, ein Cosinussignal, ein Rechtecksignal, ein Dreiecksignal oder ein Sägezahnsignal aufweisen. Die Wärme wird während der Heizperiode an das den Heizer umgebende Gasgemisch abgeführt. Wie viel Wärme von dem Heizer an das umliegende Gas abgeführt wird ist z. B. von einer Wärmeleitfähigkeit des umliegenden Gases, bzw. von einer Wärmeleitfähigkeit von Gaskomponenten des umliegenden Gasgemisches abhängig. Somit kann die Auswerteanordnung ausgelegt sein, um mittels der von dem Heizer abgeführten Wärme eine Wärmeleitfähigkeit des unbekannten Gases oder Gasgemisches zu ermitteln.

Gemäß einem Ausführungsbeispiel ist die Auswerteanordnung ausgelegt, um die Information, wieviel Wärme von dem Heizer während einer Heizperiode abgeführt wird, basierend auf einer Messung eines Stromflusses durch den Heizer bei vorgegebener Heizspannung zu erhalten. In anderen Worten ist während der Heizperiode die an den Heizer angelegte Heizspannung festgelegt und der Stromfluss ändert sich je nach dem wie viel Wärme an das Gas oder Gasgemisch abgegeben wird. Desto mehr Wärme abgegeben wird, desto weniger stark erhöht sich die Temperatur des Heizers und somit unter Voraussetzung eines positiven TKR (Temperaturkoeffizient des Widerstandes) der Wert des Heizerwiderstandes, wodurch der Stromfluss weniger sinkt. Somit kann durch eine Bestimmung des Stromflusses durch den Heizer mittels der Auswerteanordnung auf eine Zusammensetzung des zu analysierenden Gasgemisches geschlossen werden bzw. der Stromfluss als zusätzliche Information von der Auswerteanordnung genutzt werden, um eine Genauigkeit in der Gasanalyse weiter zu verbessern.

Gemäß einem Ausführungsbeispiel ist die Auswerteanordnung ausgelegt, um den Stromfluss kurz nach einem Einschalten der vorgegebenen Heizspannung und kurz vor einem Ausschalten der vorgegebenen Heizspannung zu erhalten. Aus einer Differenz dieser beiden Stromflussdaten kann die Auswerteanordnung eine Änderung des Stromflusses während der Heizperiode feststellen. Je größer die Differenz, desto geringer ist die Wärmeleitfähigkeit des zu analysierenden Gases oder Gasgemisches. Somit ist die Auswerteanordnung ausgebildet, um die Wärmeleitfähigkeit des Gases oder Gasgemisches z. B. als zusätzlichen Parameter in einer Analyse des Gases oder Gasgemisches zu nutzen. In anderen Worten kann die Auswerteanordnung ausgelegt sein, um die Messung der Wärmeleitfähigkeit als einen weiteren physikalischen Parameter des unbekannten Gasgemisches zu nutzen, indem die Auswerteanordnung z. B. ausgelegt ist, um die Differenz des Heizerstroms in einem Anfangsmaxima kurz nach Einschalten der Heizspannung und kurz vor dem Abschalten der Heizspannung (pro Periode) auszuwerten. Unter kurz nach dem Einschalten der Heizspannung kann ein Zeitpunkt in einer Zeitspanne von $10\mu s$ bis 1ms, $100\mu s$ bis $800\mu s$ oder $300\mu s$ bis $500\mu s$, wie z. B. bei $400\mu s$, nach dem Einschalten gemeint sein.

Ein Ausführungsbeispiel schafft ein Verfahren zur Auswertung von Signalen eines thermischen Gassensors mit zumindest einem Heizer und zumindest einem Detektor. Wärme kann beispielsweise von dem Heizer über ein zu analysierendes Gas zu dem Detektor übertragen werden. Das Verfahren kann ein Erhalten einer Information über eine Amplitude eines Detektorsignals eines ersten Detektors (beispielsweise D1.Uss) und einer Information über eine erste Phasendifferenz zwischen einem Heizer-Signal und dem Detektorsignal des ersten Detektors (beispielsweise (D1-Hz).phi) umfassen. Als Zwischengröße abhängig von der Information über die Amplituden des Detektorsignals und abhängig von der Information über die erste Phasendifferenz kann ein Kombinationssignal gebildet werden. Das Kombinationssignal kann beispielsweise eine Amplitudeninformation und eine Phaseninformation zusammenfassen. Eine Information über eine Gaskonzentration oder eine Information über eine Temperaturleitfähigkeit eines Fluids (beispielsweise eines Gases oder Gasgemisches) kann basierend auf dem Kombinationssignal bestimmt werden. Diese Bestimmung kann beispielsweise ohne im weiteren Fortgang der Berechnungen die in das Kombinationssignal einfließenden Einzelinformationen noch einmal separat zu betrachten erfolgen.

Ein Ausführungsbeispiel schafft ein Verfahren zur Auswertung von Signalen eines thermischen Gassensors mit zumindest einem Heizer und zwei in unterschiedlichen Abständen oder gleichen Abständen von dem Heizer angeordneten Detektoren. Wärme kann beispielsweise von dem Heizer über ein zu analysierendes Gas zu den beiden Detektoren übertragen werden. Das Verfahren kann ein Erhalten einer Information über eine Amplitude eines Detektorsignals eines ersten Detektors (beispielsweise D1.Uss), einer Information über eine Amplitude eines Detektorsignals eines zweiten Detektors (beispielsweise D2.Uss), einer Information über eine erste Phasendifferenz zwischen einem Heizer-Signal und dem Detektorsignal des ersten Detektors (beispielsweise (D1-Hz).phi), und einer Information über eine zweite Phasendifferenz zwischen dem Heizer-Signal und dem Detektorsignal des zweiten Detektors (beispielsweise (D2-Hz).phi) umfassen. Als Zwischengröße abhängig von der Information über die Amplituden der Detektorsignale und abhängig von der Information über die erste Phasendifferenz und abhängig von der Information über die zweite Phasendifferenz kann ein Kombinationssignal gebildet werden. Das Kombinationssignal kann beispielsweise eine Amplitudeninformation und eine Phaseninformation zusammenfassen. Eine Information über eine Gaskonzentration oder eine Information über eine Temperaturleitfähigkeit eines Fluids (beispielsweise eines Gases oder Gasgemisches) kann basierend auf dem Kombinationssignal bestimmt werden. Diese Bestimmung kann beispielsweise ohne im weiteren Fortgang der Berechnungen die in das Kombinationssignal einfließenden Einzelinformationen noch einmal separat zu betrachten erfolgen.

Ein Ausführungsbeispiel betrifft ein Computerprogramm mit einem Programmcode zur Durchführung eines Verfahrens, wenn das Programm auf einem Computer abläuft.

## Figurenkurzbeschreibung

Alle hierin erläuterten Beispiele, die nicht alle Merkmale, oder Äquivalente derselben, eines der unabhängigen Ansprüche aufweisen, dienen dazu das Verständnis der Erfindung zu erleichtern.

Ausführungsbeispiele gemäß der vorliegenden Erfindung werden nachfolgend Bezug nehmend auf die beiliegenden Figuren näher erläutert. Hinsichtlich der dargestellten schematischen Figuren wird darauf hingewiesen, dass die dargestellten Funktionsblöcke sowohl als Elemente oder Merkmale der erfindungsgemäßen Vorrichtung als auch als entsprechende Verfahrensschritte des erfindungsgemäßen Verfahrens zu verstehen sind, und auch entsprechende Verfahrensschritte des erfindungsgemäßen Verfahrens davon abgeleitet werden können. Es zeigen:

Fig. 1a    eine schematische Darstellung eines Gassensors gemäß einem Ausführungsbeispiel der vorliegenden Erfindung;

Fig. 1b    eine schematische Darstellung einer Auswerteanordnung für einen thermischen Gassensor gemäß einem Ausführungsbeispiel der vorliegenden Erfindung;

Fig. 1c    eine schematische Darstellung einer beispielhaften Auswerteanordnung für einen thermischen Gassensor mit einer Regelung einer Heizleistung;

Fig. 1d    eine schematische Darstellung einer beispielhaften Auswerteanordnung für einen thermischen Gassensor mit einer Abtastung eines Sensorsignals zu drei Zeitpunkten;

Fig. 2a    eine schematische Darstellung eines Gassensors am Lichtmikroskop gemäß einem Ausführungsbeispiel der vorliegenden Erfindung;

Fig. 2b    eine schematische Darstellung eines Gassensors im Raster-ElektronenMikroskop gemäß einem Ausführungsbeispiel der vorliegenden Erfindung;

Fig. 3      eine schematische Darstellung eines Ausschnittes einer Raster-ElektronenMikroskopie Aufnahme einer Mikro-Brücke für einen Gassensor gemäß einem Ausführungsbeispiel der vorliegenden Erfindung;

Fig. 4      eine schematische Darstellung eines Gassensors mit einer ersten Unterbrechung mit einer Ausdehnung senkrecht zu einem Heizer, unterschiedlich von einer Ausdehnung senkrecht zu einem Heizer einer zweiten Unterbrechung gemäß einem Ausführungsbeispiel der vorliegenden Erfindung;

Fig. 5      eine schematische Darstellung eines Gassensors mit einem ersten Unterbrechungsbereich sowie einem zweiten Unterbrechungsbereich, mit einer Mehrzahl an Unterbrechungen gemäß einem Ausführungsbeispiel der vorliegenden Erfindung;

Fig. 6a     eine schematische Darstellung eines Gassensors mit gleich vielen Unterbrechungen sowohl in einem ersten Unterbrechungsbereich als auch in einem zweiten Unterbrechungsbereich gemäß einem Ausführungsbeispiel der vorliegenden Erfindung;

Fig. 6b     eine schematische Darstellung eines Gassensors mit einer Vielzahl an Unterbrechungen in einem ersten Unterbrechungsbereich und einer einzigen Unterbrechung in einem zweiten Unterbrechungsbereich gemäß einem Ausführungsbeispiel der vorliegenden Erfindung;

Fig. 6c     eine schematische Darstellung eines Gassensors, wobei eine Vielzahl an Unterbrechungen in einem ersten Unterbrechungsbereich eine andere Ausdehnung senkrecht zu einem Heizer aufweisen als eine Vielzahl an Unterbrechungen in einem zweiten Unterbrechungsbereich gemäß einem Ausführungsbeispiel der vorliegenden Erfindung;

Fig. 7      eine schematische Darstellung eines Prinzips eines Gassensors gemäß einem Ausführungsbeispiel der vorliegenden Erfindung;

Fig. 8      eine schematische Darstellung eines Wärmetransports an einem Gassensor gemäß einem Ausführungsbeispiel der vorliegenden Erfindung;

Fig. 9      ein Diagramm eines beispielhaften Heizer-Signals, eines ersten Sensorsignals und eines zweiten Sensorsignals eines Gassensors;

Fig. 10     eine schematische Darstellung einer beispielhaften Heizer-Ansteuerung für einen Gassensor;

Fig. 11     eine schematische Darstellung einer beispielhaften Schaltung zur Auswertung eines Sensorsignals eines Gassensors;

Fig. 12     eine schematische Darstellung einer beispielhaften Regelung eines Gassensors;

Fig. 13a    ein Blockdiagramm eines beispielhaften Verfahrens zur Analyse eines Sensorsignals eines Gassensors;

Fig. 13b    ein Blockdiagramm eines beispielhaften Verfahrens zur Auswertung eines Sensorsignals eines Gassensors mit einer Nachführung von Abtastzeitpunkten;

Fig. 14     ein Diagramm einer Phasenverschiebung zwischen einem Heizer-Signal und zwei Sensorsignalen eines Gassensors gemäß einem Ausführungsbeispiel der vorliegenden Erfindung;

Fig. 15     ein Diagramm von Amplituden von zumindest einem Sensorsignal eines Gassensors gemäß einem Ausführungsbeispiel der vorliegenden Erfindung;

Fig. 16     ein Diagramm von Phasenverschiebungen zwischen einem ersten Sensorsignal und einem zweiten Sensorsignal eines Gassensors abhängig von einem Druck gemäß einem Ausführungsbeispiel der vorliegenden Erfindung;

Fig. 17a    ein Diagramm einer Phasenverschiebung eines Sensorsignals eines Gassensors in Abhängigkeit von einer Frequenz gemäß einem Ausführungsbeispiel der vorliegenden Erfindung;

Fig. 17b ein Diagramm einer Amplitude eines Sensorsignals eines Gassensors in Abhängigkeit einer Frequenz gemäß einem Ausführungsbeispiel der vorliegenden Erfindung;

Fig. 18 ein Diagramm von Phasenverschiebungen eines ersten Sensorsignals, eines zweiten Sensorsignals und eines Heizer-Signals eines Gassensors in Abhängigkeit einer Stickstoffkonzentration gemäß einem Ausführungsbeispiel der vorliegenden Erfindung;

Fig. 19 ein Diagramm einer Amplitude eines ersten Sensorsignals und eines zweiten Sensorsignals eines Gassensors in Abhängigkeit einer Stickstoffkonzentration gemäß einem Ausführungsbeispiel der vorliegenden Erfindung;

Fig. 20 ein Diagramm eines Kombinationssignals eines Gassensors für verschiedene Gasgemische gemäß einem Ausführungsbeispiel der vorliegenden Erfindung;

Fig. 21 ein Diagramm eines Kombinationssignals eines Gassensors in Abhängigkeit einer $CO_2$-Konzentration gemäß einem Ausführungsbeispiel der vorliegenden Erfindung;

Fig. 22 ein Diagramm eines Kombinationssignals eines Gassensors in Abhängigkeit eines Drucks gemäß einem Ausführungsbeispiel der vorliegenden Erfindung;

Fig. 23 ein Diagramm eines Zusammenhangs zwischen einem Gasdruck und einer Gastemperatur für einen Gassensor gemäß einem Ausführungsbeispiel der vorliegenden Erfindung;

Fig. 24 ein Blockdiagramm eines Verfahrens zur Erzeugung eines Kombinationssignals eines Gassensors gemäß einem Ausführungsbeispiel der vorliegenden Erfindung;

Fig. 25 ein Diagramm einer Temperaturleitfähigkeit in Abhängigkeit eines Kombinationssignals eines Gassensors gemäß einem Ausführungsbeispiel der vorliegenden Erfindung;

Fig. 26 ein Diagramm eines beispielhaften Stromflusses in einem Heizer während einer Heizperiode für ein erstes Gasgemisch;

Fig. 27 ein Diagramm eines beispielhaften Stromflusses in einem Heizer während einer Heizperiode für ein zweites Gasgemisch;

Fig. 28 ein Diagramm eines beispielhaften Stromflusses in einem Heizer während einer Heizperiode für ein drittes Gasgemisch;

Fig. 29 ein Diagramm unterschiedlicher Phaseninformationen für jeweils unterschiedliche Gasgemische gemäß einem Ausführungsbeispiel der vorliegenden Erfindung;

Fig. 30 ein Diagramm unterschiedlicher Amplitudeninformationen für jeweils unterschiedliche Gasgemische gemäß einem Ausführungsbeispiel der vorliegenden Erfindung; und

Fig. 31 ein Diagramm eines Kombinationssignals für unterschiedliche Gasgemische gemäß einem Ausführungsbeispiel der vorliegenden Erfindung.

## Detaillierte Beschreibung der Ausführungsbeispiele gemäß den Figuren

Bevor nachfolgend Ausführungsbeispiele der vorliegenden Erfindung im Detail anhand der Zeichnungen näher erläutert werden, wird darauf hingewiesen, dass identische, funktionsgleiche oder gleichwirkende Elemente, Objekte und/oder Strukturen in den unterschiedlichen Figuren mit den gleichen oder ähnlichen Bezugszeichen versehen sind, so dass die in unterschiedlichen Ausführungsbeispielen dargestellte Beschreibung dieser Elemente untereinander austauschbar ist bzw. aufeinander angewendet werden kann.

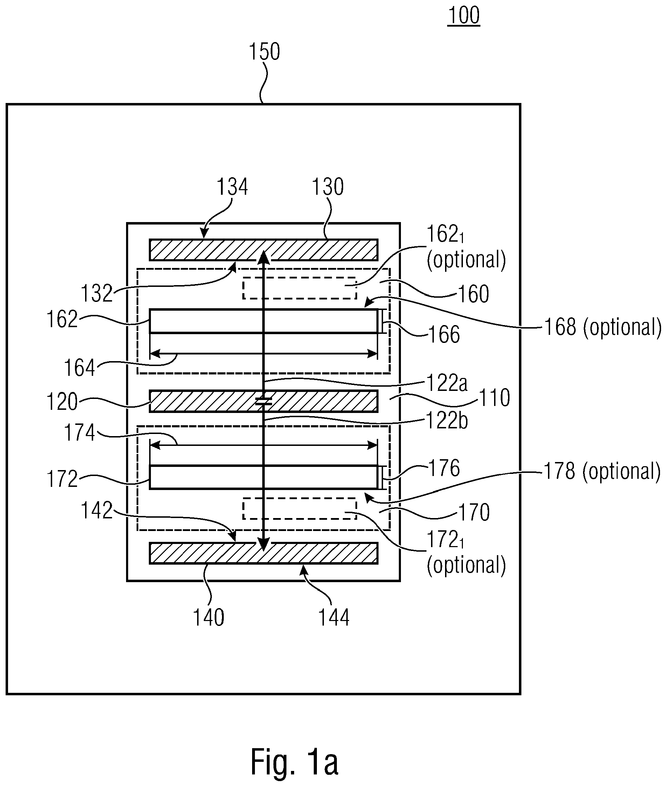

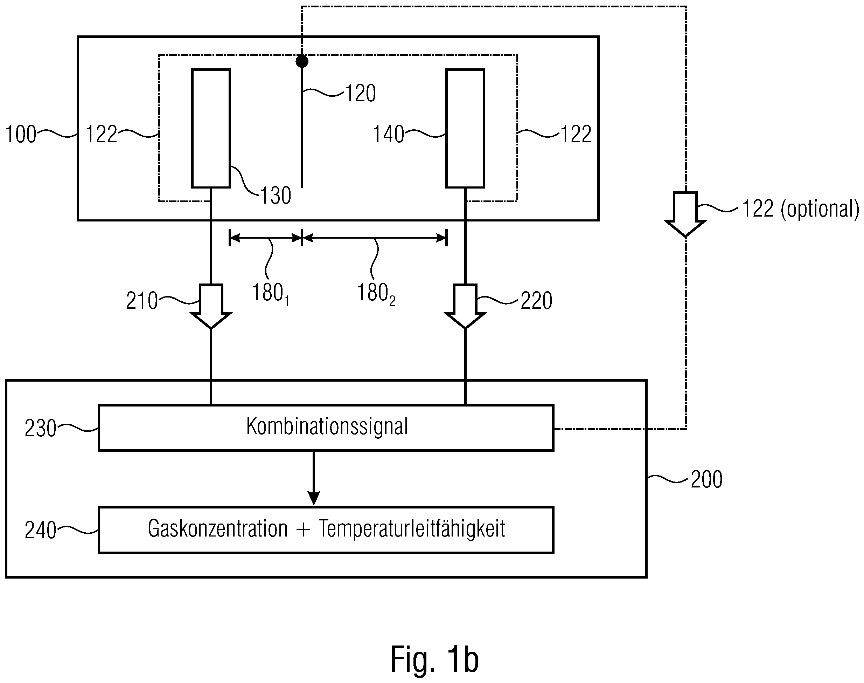

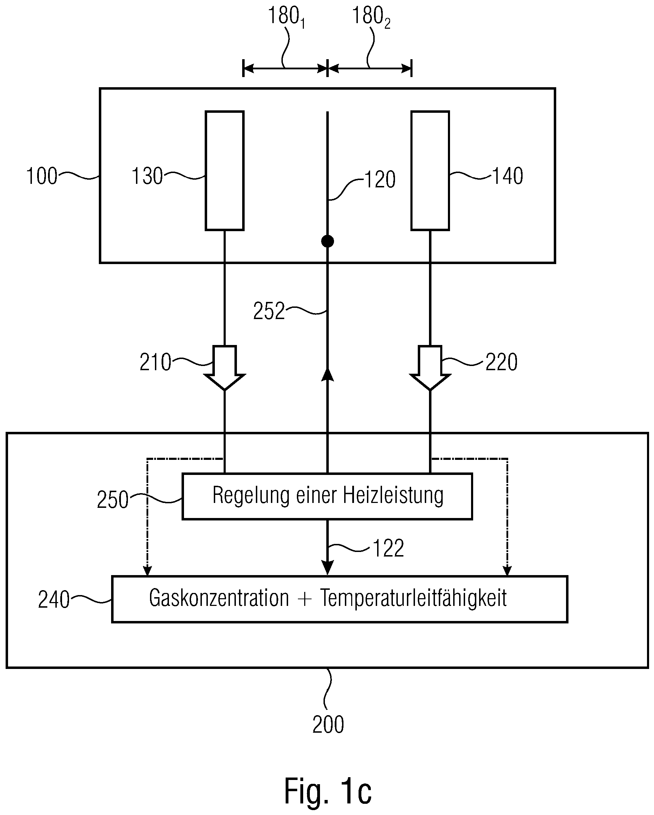

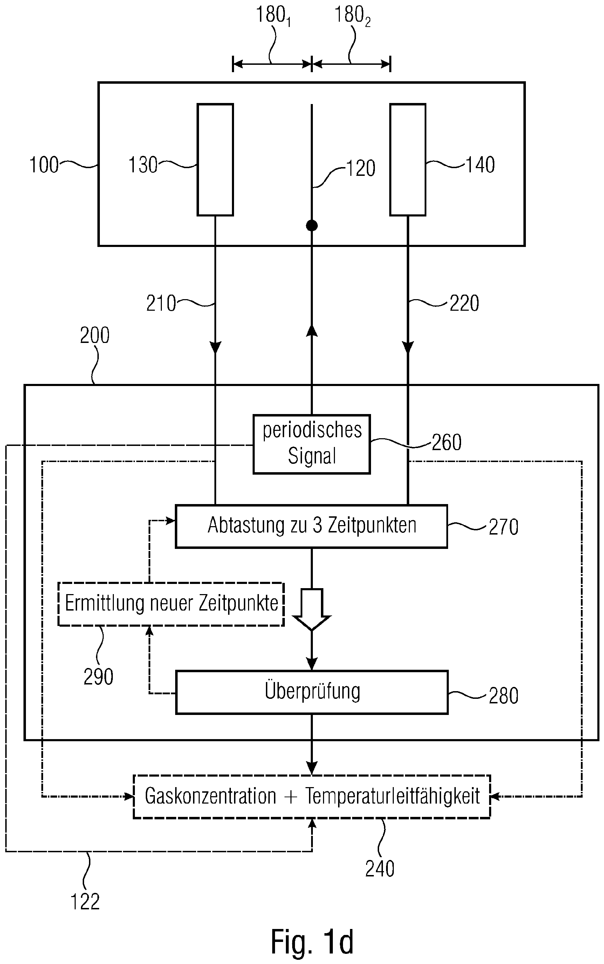

Fig. 1 zeigt eine schematische Darstellung eines Gassensors 100 gemäß einem Ausführungsbeispiel der vorliegenden Erfindung. Der Gassensor 100 kann eine Membran 110 (z. B eine Dünnschichtmembran), ein Heizelement 120, eine erste Thermoelement-Struktur 130 und eine zweite Thermoelement-Struktur 140 aufweisen. Optional kann der Gassensor auch nur die erste Thermoelement-Struktur 130 oder die zweite Thermoelement-Struktur 140 aufweisen.

Die Membran 110 kann von einem Rahmen 150 aufgespannt sein und einen ersten Unterbrechungsbereich 160 sowie einen zweiten Unterbrechungsbereich 170 aufweisen. Der erste Unterbrechungsbereich 160 der Membran 110 kann mindestens eine Unterbrechung 162 aufweisen und der zweite Unterbrechungsbereich 170 der Membran 110 kann ebenso mindestens eine Unterbrechung 172 aufweisen. Das Heizelement 120 kann beispielsweise als freitragende Brückenstruktur auf der Membran 110 zwischen dem ersten Unterbrechungsbereich 160 und dem zweiten Unterbrechungsbereich 170 der Membran 110 angeordnet sein. Die erste Thermoelement-Struktur 130 kann ein heißes Ende 132 und ein kaltes Ende 134 aufweisen. Das heiße Ende 132 der ersten Thermoelement-Struktur 130 kann auf der Membran 110 auf einer dem Heizelement 120 entgegengesetzten Seite des ersten Unterbrechungsbereichs 160 angeordnet sein. Die zweite Thermoelement-Struktur 140 kann ebenfalls ein heißes Ende 142 und ein kaltes Ende 144 aufweisen. Das heiße Ende 142 kann auf der Membran 110 auf einer dem Heizelement 120 entgegengesetzten Seite des zweiten Unterbrechungsbereichs 170 angeordnet sein.

Bei der Membran 110 kann es sich um eine Dünnschichtmembran mit einer Dicke zwischen 200 nm und 4000 nm, 300 nm und 3000 nm, 400 nm und 2000 nm oder zwischen 1 $\mu$m und 10 $\mu$m handeln. Gemäß einem Ausführungsbeispiel liegt die Dicke der Gesamt-Membran bei ca. 2 $\mu$m (sie besteht z. B. aus mehreren Membran-, Sensor- und Passivierungs-Schichten). Die Membranschicht kann z. B. Si-Oxid und/oder Si-Nitrid aufweisen. Als Dicke kann eine Ausdehnung der Membran 110 in die Blattebene hinein, also beispielsweise senkrecht zu einer Oberfläche der Membran 110, auf der das Heizelement 120, die erste Thermoelement-Struktur 130 und die zweite Thermoelement-Struktur 140 angeordnet sind, definiert sein. Die Membran 110 kann leitendes Material, isolierendes Material oder Halbleitermaterial aufweisen, wobei das Material eine sehr geringe Wärmeleitfähigkeit, von beispielsweise unter 5W/(m*K), unter 100 mW/(m*K) oder unter 50 mW/(m*K), aufweisen kann. So kann beispielsweise ein Halbleiter mit angepasster Grunddotierung in einem einfachen Fünfmasken-MEMS-Prozess als kostengünstiges Substrat für die Herstellung der Membran 110 dienen.

Gemäß einem Ausführungsbeispiel kann das Heizelement 120 (das Heizelement 120 kann im Folgenden auch als Heizer bezeichnet werden) eine freitragende Brückenstruktur bilden und/oder einen Draht umfassen. Gemäß einem Ausführungsbeispiel kann das Heizelement 120 von einer Seite des Rahmens 150 zu einer gegenüberliegenden Seite des Rahmens 150 aufgespannt sein. An das Heizelement 120 kann beispielsweise eine Spannung angelegt sein, wodurch das Heizelement 120 Heizleistung an ein zu analysierendes Gas, befindlich beispielsweise in dem ersten Unterbrechungsbereich 162 und/oder in dem zweiten Unterbrechungsbereich 172, übertragen kann. Die an das Heizelement 120 angelegte Spannung kann beispielsweise ein periodisches Spannungssignal sein, wie z. B. ein Sinussignal oder ein periodisches Rechtecksignal. Somit kann das Heizelement 120 beispielsweise ein periodisches Heizer-Signal (beispielsweise die Heizleistung) bereitstellen. Das Heizer-Signal kann beispielsweise über die Membran 110 und/oder über ein Gas, befindlich beispielsweise in der ersten Unterbrechung 162 oder der zweiten Unterbrechung 172, zu der ersten Thermoelement-Struktur 130 und/oder der zweiten Thermoelement-Struktur 140 übertragen werden.

Die erste Thermoelement-Struktur 130 und/oder die zweite Thermoelement-Struktur 140 sind beispielsweise mäanderförmig ausgebildet, was beispielsweise in Reihe geschalteten Thermoelementen entsprechen kann, die eine Thermokette bilden. Die erste Thermoelement-Struktur 130 und/oder die zweite Thermoelement-Struktur 140 können somit als Detektor dienen, wobei die erste Thermoelement-Struktur 130 und/oder die zweite Thermoelement-Struktur 140 beispielsweise das Heizer-Signal detektieren können.

Gemäß einem Ausführungsbeispiel können die erste Thermoelement-Struktur 130 und/oder die zweite Thermoelement-Struktur 140 komplett auf der Membran 110 angeordnet sein, oder zumindest teilweise auf der Membran 110 und zumindest teilweise auf dem Rahmen 150 angeordnet sein. So kann beispielsweise eine Temperatur des Rahmens 150 als Vergleichstemperatur dienen (hier können beispielsweise die kalten Enden 134 der ersten Thermoelement-Struktur 130 angeordnet sein und/oder die kalten Enden 144 der zweiten Thermoelement-Struktur 140) und der Teil der Thermoelement-Struktur, der auf der Membran 110 angeordnet ist (z. B. die heißen Enden 132, 142) kann eine Messtemperatur (z. B. das Heizer-Signal) detektieren. Die heißen Enden 132, 142 und die kalten Enden 134, 144 sind beispielsweise mit einem Leiter verbunden. So kann beispielsweise ein Leiter, umfassend ein erstes Material, ein erstes kaltes Ende mit einem ersten heißen Ende verbinden und ein zweiter Leiter, umfassend ein zweites Material, kann das erste heiße Ende mit einem zweiten kalten Ende verbinden. Diese Verbindung eines ersten Leiters und eines zweiten Leiters kann ein Thermoelement darstellen, das beispielsweise in Reihe zu einer Thermokette geschaltet werden kann und somit beispielsweise die erste Thermoelement-Struktur 130 oder die zweite Thermoelement-Struktur 140 darstellen kann. Entlang dieser Leiter kann somit beispielsweise eine Temperaturdifferenz (z. B. zwischen der Vergleichstemperatur und der Messtemperatur) auftreten, wodurch beispielsweise eine elektrische Spannung an den Enden (z. B. den heißen Enden und/oder den kalten Enden) der metallischen Leiter induziert werden kann. Somit können die erste Thermoelement-Struktur 130 und/oder die zweite Thermoelement-Struktur 140 beispielsweise ausgebildet sein, um Wärme in elektrische Energie umzuwandeln. Gemäß einem Ausführungsbeispiel kann es sich bei der ersten Thermoelement-Struktur 130 und/oder der zweiten Thermoelement-Struktur 140 um einen Draht oder eine freitragende Brückenstruktur handeln.

Gemäß einem Ausführungsbeispiel kann die Membran 110 durch den Rahmen 150 aus Trägermaterial aufgespannt sein, das so ausgelegt ist, dass der Temperaturausdehnungskoeffizient und/oder eine Wärmeleitfähigkeit von Membranmaterial von dem Temperaturausdehnungskoeffizient und/oder der Wärmeleitfähigkeit des Trägermaterials abweicht. Der Rahmen 150 kann Trägermaterial oder Substratmaterial aufweisen, mit dem die Membran 110 beispielsweise getragen werden kann. Somit kann beispielsweise an dem Rahmen 150 eine Vergleichstemperatur eingestellt werden. Gemäß einem Ausführungsbeispiel können der Rahmen 150 und die Membran 110 auch denselben Temperaturausdehnungskoeffizienten aufweisen.

Gemäß einem Ausführungsbeispiel kann die Membran 110 eine geringere Wärmeleitfähigkeit aufweisen als der Rahmen 150. Hierbei sollte insbesondere die Membran 110 beispielsweise eine sehr geringe Wärmeleitfähigkeit aufweisen, damit beispielsweise das Heizer-Signal von dem Heizelement 120 hauptsächlich über das zu analysierende Gas (angeordnet beispielsweise in der ersten Unterbrechung 162 und/oder in der zweiten Unterbrechung 172) zu der ersten Thermoelement-Struktur 130 und/oder der zweiten Thermoelement-Struktur 140, übertragen wird, anstelle über die Membran 110. Somit kann beispielsweise ein Wärmetransport über die Membran 110 unterdrückt, reduziert oder verlangsamt werden.

Die Membran 110 kann somit ausgebildet sein, um parasitäre Wärmeleitung von dem Heizelement 120 zu der ersten Thermoelement-Struktur 130 bzw. zu der zweiten Thermoelement-Struktur 140 zu unterdrücken. So kann die Wärmeleitfähigkeit der Membran 110 beispielsweise so gewählt sein, dass kaum bis keine Wärme von dem Heizelement 120 über die Membran 110 an die erste Thermoelement-Struktur 130 bzw. die zweite Thermoelement-Struktur 140 geleitet wird und ein Großteil der Wärme bzw. die komplette Wärme über das zu analysierende Gas geleitet wird.

Hingegen kann die Wärmeleitfähigkeit des Trägermaterials des Rahmens 150, der die Membran 110 hält, sehr hoch sein. So kann als Trägermaterial z. B. Silizium mit einer Wärmeleitfähigkeit von 150 W/(m*K) verwendet werden. Das Trägermaterial kann somit als Wärmesenke dienen. So ist beispielsweise die erste Thermoelement-Struktur 130 bzw. die zweite Thermoelement-Struktur 140 zumindest teilweise, z. B. mit den heißen Enden 132, 142, auf der Membran und zumindest teilweise, z. B. mit den kalten Enden 134, 144, auf dem Trägermaterial angeordnet, wodurch innerhalb der ersten Thermoelement-Struktur 130 bzw. der zweiten Thermoelement-Struktur 140 eine Temperaturdifferenz auftreten kann, mithilfe derer der Wärmetransport von dem Heizelement 120 zu der jeweiligen Thermoelement-Struktur 130, 140 detektiert werden kann.

Gemäß einem Ausführungsbeispiel können somit die kalten Enden der ersten Thermoelement-Struktur 130 und die kalten Enden der zweiten Thermoelement-Struktur 140 auf dem Trägermaterial des Rahmens 150 angeordnet sein. Sie befinden sich beispielsweise dort, wo die Membran 110 durch das Trägermaterial getragen wird.

Gemäß einem Ausführungsbeispiel kann der erste Unterbrechungsbereich 160 der Membran 110 eine durchgehende Unterbrechung 162 aufweisen, deren Längsausdehnung 164 so groß ist, dass sie den Bereich zwischen der ersten Thermoelement-Struktur 130 und dem Heizelement 120 vollständig abdeckt. Der zweite Unterbrechungsbereich 170 der Membran 110 kann eine durchgehende Unterbrechung 172 aufweisen, deren Längsausdehnung 174 so groß ist, dass sie den Bereich zwischen der zweiten Thermoelement-Struktur 140 und dem Heizelement 120 vollständig abdeckt. Somit ist die Längsausdehnung 164, 174 beispielsweise so groß wie die komplette Länge des Heizelements 120 und/oder mindestens so lang wie die komplette Länge der ersten Thermoelement-Struktur 130 und/oder der zweiten Thermoelement-Struktur 140. Somit wird ermöglicht, dass möglichst wenig Wärme von dem Heizelement 120 zu der ersten Thermoelement-Struktur 130 oder der zweiten Thermoelement-Struktur 140 über die Membran 110 übertragen wird, sondern ein Großteil über ein Gas in der ersten Unterbrechung 162 in dem ersten Unterbrechungsbereich 160 und/oder in der zweiten Unterbrechung 172 in dem zweiten Unterbrechungsbereich 170.

Gemäß einem Ausführungsbeispiel kann die Querausdehnung 166 der zumindest einen Unterbrechung 162 des ersten Unterbrechungsbereichs 160 unterschiedlich zu der Querausdehnung 176 der zumindest einen Unterbrechung 172 des zweiten Unterbrechungsbereichs 170 sein. Die Querausdehnung 166, 176 der ersten Unterbrechung 162 bzw. der zweiten Unterbrechung 172 kann beispielsweise in einer Richtung senkrecht zu einer Richtung maximaler Ausdehnung des Heizelements 120 bzw. in einer Richtung von dem Heizelement 120 zu den jeweiligen Thermoelement-Strukturen (z. B. der ersten Thermoelement-Struktur 130 und/oder der zweiten Thermoelement-Struktur 140) ausgerichtet sein. Gemäß Fig. 1a weisen die erste Unterbrechung 162 und die zweite Unterbrechung 172 beispielsweise die gleiche Querausdehnung 166, 176 auf.

Gemäß einem Ausführungsbeispiel kann die erste Unterbrechung 162 eine Längsausdehnung 164 und eine Querausdehnung 166 aufweisen, so dass die erste Unterbrechung 162 den Ausdehnungen des ersten Unterbrechungsbereichs 160 entspricht. Genauso kann die zweite Unterbrechung 172 beispielsweise eine Längsausdehnung 174 und eine Querausdehnung 176 aufweisen, so dass die zweite Unterbrechung 172 den Ausdehnungen des zweiten Unterbrechungsbereichs 170 entspricht. Somit kann beispielsweise der komplette erste Unterbrechungsbereich 160 die erste Unterbrechung 162 darstellen und der komplette Unterbrechungsbereich 170 die Unterbrechung 172.

Optional kann die Membran 110 aufseiten der kalten Enden 134, 144 der ersten Thermoelement-Struktur 130 und/oder der zweiten Thermoelement-Struktur 140 einen dritten und/oder einen vierten Unterbrechungsbereich aufweisen. Somit kann beispielsweise die erste Thermoelement-Struktur 130 in Form eines Drahtes oder einer freitragenden Brückenstruktur zwischen dem ersten Unterbrechungsbereich 160 und einem dritten Unterbrechungsbereich angeordnet sein bzw. die zweite Thermoelement-Struktur 140 kann beispielsweise als Draht oder als freitragende Brückenstruktur zwischen dem zweiten Unterbrechungsbereich 170 und dem vierten Unterbrechungsbereich angeordnet sein. Somit können die erste Thermoelement-Struktur 130 und/oder die zweite Thermoelement-Struktur 140 beispielsweise von zwei Seiten mit dem zu analysierenden Gas umgeben sein.

Gemäß einem Ausführungsbeispiel kann die erste Thermoelement-Struktur 130 einen anderen Abstand zu dem Heizelement 120 aufweisen, als die zweite Thermoelement-Struktur 140. In Fig. 1a weist die erste Thermoelement-Struktur 130 beispielsweise denselben Abstand zu dem Heizelement 120 auf, wie die zweite Thermoelement-Struktur 140. Bei der Übertragung des Heizer-Signals von dem Heizelement 120 über die erste Unterbrechung 162 zu der ersten Thermoelement-Struktur 130 bzw. von dem Heizelement 120 über die zweite Unterbrechung 172 zu der zweiten Thermoelement-Struktur 140 können unbekannte Wärmeübergänge von dem Heizelement in das zu analysierende Gas, angeordnet in der ersten Unterbrechung 162 und/oder der zweiten Unterbrechung 172, und von dem Gas zu der ersten Thermoelement-Struktur 130 und/oder der zweiten Thermoelement-Struktur 140 auftreten. Das von der ersten Thermoelement-Struktur 130 detektierte Heizer-Signal von dem Heizelement 120 kann beispielsweise als erstes Sensorsignal bezeichnet werden und das von der zweiten Thermoelement-Struktur 140 detektierte Heizer-Signal von dem Heizelement 120 kann beispielsweise als ein zweites Sensorsignal bezeichnet werden.

Das erste Sensorsignal und/oder das zweite Sensorsignal können beispielsweise die zwei unbekannten Wärmeübergänge (z. B.: Heizelement -> Gas, Gas -> Thermoelement-Struktur) und einen Wärmeübertrag über das zu analysierende Gas aufweisen. Ist die erste Thermoelement-Struktur 130 von dem Heizelement 120 anders beabstandet als die zweite Thermoelement-Struktur 140, so kann beispielsweise aus dem ersten Sensorsignal und dem zweiten Sensorsignal ein Differenzsignal von dem Gassensor erstellt werden, indem beispielsweise die unbekannten Wärmeübergänge (das erste Sensorsignal kann dieselben Wärmeübergänge wie das zweite Sensorsignal aufweisen) herausgerechnet sein können und somit das Differenzsignal nur oder zu einem großen Anteil den Wärmeübertrag über das zu analysierende Gas von dem Heizelement 120 zu der jeweiligen Thermoelement-Struktur 130, 140 aufweist, aber nicht oder zu einem sehr geringen Anteil die unbekannten Wärmeübergänge aufweist.

Gemäß einem Ausführungsbeispiel können der erste Unterbrechungsbereich 160 und der zweite Unterbrechungsbereich 170 mehrere Unterbrechungen (z. B. die Unterbrechung 162 und die Unterbrechung $162_1$ oder die Unterbrechung 172 und die Unterbrechung $172_1$) aufweisen, die so angeordnet sein können, dass (beispielsweise durch das verbliebene Membranmaterial 110) eine Gitterstruktur (beispielsweise in dem ersten Unterbrechungsbereich 160 oder dem zweiten Unterbrechungsbereich 170) entsteht, bei der die Unterbrechungen in Reihen parallel zu dem Heizelement 120 angeordnet sind und die Reihen gegeneinander versetzt angeordnet sind. Dabei können sich die Unterbrechungen in einem Unterbrechungsbereich 160, 170 sowohl in der Längsausdehnung 164, 174 als auch in der Querausdehnung 166, 176 voneinander unterscheiden. Gemäß Fig. 1a weist beispielsweise die Unterbrechung $162_1$ des ersten Unterbrechungsbereichs 160 eine kleinere Längsausdehnung als die Längsausdehnung 164 der Unterbrechung 162 auf. Genauso kann die Unterbrechung $172_1$ des zweiten Unterbrechungsbereichs 170 eine geringere Längsausdehnung aufweisen als die Längsausdehnung 174 der Unterbrechung 172.

Gemäß einem Ausführungsbeispiel können der erste Unterbrechungsbereich 160 und der zweite Unterbrechungsbereich 170 mehrere Unterbrechungen aufweisen, die so angeordnet sein können, dass eine Gitterstruktur entsteht, bei der ein Weg einer Wärmeleitung durch die Membran 110 länger ist als ein direkter Weg 122a, 122b. Der direkte Weg 122a, 122b kann beispielsweise ein gerader Weg senkrecht zu dem Heizelement 120, von dem Heizelement 120 zu der Thermoelement-Struktur 130, 140 sein. Der direkte Weg 122a, 122b kann dabei durch die Unterbrechungen 162 und $162_1$ bzw. durch die Unterbrechungen 172 und $172_1$ verlaufen, wodurch eine Wärmeleitung durch das zu analysierende Gas von der ersten Thermoelement-Struktur 130 und/oder der zweiten Thermoelement-Struktur 140 erfasst werden kann. Würde der direkte Weg 122a, 122b hingegen nur über die Membran 110 und nicht über das zu analysierende Gas erfolgen, so kann der Gassensor 100 keine aussagekräftige Analyse des Gases gewährleisten.

Gemäß einem Ausführungsbeispiel kann die zumindest eine Unterbrechung 162, 172 in dem ersten Unterbrechungsbereich 160 und in dem zweiten Unterbrechungsbereich 170 rechteckige Ausschnitte mit optional abgerundeten Ecken bilden. Es handelt sich hierbei beispielsweise um ein Langloch. Es kann sich hierbei beispielsweise auch um ovale Löcher handeln. Auch wenn in Fig. 1a die Unterbrechung 162 des ersten Unterbrechungsbereichs 160 sowie die Unterbrechung 172 des zweiten Unterbrechungsbereichs 170 als rechteckige Unterbrechungen (Löcher) dargestellt sind, können die Unterbrechungen beliebige Formen (wie z. B. dreiecksförmig, kreisförmig, quadratisch, polygonförmig, etc.) aufweisen. Die Formgebung der Unterbrechungen 162, 172 kann so angepasst werden, dass ein Wärmepfad von dem Heizelement zur ersten Thermoelement-Struktur 130 und/oder zu der zweiten Thermoelement-Struktur 140 über die Membran 110 möglichst lang ist und ein Pfad über das zu analysierende Gas einen sehr Weg darstellt. Somit wird ermöglicht möglichst viel Wärme über das zu analysierende Gas zu transportieren und nicht über die Membran 110, wodurch der Gassensor 100 sehr genau das Gas analysieren kann.

Gemäß einem Ausführungsbeispiel kann die zumindest eine Unterbrechung 162, 172 mindestens dreimal länger als breit sein. Somit kann beispielsweise die Längsausdehnung 164 der Unterbrechung 162 dreimal länger als die Querausdehnung 166 sein bzw. die Längsausdehnung 174 der Unterbrechung 172 kann dreimal länger als die Querausdehnung 176 sein. Somit stellt die Länge beispielsweise die Längsausdehnung 164, 174 und die Breite beispielsweise die Querausdehnung 166, 176 dar. Die Länge kann beispielsweise definiert sein, als eine Richtung parallel zu dem Heizelement 120 (bzw. zu einer Richtung maximaler Ausdehnung des Heizelements 120) und die Breite kann definiert sein als eine Richtung senkrecht zu dem Heizelement 120 (bzw. zu einer Richtung maximaler Ausdehnung des Heizelements 120).

Gemäß einem Ausführungsbeispiel können ein Abstand 168 zwischen mehreren Unterbrechungen 162, $162_1$ in dem ersten Unterbrechungsbereich 160 und ein Abstand 178 zwischen mehreren Unterbrechungen 172, $172_1$ in dem zweiten Unterbrechungsbereich 170 der kleinsten zu realisierenden Strukturbreite, die eine mechanisch haltbare Gitterstruktur ergibt, entsprechen. Der Abstand 168, 178 kann eine Breite von Stegen, zwischen zwei Unterbrechungen, bestehend aus Membranmaterial der Membran 110, definieren. Je geringer der Abstand 168, 178 ist, desto weniger Wärme wird über die Membran 110 von dem Heizelement 120 zu der ersten Thermoelement-Struktur 130 und/oder der zweiten Thermoelement-Struktur 140 übertragen und desto mehr Wärme wird über das zu analysierende Gas übertragen.

Gemäß einem Ausführungsbeispiel können die erste Thermoelement-Struktur 130 und die zweite Thermoelement-Struktur 140 mit einer Schutzschicht passiviert sein. Die Schutzschicht kann die erste Thermoelement-Struktur 130 sowie die zweite Thermoelement-Struktur 140 vor Beschädigungen durch das zu analysierende Gas schützen und somit mögliche Ungenauigkeiten des Gassensors bei der Gasanalyse durch Beschädigungen der ersten Thermoelement-Struktur 130 bzw. der zweiten Thermoelement-Struktur 140 vermeiden.

Gemäß einem Ausführungsbeispiel können das heiße Ende 132 der ersten Thermoelement-Struktur bis zu einem Rand des ersten Unterbrechungsbereichs 160 der Membran 110 reichen und das heiße Ende 142 der zweiten Thermoelement-Struktur 140 bis zu einem Rand des zweiten Unterbrechungsbereichs 170 der Membran 110 reichen. Der Abstand zwischen dem heißen Ende 132 und dem ersten Unterbrechungsbereich 160 bzw. der Abstand zwischen dem heißen Ende 142 und dem zweiten Unterbrechungsbereich 170 sollte beispielsweise nicht größer als 0,5 mm, 100 nm oder 10 μm betragen. Reicht beispielsweise die Unterbrechung 162 bzw. die Unterbrechung 142 bis zu diesem Rand heran, so weist die Membran 110 nur einen sehr geringen Abstand zwischen den jeweiligen heißen Enden und der jeweiligen Unterbrechung auf. Dadurch kann ermöglicht werden, dass das Membranmaterial der Membran 110 eine Detektion des Heizer-Signals durch die erste Thermoelement-Struktur 130 bzw. durch die zweite Thermoelement-Struktur 140 nicht oder nur geringfügig beeinträchtigt, wodurch der Gassensor 100 sehr genau das Gas analysieren kann.

Fig. 1b zeigt eine schematische Darstellung einer Auswerteanordnung 200, die hierin auch als Auswerteeinrichtung bezeichnet werden kann, für einen thermischen Gassensor 100 mit zumindest einem Heizer 120 und zwei in unterschiedlichen Abständen $180_1$, $180_2$ von dem Heizer 120 angeordneten Detektoren (ein erster Detektor 130 und ein zweiter Detektor 140). Der erste Detektor 130 kann von dem Heizer 120 mit dem Abstand $180_1$ beabstandet sein und der zweite Detektor 140 kann von dem Heizer 120 mit dem Abstand $180_2$ beabstandet sein. Die Auswerteanordnung 200 kann ausgelegt sein, um eine Information 210 über eine Amplitude eines Detektorsignals eines ersten Detektors 130, eine Information 220 über eine Amplitude eines Detektorsignals eines zweiten Detektors 140, eine Information 210 über eine erste Phasendifferenz zwischen einem Heizer-Signal und dem Detektorsignal des ersten Detektors 130 und eine Information 220 über eine zweite Phasendifferenz zwischen dem Heizer-Signal und dem Detektorsignal des zweiten Detektors 140 zu erhalten.

Gemäß einem Ausführungsbeispiel kann die Information 210 sowohl die Amplitude des Detektorsignals des ersten Detektors 130 als auch die erste Phasendifferenz zwischen dem Heizer-Signal und dem Detektorsignal des ersten Detektors 130 umfassen, genauso wie die Information 220 sowohl die Amplitude des Detektorsignals des zweiten Detektors 140 als auch die zweite Phasendifferenz zwischen dem Heizer-Signal und dem Detektorsignal des zweiten Detektors 140 umfassen kann. Es ist aber auch möglich, dass die Amplitude des Detektorsignals des jeweiligen Detektors (des ersten Detektors 130 bzw. des zweiten Detektors 140) getrennt von der ersten Phasendifferenz bzw. der zweiten Phasendifferenz von dem thermischen Gassensor an die Auswerteanordnung gesendet wird. Gemäß einem Ausführungsbeispiel ist es ebenso möglich, dass die Information 210 sowie die Information 220 nicht über getrennte Leitungen zu der Auswerteanordnung 200 übertragen werden, sondern beispielsweise über eine gemeinsame Leitung oder kabellos.

Gemäß einem Ausführungsbeispiel kann die Auswerteanordnung 200 ausgelegt sein, um als Zwischengröße abhängig von der Information 210, 220 über die Amplituden der Detektorsignale und abhängig von der Information 210, 220 über die erste Phasendifferenz und die zweite Phasendifferenz ein Kombinationssignal 230 zu bilden. Das Kombinationssignal 230 kann eine Amplitudeninformation und eine Phaseninformation des Detektorsignals des ersten Detektors 130 sowie des Detektorsignals des zweiten Detektors 140 zusammenfassen. Die Auswerteanordnung 200 kann ausgelegt sein, um eine Information 240 über eine Gaskonzentration oder eine Temperaturleitfähigkeit eines Fluids, beispielsweise eines Gases oder Gasgemisches, basierend auf dem Kombinationssignal 230 zu bestimmen. Diese Bestimmung kann die Auswerteanordnung 200 beispielsweise durchführen, ohne im weiteren Fortgang der Berechnungen, die in das Kombinationssignal 230 einfließenden Einzelinformationen 210, 220 noch einmal separat zu betrachten.

Die Amplitude des Detektorsignals kann beispielsweise direkt als Information 210, 220 von dem jeweiligen Detektor 130, 140 bereitgestellt werden. Die Information 210, 220 über die erste Phasendifferenz und zweite Phasendifferenz zwischen dem Heizer-Signal 122 und dem Detektorsignal des jeweiligen Detektors 130, 140 kann z. B. von dem thermischen Gassensor 100 bestimmt werden und an die Auswerteanordnung 200 übertragen werden.

Alternativ kann über die Information 210 bzw. die Information 220 das Detektorsignal des ersten Detektors 130 bzw. das Detektorsignal des zweiten Detektors 140 an die Auswerteanordnung 200 übertragen werden und zusätzlich kann das Heizer-Signal 122 direkt an die Auswerteanordnung 200 übertragen werden. Dabei kann die Auswerteanordnung ausgebildet sein, um aus dem Detektorsignal des ersten Detektors 130 und aus dem Detektorsignal des zweiten Detektors 140 die jeweilige Amplitude zu bestimmen und die erste Phasendifferenz sowie die zweite Phasendifferenz zu bestimmen, um abhängig von den so bestimmten Informationen das Kombinationssignal 230 zu bilden.

Dadurch dass die Auswerteanordnung 200 das Kombinationssignal 230 bildet, kann die Auswerteanordnung 200 mögliche Fehler des thermischen Gassensors 100 sehr leicht und viel schneller korrigieren, um die Information 240 über die Gaskonzentration und eine Temperaturleitfähigkeit zu erhalten, als wenn die Auswerteanordnung 200 die Information 210 über die Amplitude des Detektorsignals des ersten Detektors 130 und die erste Phasendifferenz sowie die Information 220 über die Amplitude des Detektorsignals des zweiten Detektors 140 sowie die zweite Phasendifferenz getrennt voneinander korrigiert. Das Kombinationssignal 230 kann somit erleichtern die Information 240 über die Gaskonzentration und die Temperaturleitfähigkeit des zu analysierenden Gases zu bestimmen sowie ermöglichen, Fehler erzeugt durch den thermischen Gassensor 100, zu unterdrücken oder zu reduzieren.

Gemäß einem Ausführungsbeispiel kann die Auswerteanordnung 200 ausgelegt sein, um eine Information über eine Heizer-Amplitude, beispielsweise eine Information über eine Heizleistung aus dem Heizer-Signal 122, zu erhalten und um eine Linearkombination der Information über die Heizer-Amplitude, der Information 210 und der Information 220 zu bilden, um das Kombinationssignal 230 zu bestimmen.

Alternativ kann die Auswerteanordnung 200 aus dem Heizer-Signal 122 nicht nur die Information über die Heizer-Amplitude erhalten, sondern auch, wie bereits oben beschrieben, eine Information über die erste Phasendifferenz sowie über die zweite Phasendifferenz berechnen, wenn die Information 210 beispielsweise das Detektorsignal des ersten Detektors 130 und die Information 220 das Detektorsignal des zweiten Detektors 140 umfasst.

Somit geht in das Kombinationssignal 230 nicht nur die Phase des Heizer-Signals in Form der ersten Phasendifferenz und der zweiten Phasendifferenz ein, sondern zusätzlich die Heizer-Amplitude, die ermöglicht, dass die Auswerteanordnung 200 in Abhängigkeit des ersten Abstands $180_1$ und des zweiten Abstands $180_2$ der zwei Detektoren von dem Heizer 120, die Information 240 über die Gaskonzentration und die Temperaturleitfähigkeit des zu analysierenden Gases bestimmen kann. So weist beispielsweise das Detektorsignal des ersten Detektors 130 eine größere Amplitude auf als das Detektorsignal des zweiten Detektors 140, da der Abstand $180_2$ des zweiten Detektors 140 von dem Heizer 120 größer ist als der Abstand $180_1$ des ersten Detektors 130 von dem Heizer 120. Mit zunehmenden Abstand von dem Heizer 120 kann die Heizer-Amplitude, detektiert von dem jeweiligen Detektor 130, 140 abnehmen. Durch die zusätzliche Information über die Heizer-Amplitude kann die Auswerteanordnung 200 somit noch genauer die Information 240 über die Gaskonzentration und die Temperaturleitfähigkeit bestimmen, da die Heizer-Amplitude des Heizer-Signals 122 als Referenz betrachtet werden kann, und somit das Kombinationssignals 230 ein relatives Amplituden-Signal aufweisen kann. Ein relatives Amplituden-Signal ist beispielsweise weniger fehleranfällig als ein absolutes Amplituden-Signal.

Gemäß einem Ausführungsbeispiel kann die Auswerteanordnung 200 ausgelegt sein, um das Kombinationssignal sigX 230 gemäß sigX=sigUss*Ka+sigPhi*Kp zu erhalten. Der Term sigUss kann eine Amplitudeninformation oder ein Amplituden-Signal sein, das von der Information 210 über die Amplitude des Detektorsignals des ersten Detektors 130 und von der Information 220 über die Amplitude des Detektorsignals des zweiten Detektors 140 abhängig sein kann. So kann sigUss beispielsweise eine Linearkombination der Information 210 über die Amplitude des Detektorsignals des ersten Detektors 130 und der Information 220 über die Amplitude des Detektorsignals des zweiten Detektors 140 sein. sigPhi kann eine Phaseninformation oder ein addiertes Phasensignal sein, das von der Information 210 über eine ersten Phasendifferenz und von der Information 220 über die zweite Phasendifferenz abhängig sein kann. So kann sigPhi beispielsweise eine Addition der Information 210 über die erste Phasendifferenz und der Information 220 über die zweite Phasendifferenz sein. Ka und Kp können Konstanten sein. Das so bestimmte Kombinationssignal 230 kann eine Amplitudeninformation sigUss und eine Phaseninformation sigPhi umfassen, wodurch vier Informationen (z. B. die Information 210 über die Amplitude des Detektorsignals des ersten Detektors 130, die Information 220 über die Amplitude des Detektorsignals des zweiten Detektors 140, die Information 210 über eine erste Phasendifferenz zwischen dem Heizer-Signal und dem Detektorsignal des ersten Detektors 130 und die Information 220 über die zweite Phasendifferenz zwischen dem Heizer-Signal und dem Detektorsignal des zweiten Detektors 140) in dem Kombinationssignal 230 zusammengefasst werden, wodurch die Auswerteanordnung 200 weniger Leistung auf die Verarbeitung der Informationen 210, 220 verwenden kann. Somit kann die Auswerteanordnung 200 ausgelegt sein, um sehr effizient, schnell und genau eine Information 240 über die Gaskonzentration und Temperaturleitfähigkeit zu bestimmen.

Gemäß einem Ausführungsbeispiel kann die Auswerteanordnung 200 ausgelegt sein, um die Amplitudeninformation sigUss gemäß sigUss=2*Hz.Uss-(D1.Uss+D2.Uss) zu erhalten. Hz.Uss kann eine Information über die Heizer-Amplitude sein, die aus dem Heizer-Signal 122 erhalten werden kann. D1.Uss kann eine Information 210 über die Amplitude des Detektorsignals des ersten Detektors 130 sein und D2.Uss kann eine Information 220 über die Amplitude des Detektorsignals des zweiten Detektors 140 sein. Somit kann die Amplitudeninformation sigUss ein relatives Amplituden-Signal darstellen, da die Information 210 über die Amplitude des Detektorsignals des ersten Detektors 130, die Information 220 über die Amplitude des Detektorsignals des zweiten Detektors 140 und die Heizer-Amplitude Hz.Uss miteinander verrechnet werden, so dass die Information 210 über die Amplitude des Detektorsignals des ersten Detektors 130 und die Information 220 über die Amplitude des Detektorsignals des zweiten Detektors 140 relativ zu der Heizer-Amplitude betrachtet werden können. Durch die relative Betrachtungsweise der Amplituden können mögliche Fehler von absoluten Amplitudenwerten vermieden werden, wodurch die Auswerteanordnung 200 sehr genau die Information 240 über die Gaskonzentration und Temperaturleitfähigkeit bestimmen kann.

Gemäß einem Ausführungsbeispiel kann die Auswerteanordnung 200 ausgelegt sein, um ein Polynom, beispielsweise ersten Grades, des Kombinationssignals 230 zu berechnen, um die Information 240 über die Gaskonzentration oder die Temperaturleitfähigkeit zu erhalten. Das Polynom (z. B. Polynom y) kann beispielsweise gemäß $y=A0+A1*sigX+A2*sigX^2$ erhalten werden. Durch die Polynombildung des Kombinationssignals 230 durch die Auswerteanordnung 200 kann das Kombinationssignal 230 sehr leicht und effizient um mögliche Druck-Drift bzw. Temperatur-Drift-Fehler korrigiert werden.

Gemäß einem Ausführungsbeispiel kann die Auswerteanordnung 200 ausgelegt sein, um das Polynom des Kombinationssignals 230 mit einem Korrekturterm zu multiplizieren, um die Information 240 über die Gaskonzentration und/oder die Temperaturleitfähigkeit zu erhalten. Der Korrekturterm von dem Kombinationssignal 230, kann von einer Information über einen Druck und von einer Information über eine Temperatur abhängig sein und beispielsweise eine Druck- und Temperaturabhängigkeit kompensieren. In anderen Worten kann der Korrekturterm von dem Kombinationssignal 230 eine Druck-Drift und/oder eine Temperatur-Drift kompensieren. Somit wird eine mögliche Fehlinterpretation durch die Auswerteanordnung 200 der von dem thermischen Gassensor 100 detektierten Signale reduziert.

Gemäß einem Ausführungsbeispiel kann die Auswerteanordnung 200 ausgelegt sein, um eine Berechnung

$$C = pol(sigX) \cdot \left(1 - \left[\frac{f(p)}{sigX-const1}\right] \cdot \left(1 - \left[\frac{f(T)}{p-const2}\right]\right)\right)$$

gemäß durchzuführen, um die Information C 240 über die Gaskonzentration zu erhalten. sigX kann das Kombinationssignal 230 sein, pol(sigX) kann ein Polynom des Kombinationssignals sigX 230 sein, f(p) kann eine Funktion eines Drucks p sein, const1 kann eine Konstante sein, f(T) kann eine Funktion der Temperatur T sein und const2 kann eine zweite Konstante sein. f(p) kann eine Funktion eines gemessenen Drucks p in einer Umgebung des thermischen Gassensors 100 sein und f(T) kann eine Funktion einer gemessenen Temperatur T in einer Umgebung des thermischen Gassensors 100 sein. Der zweite Term der Multiplikation

$$\left(1 - \left[\frac{f(p)}{sigX-const1}\right] \cdot \left(1 - \left[\frac{f(T)}{p-const2}\right]\right)\right)$$

kann auch als Korrekturterm des Kombinationssignals 230 verstanden werden. Der Korrekturterm kann von Messbedingungen des Gassensors 100 (wie z. B. ein Umgebungsdruck/Messdruck oder einer Umgebungstemperatur/Messtemperatur) abhängig sein. Der Korrekturterm kann somit mögliche Einflüsse eines Umgebungsdrucks oder einer Umgebungstemperatur des thermischen Gassensors 100 auf die Bestimmung der Information 240 über die Gaskonzentration korrigieren. Somit kann mögliche Druck-Drift bzw. Temperatur-Drift unterdrückt werden.

Gemäß einem Ausführungsbeispiel kann die Auswerteanordnung 200 ausgelegt sein, um eine Berechnung

$$C[vol\%] = A.y(sigX) \cdot \left(1 - \left[\frac{B.y(p)-B.ref}{sigX-B.ref}\right] \cdot \left(1 - \left[\frac{C.y(T)-C.ref}{p-C.ref}\right]\right)\right)$$

gemäß durchzuführen, um die Information C 240 über die Gaskonzentration zu erhalten. In der Formel kann das sigX das Kombinationssignal 230 sein, A.y(sigX) kann ein Polynom des Kombinationssignals sigX 230 (beispielsweise erster Ordnung) sein, B.y(p) kann eine Funktion des Drucks p (beispielsweise eine Polynomfunktion, beispielsweise zweiter Ordnung) sein, B.ref kann eine Konstante sein, C.y(T) kann eine Funktion der Temperatur T (beispielsweise eine Polynomfunktion, beispielsweise zweiter Ordnung) sein und C.ref kann eine zweite Konstante sein. Die Funktion B.y(p) kann beispielsweise eine Funktion eines gemessenen Drucks p in einer Umgebung des thermischen Gassensors 100 sein und die Funktion C.y(T) kann eine Funktion einer gemessenen Temperatur T in einer Umgebung des thermischen Gassensors 100 sein. Der zweite

$$\left(1 - \left[\frac{B.y(p)-B.ref}{sigX-B.ref}\right] \cdot \left(1 - \left[\frac{C.y(T)-C.ref}{p-C.ref}\right]\right)\right)$$

Term der Multiplikation zur Berechnung der Information C 240 über die Gaskonzentration kann einen Korrekturterm definieren. Der Korrekturterm kann hierbei beispielsweise von dem Druck p und der Temperatur T abhängen. So kann beispielsweise B.y(p) eine Polynomfunktion abhängig von dem Druck p sein, wodurch beispielsweise eine Korrektur von möglichen Druckeinflüssen auf die Berechnung der Information 240 über die Gaskonzentration berücksichtigt werden kann. Genauso kann durch die Bildung der Polynomfunktion C.y(T), als Funktion der Temperatur T, sehr genau ein möglicher Einfluss der Temperatur T auf die Berechnung der Information 240 über die Gaskonzentration berücksichtigt werden. Durch die Bildung der Polynomfunktionen, sowohl als Funktion des Drucks p als auch als Funktion der Temperatur T, können Fehlerkorrekturen sehr gut angenähert werden, wodurch die Auswerteanordnung 200 ausgelegt sein kann, um sehr effektiv und sehr genau die Information 240 über die Gaskonzentration zu bestimmen.

Gemäß einem Ausführungsbeispiel kann die Auswerteanordnung 200 ausgelegt sein, um einen Druck und/oder eine Temperatur in einer Umgebung des thermischen Gassensors 100 bei der Bestimmung der Information 240 über die Gaskonzentration und/oder die Temperaturleitfähigkeit zu berücksichtigen. Hierfür kann beispielsweise der thermische Gassensor 100 Druck- und Temperatursensoren aufweisen, mit denen er den Druck und/oder die Temperatur in der Umgebung detektieren kann und an die Auswerteanordnung 200 übertragen kann. Somit kann die Auswerteanordnung 200 beispielsweise mögliche Fehlberechnungen der Information 240 über die Gaskonzentration und/oder die Temperaturleitfähigkeit aufgrund unterschiedlicher Druck und/oder Temperaturbedingungen in der Umgebung des thermischen Gassensors 100 berücksichtigen und korrigieren. Somit kann die Auswerteanordnung 200 auf den Druck und/oder die Temperatur in der Umgebung des thermischen Gassensors 100 reagieren und dementsprechend die Information 240 über die Gaskonzentration und/oder die Temperaturleitfähigkeit sehr genau bestimmen.

Gemäß einem Ausführungsbeispiel der vorliegenden Erfindung kann die Auswerteanordnung 200 ausgelegt sein, um bei der Bestimmung der Information 240 über die Gaskonzentration und/oder die Temperaturleitfähigkeit als Eingangsgrößen einer Drift-Korrektur das Kombinationssignal 230, eine Information über die Temperatur in einer Umgebung des thermischen Gassensors 100 und eine Information über einen Druck in einer Umgebung des thermischen Gassensors 100 zu verwenden, um die Information über die Gaskonzentration und/oder Temperaturleitfähigkeit als Ergebnis der Drift-Korrektur zu erhalten. Somit kann beispielsweise die Drift-Korrektur abhängig von der Information über die Temperatur und den Druck auf das Kombinationssignal angewendet werden, um die Information 240 über die Gaskonzentration und/oder die Temperaturleitfähigkeit zu erhalten. Die Drift-Korrektur kann beispielsweise außer den drei genannten Eingangsvariablen (das Kombinationssignal, die Information über die Temperatur und die Information über den Druck) keine weiteren Variablen mehr erhalten, sondern nur noch vorher erhaltene, beispielsweise im Rahmen einer Kalibration bestimmte, Konstanten verwenden. Die Konstanten können dabei spezifisch für den verwendeten thermischen Gassensor 100 sein. Somit kann die Auswerteanordnung 200 ausgelegt sein, um kleine Unterschiede zwischen thermischen Gassensoren 100 in der Berechnung der Information 240 über die Gaskonzentration und/oder Temperaturleitfähigkeit zu berücksichtigen, um ein sehr genaues Ergebnis (Information 240) zu erhalten. Die Drift-Korrektur kann beispielsweise eine Temperatur-Drift und/oder eine Druck-Drift korrigieren.

Fig. 1c zeigt eine schematische Darstellung einer beispielhaften Auswerteanordnung 200 für einen thermischen Gassensor 100 mit zumindest einem Heizer 120 und zwei Detektoren (einem ersten Detektor 130 und einem zweiten Detektor 140). Der erste Detektor 130 kann einen ersten Abstand $180_1$ zu dem Heizer 120 aufweisen und der zweite Detektor 140 kann einen zweiten Abstand $180_2$ zu dem Heizer 120 aufweisen. Gemäß Fig. 1c weisen der erste Detektor 130 und der zweite Detektor 140 den gleichen Abstand $180_1$, $180_2$ zu dem Heizer 120 auf. Es ist aber auch möglich, dass der erste Abstand $180_1$ sich von dem zweiten Abstand $180_2$ unterscheidet. So kann beispielsweise der erste Detektor 130 in einem anderen Abstand als der zweite Detektor 140 von dem Heizer 120 angeordnet sein. Die Auswerteanordnung 200 kann ausgelegt sein, um eine Heizleistung, mit der der Heizer 120 beaufschlagt werden kann, abhängig von zumindest einem Sensorsignal (z. B. einem ersten Sensorsignal 210 und/oder einem zweiten Sensorsignal 220) von zumindest einem der Detektoren (z. B. dem ersten Detektor 130 und/oder dem zweiten Detektor 140) zu regeln (beispielsweise mit einer Regeleinheit 250 zur Regelung einer Heizleistung), um das zumindest eine Sensorsignal 210, 220 in einen vorbestimmten Wertebereich zu bringen.

Um das zumindest eine Sensorsignal 210, 220 durch die Auswerteanordnung beispielsweise zu analysieren bzw. weiterzuverarbeiten, ist es von Vorteil, wenn das zumindest eine Sensorsignal 210, 220 in den vorbestimmten Wertebereich durch die Auswerteanordnung 200 gebracht wird. Wird die Heizleistung beispielsweise erhöht, so kann beispielsweise eine Amplitude oder eine Frequenz des zumindest einen Sensorsignals 210, 220 ebenfalls erhöht werden. Dies kann beispielsweise durch die Auswerteanordnung 200 durchgeführt werden, wenn das zumindest eine Sensorsignal 210, 220 zu klein ist und der vorbestimmte Wertebereich sehr groß ist. Somit kann das neue Sensorsignal 210, 220 nach der Regelung der Heizleistung durch die Regeleinheit 250 den vorbestimmten Wertebereich ausfüllen bzw. in diesem liegen. Der vorbestimmte Wertebereich kann beispielsweise abhängig von verwendeten Bauteilen der Auswerteanordnung 200, wie z. B. einem Analog-Digital-Wandler (ADC), abhängig sein. So kann der ADC beispielsweise das zumindest eine Sensorsignal 210, 220 sehr gut weiterverarbeiten, wenn das zumindest eine Sensorsignal 210, 220 in dem auf den ADC angepassten vorbestimmten Wertebereich angepasst ist (z. B. ADC Arbeitsbereich).

Die Auswerteanordnung 200 kann auch ausgelegt sein, um die Heizleistung des Heizers 120 mit der Regeleinheit 250 dahin gehend zu regeln, dass die Heizleistung des Heizers 120 reduziert wird. Dadurch kann das zumindest eine Sensorsignal 210, 220 ebenfalls reduziert werden. Dies kann beispielsweise vorteilhaft sein, wenn das zumindest eine Sensorsignal 210, 220 den vorbestimmten Wertebereich überschreitet, also zu groß ist. Dadurch, dass die Auswerteanordnung 200 ausgelegt ist, um mit der Regeleinheit 250 die Heizleistung des Heizers 120 zu regeln, wird ermöglicht, dass bei einer Weiterverarbeitung des zumindest einen Sensorsignals 210, 220 durch beispielsweise Bauelemente der Auswerteanordnung 200, wie z. B. dem ADC, keine oder nur kaum Informationen des zumindest einen Sensorsignals 210, 220 verlorengehen.

Gemäß einem Beispiel kann die Regeleinheit 250 der Auswerteanordnung 200 zur Regelung der Heizleistung des Heizers 120 ein Regelungssignal 252 an den Heizer 120 übertragen. Zusätzlich kann die Regeleinheit 250 eine Information 122 über die geregelte Heizleistung des Heizers 120 der Auswerteanordnung 200 bereitstellen.

Die Auswerteanordnung 200 kann ausgelegt sein, um eine Information 122 über die Heizleistung bei einer Ableitung einer Information 240 über eine Gaskonzentration und/oder eine Temperaturleitfähigkeit von dem zumindest einen Sensorsignal 210, 220 zu berücksichtigen. Somit kann ermöglicht werden, dass durch die Regeleinheit 250 das Sensorsignal 210, 220 in den vorbestimmten Wertebereich gebracht wird und zusätzlich die Information 122 über die Heizleistung in der Analyse berücksichtigt wird, da das zumindest eine Sensorsignal 210, 220 von der Heizleistung abhängig ist. Zusätzlich ermöglicht diese Auswerteanordnung 200, dass ein Sensorsignal, z. B. das erste Sensorsignal 210 oder das zweite Sensorsignal 220, ausreichend sein kann, um mit einer gewissen Genauigkeit die Information 240 über die Gaskonzentration und/oder die Temperaturleitfähigkeit eines Gases oder Fluids (z. B. eines Gases oder Gasgemisches) abzuleiten. Wird sowohl das erste Sensorsignal 210 als auch das zweite Sensorsignal 220 und die Heizleistung 122 verwendet, um die Information 240 abzuleiten, so ist die Bestimmung der Information 240 überbestimmt, wodurch die Information 240 sehr genau durch die Auswerteanordnung 200 bestimmt werden kann. Unterscheidet sich der erste Abstand $180_1$ des ersten Detektors 130 von dem zweiten Abstand $180_2$ des zweiten Detektors 140 von dem Heizer 120, so kann beispielsweise die Information 240 über die Gaskonzentration und/oder die Temperaturleitfähigkeit eines Gases auch nur aus dem ersten Sensorsignal 210 und dem zweiten Sensorsignal 220 abgeleitet werden, ohne die Information 122 über die Heizleistung des Heizers 120.

Gemäß einem Beispiel kann die Auswerteanordnung 200 die Information 122 über die Heizleistung auch von dem thermischen Gassensor 100 erhalten anstelle von der Regelungseinheit 250.

Gemäß einem Beispiel kann die Auswerteanordnung 200 ausgelegt sein, um ein periodisches Signal (z. B. das Regelungssignal 252) an den Heizer 120 anzulegen. Bei dem periodischen Signal kann es sich beispielsweise um ein periodisches Rechtecksignal oder Sinussignal handeln. Ist das Regelungssignal 252, und damit die von dem Heizer 120 an das zu analysierende Gas abgegebene Wärme, ein periodisches Signal, so kann ebenfalls das von dem ersten Detektor 130 detektierte erste Sensorsignal 210 und das von dem zweiten Detektor 140 detektierte zweite Sensorsignal 220 periodisch sein. Aufgrund des ersten Abstands $180_1$ und des zweiten Abstands $180_2$ können allerdings das erste Sensorsignal 210 und/oder das zweite Sensorsignal 220 sich in der Phase von dem periodischen Signal des Heizers 120 als auch in der Amplitude von dem periodischen Signal des Heizers 120 unterscheiden. Diese Unterschiede kann beispielsweise die Auswerteanordnung 200 nutzen, um die Information 240 über die Gaskonzentration und/oder die Temperaturleitfähigkeit sehr genau zu bestimmen.

Gemäß einem Beispiel kann die Auswerteanordnung 200 ausgelegt sein, um die Heizleistung, mit der der Heizer 120 beaufschlagt wird, (beispielsweise durch das Regelungssignal 252) zwischen zwei Werten umzuschalten. Somit kann beispielsweise an den Heizer 120 ein periodisches Rechtecksignal angelegt sein. Somit kann der Heizer 120 z. B. abwechselnd eine erste Heizleistung und eine zweite Heizleistung an das zu analysierende Gas übertragen.

Gemäß einem Beispiel kann die Auswerteanordnung 200 ausgelegt sein, um eine Amplitude der Heizleistung so zu regeln (beispielsweise mit der Regelungseinheit 250), dass sowohl ein Minimalwert des zumindest einen Sensorsignals 210, 220 als auch ein Maximalwert des zumindest einen Sensorsignals 210, 220 in dem vorbestimmten Wertebereich liegt. Wird die Amplitude der Heizleistung des Heizers 120 beispielsweise durch das Regelungssignal 252 erhöht, so kann der Minimalwert des zumindest einen Sensorsignals 210, 220 gesenkt werden und der Maximalwert des zumindest einen Sensorsignals 210, 220 beispielsweise erhöht werden. Wird die Amplitude der Heizleistung durch das Regelungssignal 252 beispielsweise verringert, so kann der Minimalwert des zumindest einen Sensorsignals 210, 220 erhöht werden und der Maximalwert des zumindest einen Sensorsignals 210, 220 gesenkt werden.

Gemäß einem Beispiel kann der vorbestimmte Wertebereich abhängig von einem Wertebereich eines Bauteils, wie z. B. einem ADC, der Auswerteanordnung 200 sein. Somit kann beispielsweise der vorbestimmte Wertebereich abhängig von einem Bauteilwertebereich (beispielsweise eines Bauteils der Auswerteanordnung 200) bestimmt werden. So kann der vorbestimmte Wertebereich beispielsweise vorgeben, dass der Minimalwert des zumindest einen Sensorsignals 210, 220 beispielsweise in einem Bereich von 0% bis 30%, 1% bis 25% oder 2% bis 20% des Bauteilwertebereichs liegen soll und dass der Maximalwert des zumindest einen Sensorsignals 210, 220 in einem Bereich von 70% bis 100%, 75% bis 99% oder 80% bis 98% des Bauteilwertebereichs liegen soll. Somit kann der vorbestimmte Wertebereich beispielsweise einen unteren Wertebereich, in dem der Minimalwert liegen soll und einen oberen Wertebereich, in dem der Maximalwert liegen soll, aufweisen.

Gemäß einem Beispiel kann die Auswerteanordnung 200 ausgelegt sein, um eine Amplitude der Heizleistung so einzustellen oder einzuregeln (beispielsweise mit der Regelungseinheit 250), dass ein Amplitude des zumindest einen Sensorsignals 210, 220 in einem vorgegebenen Amplitudenbereich liegt. Weist das zumindest eine Sensorsignal 210, 220 beispielsweise ein periodisches Sinussignal auf, so sollte die Amplitude zu jedem Zeitpunkt des Sensorsignals in dem vorgegebenen Amplitudenbereich liegen. Hierbei sollte die Amplitude des zumindest einen Sensorsignals den kompletten vorgegebenen Amplitudenbereich ausnutzen. Der vorgegebene Amplitudenbereich kann beispielsweise einen oberen, mittleren und unteren Amplitudenbereich aufweisen/unterteilt sein. Damit der vorgegebene Amplitudenbereich durch die Amplitude des zumindest einen Sensorsignals ausgenutzt wird, sollte eine maximale Amplitude des zumindest einen Sensorsignals beispielsweise in dem oberen Bereich liegen und eine minimale Amplitude in dem unteren Bereich liegen. Der vorgegebene Amplitudenbereich kann beispielsweise von dem Bauteilbereich abhängig sein. So kann der vorgegebene Amplitudenbereich beispielsweise so bestimmt sein, dass die Amplitude des zumindest einen Sensorsignals beispielsweise zumindest 50% oder zumindest 65% oder zumindest 75% eines Bauteilwertebereichs eines beispielsweise Analog-Digital-Wandlers ausnutzt.

Gemäß einem Beispiel kann die Auswerteanordnung 200 ausgelegt sein, um Abtastzeitpunkte, zu denen ein Sensorsignal 210, 220 abgetastet werden kann, einzustellen oder einzuregeln. Das Sensorsignal 210, 220 kann optional beispielsweise von der Auswerteanordnung 200 oder dem thermischen Gassensor 100 vorverarbeitet und/oder mit einem DC-Offset beaufschlagt sein. Es kann vorteilhaft sein, wenn das Sensorsignal 210, 220 zu einem Zeitpunkt maximaler Amplitude und zu einem Zeitpunkt minimaler Amplitude abgetastet wird. Diese beide Abtastzeitpunkte können beispielsweise von der Auswerteanordnung 200 eingestellt bzw. nachgeregelt werden, falls die Auswerteanordnung 200 feststellt, dass die Abtastzeitpunkte falsch gewählt worden sind. Durch ein exaktes Einstellen der Abtastzeitpunkte kann ermöglicht werden, dass beispielsweise sehr leicht durch die Auswerteanordnung eine Phasendifferenz oder Amplitudendifferenz zwischen dem ersten Sensorsignal 210 und einem Heizer-Signal (beispielsweise ausgesendet von dem Heizer 120 und geregelt durch das Regelungssignal 252) bzw. zwischen dem zweiten Sensorsignal 220 und dem Heizer-Signal bestimmt werden können. Mittels der sehr genauen Phasendifferenz und/oder Amplitudendifferenzen kann die Auswerteanordnung 200 sehr genau die Information 240 über die Gaskonzentration und/oder Temperaturleitfähigkeit des zu analysierenden Gases bestimmen bzw. ableiten.

Gemäß einem Beispiel kann die Auswerteanordnung 200 ausgelegt sein, um die Abtastzeitpunkte so einzustellen, dass eine Abtastung, beispielsweise mit einer Phasendifferenz von maximal +/- 2° zu einem Zeitpunkt erfolgt, zu dem das Sensorsignal 210, 220 einen Maximalwert erreicht, und so dass die Abtastung, beispielsweise mit einer Phasendifferenz von maximal +/- 2° zu einem Zeitpunkt erfolgt, zu dem das Sensorsignal 210, 220 einen Minimalwert erreicht. Der Maximalwert kann beispielsweise eine maximale Amplitude des Sensorsignals 210, 220 definieren und der Minimalwert kann eine minimale Amplitude des Sensorsignals 210, 220 definieren, wie bereits weiter oben erklärt.

Gemäß einem Beispiel kann die Auswerteeinrichtung 200 ausgelegt sein, um ein Sensorsignal 210, 220 von zumindest einem der Detektoren 130, 140 mit einem durch einen Digital-Analog-Wandler erzeugten Offset-Signal zu kombinieren, um ein Eingangssignal für den Analog-Digital-Wandler zu erhalten. Die Auswerteeinrichtung 200 kann ausgelegt sein, um das Offset-Signal einzuregeln, um zu erreichen, dass das Eingangssignal des Analog-Digital-Wandlers während einer gesamten Periode des Sensorsignals 210, 220 innerhalb eines vorgegebenen Bereichs bleibt. Somit kann das Offset-Signal beispielsweise ausgelegt sein, um das Sensorsignal 210, 220 so anzupassen, dass das Eingangssignal entsteht, das in einem Bauteilwertebereich des Analog-Digital-Wandlers liegt. So kann das Offset-Signal beispielsweise eingeregelt/angepasst werden, um auf unterschiedliche Sensorsignale 210, 220 von unterschiedlichen zu analysierenden Gasen reagieren zu können. So kann das Offset-Signal beispielsweise ausgebildet sein, um ein zu großes Sensorsignal 210, 220 zu verkleinern, damit das so entstandene Eingangssignal in dem vorgegebenen Bereich liegt. Außerdem kann das Offset-Signal ausgebildet sein, um bei einem zu kleinen Sensorsignal 210, 220 das Sensorsignal 210, 220 zu erhöhen, damit ein Eingangssignal entsteht, das in dem vorgegebenen Bereich liegt.

Somit kann die Auswerteanordnung 200 einerseits ausgelegt sein, um die Amplitude des Sensorsignals 210, 220 durch die Regelung der Heizleistung in den vorbestimmten Wertebereich zu bringen und durch die Kombination des Sensorsignals 210, 220 mit dem Offset-Signal einen Offset des Sensorsignals 210, 220 so zu verändern, dass das Sensorsignal 210, 220 in dem vorbestimmten Wertebereich liegt. Hierdurch wird ermöglicht, dass das Sensorsignal 210, 220 sehr genau analysiert werden kann und somit eine sehr genaue Information 240 über die Gaskonzentration und/oder Temperaturleitfähigkeit des zu analysierenden Gases durch die Auswerteanordnung 200 ermittelt werden kann.

Gemäß einem Beispiel kann die Auswerteeinrichtung 200 ausgelegt sein, um die Heizleistung nur dann zu regeln, wenn eine Einstellung oder Einregelung der Abtastzeitpunkte sich in einem eingeschwungenen Zustand befindet und wenn sich eine Einregelung des Offset-Signals in einem eingeschwungenen Zustand befindet. Unter einem eingeschwungenen Zustand kann verstanden werden, dass die Abtastzeitpunkte von der Auswerteeinrichtung 200 so bestimmt wurden, dass das Sensorsignal 210, 220 an vordefinierten Ereignissen (wie z. B. eine maximale Amplitude (Maximalwert), ein Nulldurchgang oder eine minimale Amplitude (Minimalwert)) abgetastet werden kann. Genauso kann der eingeschwungene Zustand bedeuten, dass das Offset-Signal so eingeregelt wurde, dass das Sensorsignal 210, 220 bei einer Kombination des Offset-Signals mit dem Sensorsignal 210, 220 ein Eingangssignal erzeugt, das in dem vorgegebenen Bereich liegt, um somit das Sensorsignal 210, 220 sehr genau, ohne oder nur mit geringen Informationsverlusten, mittels der Auswerteanordnung zu analysieren. Somit können beispielsweise von der Auswerteeinrichtung 200 Voreinstellungen (wie z. B. die Abtastzeitpunkte in dem eingeschwungenen Zustand oder das Offset-Signal in dem eingeschwungenen Zustand) bestimmt werden, so dass bei einer Regelung der Heizleistung durch die Regelungseinheit 250 das neue Sensorsignal 210, 220 mit den Voreinstellungen sehr genau analysiert werden kann und unter Umständen keine neue Regelung der Abtastzeitpunkte bzw. des Offset-Signals mehr nötig ist, um aus dem Sensorsignal 210, 220 die Information 240 über die Gaskonzentration und/oder die Temperaturleitfähigkeit abzuleiten.

Gemäß einem Beispiel kann die Auswerteanordnung 200 ausgelegt sein, um die Regelung der Heizleistung (beispielsweise durch die Regelungseinheit 250) anzuhalten, während eine Einstellung oder Einregelung der Abtastzeitpunkte erfolgt und/oder während eine Einregelung des Offset-Signals erfolgt. Somit kann beispielsweise sichergestellt werden, dass keine Veränderungen des Sensorsignals 210, 220 durchgeführt werden, während die Abtastzeitpunkte und das Offset-Signal sich noch nicht in einem eingeschwungenen Zustand befinden. Somit kann sichergestellt werden, dass das Sensorsignal 210, 220 sehr genau analysiert werden kann, da die Abtastzeitpunkte und das Offset-Signal sehr genau mit nur sehr geringer oder keiner Fehleranfälligkeit bestimmt werden können.

Gemäß einem Beispiel kann die Auswerteanordnung 200 ausgelegt sein, um sowohl eine mittlere Heizleistung oder eine maximale Heizleistung als auch eine Amplitude der Heizleistung zu regeln. Somit kann die Regelungseinheit 250 beispielsweise als Regelungssignal 252 ein neues Heizer-Signal für den Heizer 120 an den thermischen Gassensor 100 übertragen, wobei das Regelungssignal z. B. eine geänderte mittlere Heizleistung, maximale Heizleistung oder Amplitude der Heizleistung aufweist. Es ist aber auch möglich, dass das Regelungssignal 152 eine Information umfasst, die besagt, wie die mittlere Heizleistung, die maximale Heizleistung oder die Amplitude der Heizleistung von dem thermischen Gassensor für den Heizer 120 geändert werden sollte.

Fig. 1d zeigt eine schematische Darstellung einer beispielhaften Auswerteanordnung 200 für einen thermischen Gassensor 100 mit zumindest einem Heizer 120 und zwei in unterschiedlichen Abständen (z. B. einem ersten Abstand $180_1$ und einem zweiten Abstand $180_2$) von dem Heizer 120 angeordneten Detektoren (z. B. einem ersten Detektor 130 und einem zweiten Detektor 140). Der erste Detektor 130 kann beispielsweise von dem Heizer 120 den ersten Abstand $180_1$ aufweisen und der zweite Detektor 140 kann von dem Heizer 120 den zweiten Abstand $180_2$ aufweisen. Die Auswerteanordnung 200 kann ausgelegt sein, um ein periodisches Signal 260 mit einer vorgegebenen Periodendauer an den Heizer 120 anzulegen. Dabei kann es sich bei dem periodischen Signal beispielsweise um ein Rechtecksignal, ein Impulssignal mit bekannter Leistung oder ein Sinussignal handeln. Optional kann es sich auch um ein Sinussignal mit Oberschwingungen oder um ein Dreieckecksignal handeln. Das periodische Signal kann auch als Heizer-Signal bezeichnet werden und kann von dem Heizer 120 in Form von Wärme über ein zu analysierendes Gas an den ersten Detektor 130 und/oder an den zweiten Detektor 140 übertragen werden. Die übertragene Wärme kann von dem ersten Detektor 130 als erstes Sensorsignal 210 detektiert werden und von dem zweiten Detektor 140 als zweites Sensorsignal 220 detektiert werden. Das erste Sensorsignal 210 und das zweite Sensorsignal 220 können ein erstes periodisches Signal bzw. ein zweites periodisches Signal mit jeweils der vorgegebenen Periodendauer aufweisen. Dadurch wird ermöglicht, dass das zu analysierende Gas sehr genau bezüglich seiner Gaskonzentration und/oder Temperaturleitfähigkeit von dem thermischen Gassensor 100 bzw. der Auswerteanordnung 200 analysiert werden kann. Die Auswerteanordnung 200 kann ausgelegt sein, um zumindest ein Sensorsignal (z. B. das erste Sensorsignal 210 und/oder das zweite Sensorsignal 220) von einem der Detektoren 130, 140 zu drei Zeitpunkten abzutasten (beispielsweise durch eine Abtasteinrichtung 270). Ein zweiter Abtastzeitpunkt kann beispielsweise gegenüber einem ersten Abtastzeitpunkt um 90°, bezogen auf die Periodendauer (beispielsweise mit +/- 2°), zeitversetzt sein. Somit kann beispielsweise der zweite Abtastzeitpunkt gegenüber dem ersten Abtastzeitpunkt um 1/4 Periodendauer, 5/4 Periodendauern oder um 9/4 Periodendauern zeitversetzt sein. Ein dritter Abtastzeitpunkt kann gegenüber dem ersten Abtastzeitpunkt um 180° bzw. gegenüber dem zweiten Abtastzeitpunkt um 90°, bezogen auf die Periodendauer, zeitversetzt sein. Der erste Abtastzeitpunkt, der zweite Abtastzeitpunkt und der dritte Abtastzeitpunkt können eine Toleranz von +/- 2% aufweisen. Der dritte Abtastzeitpunkt kann also beispielsweise um 1/2 Periodendauer, 3/2 Periodendauern oder um 5/2 Periodendauern gegenüber dem ersten Abtastzeitpunkt zeitversetzt sein. Somit kann das Sensorsignal 210, 220 an exakt definierten Stellen abgetastet werden, wodurch sehr genau eine Information 240 über eine Gaskonzentration und/oder eine Temperaturleitfähigkeit aus dem Sensorsignal 210, 220 ermittelt werden kann. Die Auswerteanordnung 200 kann ausgelegt sein, um basierend auf drei Abtastwerten, die auf einer Abtastung des Sensorsignals zu dem ersten Abtastzeitpunkt, zu dem zweiten Abtastzeitpunkt und zu dem dritten Abtastzeitpunkt (beispielsweise durchgeführt mittels der Abtastvorrichtung 270) basieren, zu erkennen, ob ein erster Abtastwert und ein dritter Abtastwert einen Maximalwert und einen Minimalwert des Sensorsignals 210, 220 darstellen. Dies kann beispielsweise durch die Überprüfungsvorrichtung 280 erfolgen. Die Überprüfungsvorrichtung 280 kann dabei beispielsweise einen DC-Offset ignorieren und somit bis auf einen DC-Offset überprüfen, ob der erste Abtastwert beispielsweise einen Maximalwert darstellt und der dritte Abtastwert beispielsweise einen Minimalwert des Sensorsignals 210, 220 darstellt. Der zweite Abtastzeitpunkt kann somit beispielsweise ein "Nulldurchgang" des Sensorsignals 210, 220 sein und von der Überprüfungseinrichtung 280 mit in Betracht gezogen werden.

Der erste Abtastzeitpunkt, der zweite Abtastzeitpunkt und/oder der dritte Abtastzeitpunkt sowie das erste Sensorsignal 210 und das zweite Sensorsignal 220 können verwendet werden, um die Information 240 über die Gaskonzentration und/oder die Temperaturleitfähigkeit eines Gases, detektiert durch den thermischen Gassensor 100, zu bestimmen. Optional kann zusätzlich das Heizer-Signal 122 in die Bestimmung der Information 240 miteinfließen. Somit können beispielsweise eine Phasendifferenz zwischen dem ersten Sensorsignal 210 und dem zweiten Sensorsignal 220 sowie eine Amplitudendifferenz zwischen dem ersten Sensorsignal 210 und dem zweiten Sensorsignal 220 beispielsweise anhand der Abtastzeitpunkte/Abtastwerte bestimmt werden. Optional können auch eine Phasendifferenz und/oder eine Amplitudendifferenz zwischen dem ersten Sensorsignal 210 und dem Heizer-Signal 122 bzw. zwischen dem zweiten Sensorsignal 220 und dem Heizer-Signal 122 ermittelt werden. Aus den so ermittelten Phasendifferenzen und Amplitudendifferenzen kann die Information 240 über die Gaskonzentration und/oder Temperaturleitfähigkeit bestimmt werden.

Gemäß einem Beispiel kann die Auswerteanordnung 200 ausgelegt sein, um die Abtastzeitpunkte abhängig von einer Erkennung, ob der erste Abtastwert und der dritte Abtastwert einen Maximalwert und/oder einen Minimalwert des Sensorsignals 210, 220 darstellen, zu verändern. Dies kann beispielsweise mittels einer Abtastregelungseinrichtung 290 erfolgen. Somit können beispielsweise neue Abtastzeitpunkte bestimmt werden, wenn der erste Abtastwert und der dritte Abtastwert nicht einen Maximalwert und/oder einen Minimalwert des Sensorsignals 210, 220 entsprechen. Durch die Regelung der Abtastzeitpunkte kann sichergestellt werden, dass die Abtastwerte vorbestimmten Werten entsprechen. Stellt die Überprüfungseinrichtung 280 z. B. fest, dass Abweichungen außerhalb einer Toleranz auftreten (beispielsweise +/- 2°), so können die Abtastzeitpunkte durch die Abtastregelungseinrichtung 290 verändert/nachgeregelt werden.

Gemäß einem Beispiel kann die Auswerteanordnung 200 ausgelegt sein, um die Abtastzeitpunkte so einzustellen oder einzuregeln, dass der erste Abtastwert einen ersten Extremwert des Sensorsignals 210, 220, beispielsweise einen Maximalwert oder Minimalwert, darstellt und der dritte Abtastwert einen zweiten Extremwert, beispielsweise den Minimalwert oder den Maximalwert des Sensorsignals 210, 220 darstellt. Der zweite Abtastwert kann beispielsweise einen Mittelwert oder Gleichanteil des Sensorsignals 210, 220 darstellen, wie z. B. einen "Nulldurchgang".

Gemäß einem Beispiel kann die Auswerteanordnung 200 ausgelegt sein, um bei der Einstellung oder Einregelung der Abtastzeitpunkte eine Information über einen Zeitpunkt eines Durchgangs des Sensorsignals 210, 220 durch einen vorgegebenen Schwellwert zu berücksichtigen. Dieser Zeitpunkt kann beispielsweise der zweite Zeitpunkt sein, der beispielsweise einen Gleichanteil oder einen Mittelwert des Sensorsignals 210, 220 darstellen kann. So kann beispielsweise die Überprüfungseinrichtung 280 den zweiten Abtastzeitpunkt für die Überprüfung des ersten Abtastzeitpunktes und/oder des zweiten Abtastzeitpunktes verwenden. Stellt somit die Überprüfungseinrichtung 280 basierend auf dem zweiten Abtastzeitpunkt fest, dass der erste Abtastwert nicht einen Maximalwert oder Minimalwert des Sensorsignals 210, 220 entspricht und/oder der dritte Abtastwert nicht dem Minimalwert oder dem Maximalwert des Sensorsignals 210, 220 entspricht, so kann die Abtastregelungsvorrichtung 290 die Abtastzeitpunkte neu einstellen oder einregeln. Der vorgegebene Schwellwert kann beispielsweise einen "Nulldurchgang" (beispielsweise bis auf einen DC-Offset) definieren.

Gemäß einem Beispiel kann die Auswerteanordnung 200 ausgelegt sein, um zu überprüfen, ob ein zweiter Abtastwert zu dem zweiten Abtastzeitpunkt gleich einem Mittelwert aus dem Abtastwert zu dem ersten Abtastzeitpunkt und dem dritten Abtastwert zu dem dritten Abtastzeitpunkt ist und um abhängig von der Überprüfung zu erkennen, ob der erste Abtastwert und der dritte Abtastwert einen Maximalwert und einen Minimalwert des Sensorsignals darstellen. Der zweite Abtastwert sollte beispielsweise mit einer Toleranz von höchstens $\pm 1\%$ einer Differenz zwischen dem ersten Abtastwert und dem dritten Abtastwert oder gleich einem Mittelwert aus dem Abtastwert und dem zweiten Abtastwert sein. Ist dies nicht der Fall, so kann die Überprüfungsvorrichtung 280 erkennen, dass die Abtastzeitpunkte falsch gewählt sind. Da der erste Abtastwert einen ersten Extremwert darstellt und der dritte Abtastwert um 180° bezogen auf die Periodendauer zeitversetzt einen zweiten Extremwert des Sensorsignals 210, 220 darstellt, kann der zweite Abtastwert, genau zur halben Zeit zwischen dem ersten Abtastzeitpunkt und dem zweiten Abtastzeitpunkt liegen. Somit kann der zweite Abtastwert dem Mittelwert aus den anderen beiden Abtastwerten entsprechen. Somit kann dies eine effiziente und genaue Methode darstellen, um mithilfe der Überprüfungsvorrichtung 280 die Abtastwerte zu überprüfen.

Gemäß einem Beispiel kann die Auswerteanordnung 200 ausgelegt sein, um ein periodisches Rechtecksignal 260 mit einem Tastverhältnis von bevorzugt 50% an den Heizer 120 anzulegen. Es ist aber auch möglich, dass das periodische Rechtecksignal ein Tastverhältnis im Bereich von 5% bis 50%, 8% bis 48% oder 10% bis 45% aufweist. Das an den Heizer 120 angelegte periodische Rechtecksignal 260 kann eine Toleranz von +/- 2% aufweisen. Das Tastverhältnis gibt z. B. für eine periodische Folge von Impulsen ein Verhältnis einer Impulsdauer zu einer Periodendauer an.

Gemäß einem Beispiel kann die Auswerteanordnung 200 ausgelegt sein, um ein Sensorsignal 210, 220 mit einem durch einen Digital-Analog-Wandler erzeugten Offset-Signal zu kombinieren, um ein Eingangssignal für einen Analog-Digital-Wandler zu erhalten. Der Analog-Digital-Wandler kann beispielsweise die zu den Abtastzeitpunkten anliegenden Signalwerte (z. B. den ersten Abtastwert, den zweiten Abtastwert und/oder den dritten Abtastwert) digitalisieren und damit das Sensorsignal 210, 220 abtasten. Die Abtastvorrichtung 270 kann beispielsweise den Analog-Digital-Wandler aufweisen.

Gemäß einem Beispiel kann die Auswerteanordnung 200 ausgelegt sein, um das Offset-Signal einzuregeln, um zu erreichen, dass das Eingangssignal des Analog-Digital-Wandlers während einer gesamten Periode des Sensorsignals 210, 220 innerhalb eines vorgegebenen Bereichs bleibt. So kann das Offset-Signal beispielsweise einen Offset des Sensorsignals 210, 220 so verändern, dass ein Eingangssignal entsteht, das in einem Arbeitsbereich (z. B. dem vorgegebenen Bereich) des Analog-Digital-Wandlers liegt, damit keine Informationen des Sensorsignals 210, 220 bei der Digitalisierung verlorengehen bzw. damit ein Informationsverlust reduziert wird. So kann beispielsweise die Abtastvorrichtung 270 überprüfen, ob ein Eingangswert des Analog-Digital-Wandlers einen vorgegebenen oberen Schwellwert, beispielsweise des vorgegebenen Bereichs, überschreitet, oder einen vorgegebenen unteren Schwellwert, beispielsweise des vorgegebenen Bereichs, unterschreitet. Dementsprechend kann die Abtastvorrichtung 270 das Offset-Signal erzeugen, das mit dem Sensorsignal 210, 220 kombiniert werden kann, damit der Eingangswert, beispielsweise ein Wert des Eingangssignals, in dem vorgegebenen Bereich bleibt. Die Auswerteanordnung 200 kann ausgelegt sein, um die Abtastzeitpunkte nach einer Einregelung des Offset-Signals einzuregeln und um nach einer Veränderung der Abtastzeitpunkte eine erneute Überprüfung durchzuführen, ob mit der veränderten Einstellung der Abtastzeitpunkte erhaltene Abtastwerte weiterhin innerhalb des vorgegebenen Bereichs liegen. Somit kann beispielsweise zunächst das Offset-Signal mittels der Auswerteanordnung 200 für das Sensorsignal 210, 220 erzeugt werden und daraufhin Abtastzeitpunkte mittels der Abtastvorrichtung 270 bestimmt werden, überprüft werden und ggf. nachgeregelt werden (dies kann beispielsweise eine Nachführung der Abtastzeitpunkte darstellen). Durch diese Nachführung können neue Abtastwerte entstehen, die eine erneute Einregelung des Offset-Signals durch die Auswerteanordnung 200 nach sich ziehen können. Somit können beispielsweise das Offset-Signal und die Abtastzeitpunkte immer abwechselnd so lange eingeregelt bzw. nachgeführt werden, bis beispielsweise der Analog-Digital-Wandler das Sensorsignal 210, 220 weiterverarbeiten kann. Somit können sich zu diesem Zeitpunkt das Offset-Signal und die Abtastzeitpunkte in einem eingeschwungenen Zustand befinden.

Die durch die Abtastregelungseinrichtung 290 veränderten Einstellungen der Abtastzeitpunkte erzeugen beispielsweise neue Abtastwerte, die als Eingangswerte des Analog-Digital-Wandlers angesehen werden können. Damit das Eingangssignal des Analog-Digital-Wandlers in dem vorgegebenen Bereich bleibt, kann sowohl das Offset-Signal als auch die Heizleistung des Heizers 120 nachgeregelt werden. Das Offset-Signal kann beispielsweise einen Offset des Sensorsignals 210, 220 anpassen und die Veränderung der Heizleistung kann eine Amplitude des Sensorsignals 210, 220 anpassen, so dass ein Eingangssignal entsteht, das in dem vorgegebenen Bereich liegt.

Gemäß einem Beispiel kann die Auswerteanordnung 200 ausgelegt sein, um eine Heizleistung, mit der der Heizer 120 beaufschlagt wird, abhängig von zumindest einem Sensorsignal 210, 220 von zumindest einem der Detektoren 130, 140 zu regeln, um das zumindest eine Sensorsignal 210, 220 in einen vorbestimmten Wertebereich zu bringen. Die Auswerteanordnung 200 kann ausgelegt sein, um eine Information über die Heizleistung (z. B. das Heizer-Signal 122) bei einer Ableitung einer Information 240 über eine Gaskonzentration und/oder Temperaturleitfähigkeit von den Sensorsignalen 210, 220 zu berücksichtigen. So kann beispielsweise bei einer Erhöhung der Heizleistung des Heizers 120 das Sensorsignal 210, 220 eine Erhöhung einer Amplitude des Sensorsignals 210, 220 erfahren bzw. bei einer Reduktion der Heizleistung kann das zumindest eine Sensorsignal 210, 220 eine Verkleinerung einer Amplitude des Sensorsignals 210, 220 erfahren. Somit kann beispielsweise das Sensorsignal 210, 220 durch eine Regelung der Heizleistung des Heizers 120 in den vorbestimmten Wertebereich gebracht werden.

Im Folgenden werden anhand weiterer Figuren Ausführungsbeispiele des thermischen Gassensors und der Auswerteanordnung beschrieben.

## 1.1 Technologie-Varianten für einen thermischen Gassensor

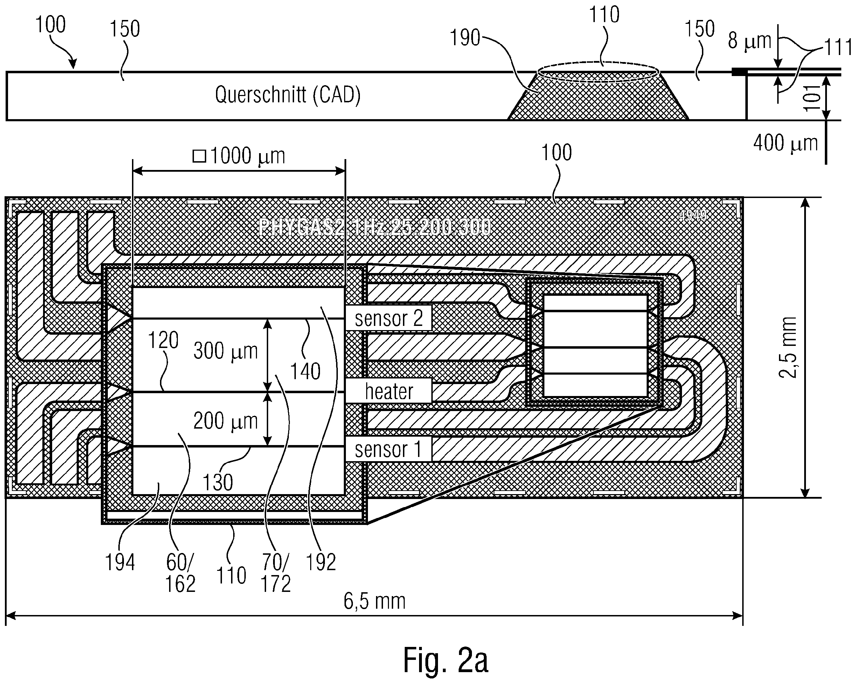

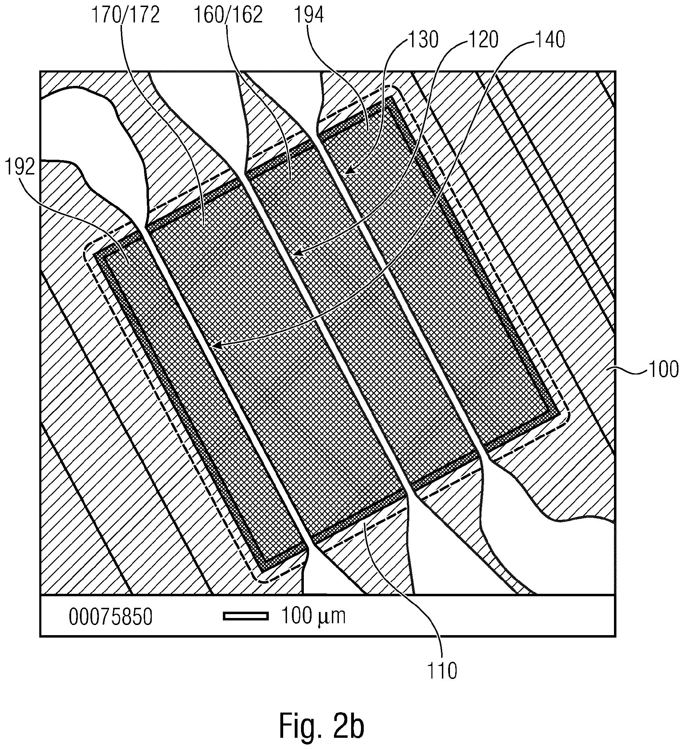

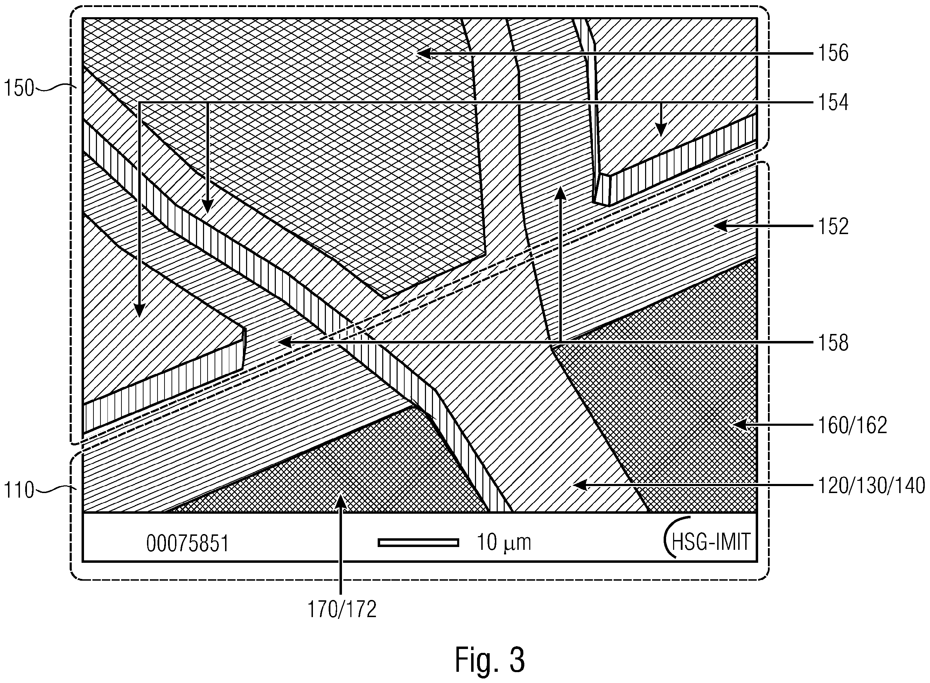

Fig. 2a und Fig. 2b zeigen jeweils eine schematische Darstellung eines Gassensors 100 zur Messung physikalischer Gaseigenschaften. Der thermische Gassensor 100 kann eine Dünnschicht-Membran 110 und ein Heizelement 120 aufweisen, das beispielsweise als freitragende Brückenstruktur auf der Membran 110 zwischen einem ersten Unterbrechungsbereich 160 der Membran 110 und einem zweiten Unterbrechungsbereich 170 der Membran 110 angeordnet sein kann. Dabei kann eine Dicke der Dünnschicht-Membran 110 (sie besteht z. B. aus mehreren Grund-, Sensor- und Passivierung-Schichten) im Fall eines Drahtsensors (ein Beispiel für die Temperatursensor-Strukturen 130, 140; siehe Fig. 2 und Fig. 3) z. B. zwischen 1-10μm liegen. Das Heizelement 120 kann auch als Heizer bezeichnet werden. Gemäß Fig. 2a und Fig. 2b kann der komplette erste Unterbrechungsbereich 160 eine Unterbrechung 162 der Membran 110 aufweisen und der komplette zweite Unterbrechungsbereich 170 eine Unterbrechung 172 der Membran aufweisen. Somit kann das Heizelement 120 freitragend zwischen der ersten Unterbrechung 162 und der zweiten Unterbrechung 172 angeordnet sein. Die erste Unterbrechung 162 kann von dem Heizelement 120 und einer ersten Temperatursensor-Struktur 130, in Form einer freitragenden Brückenstruktur, begrenzt sein. Die zweite Unterbrechung 172 kann von dem Heizelement 120 und von einer zweiten Temperatursensor-Struktur 140, beispielsweise in Form einer freitragenden Brückenstruktur, begrenzt sein. Bei der ersten Temperatursensor-Struktur 130 und/oder bei der zweiten Temperatursensor-Struktur kann es sich um einen Drahtsensor, Thermopiles, temperaturveränderlich Widerstände oder Thermistoren handeln.

Optional kann der Gassensor 100 eine erste äußere Unterbrechung 192 und eine zweite äußere Unterbrechung 194 aufweisen. Somit kann beispielsweise die erste Thermoelement-Struktur 130 eine freitragende Brückenstruktur zwischen der ersten Unterbrechung 160 und der zweiten äußeren Unterbrechung 194 sein und die zweite Thermoelement-Struktur 140 kann eine freitragende Brückenstruktur zwischen der zweiten Unterbrechung 172 und der ersten äußeren Unterbrechung 192 sein. Die erste Thermoelement-Struktur 130 kann auch als erster Detektor oder erster Sensor bezeichnet werden und die zweite Thermoelement-Struktur 140 kann auch als zweiter Sensor bzw. zweiter Detektor bezeichnet werden.

Oberhalb Fig. 2a ist ein Querschnitt des Gassensors 100 zu sehen. Der Gassensor 100 umfasst beispielsweise einen Rahmen 150 aus Trägermaterial. Der Rahmen 150 aus Trägermaterial kann beispielsweise die Membran 110 aufspannen. Gemäß einem Ausführungsbeispiel kann die Membran 110 eine Dicke 111 (beispielsweise eine Ausdehnung senkrecht zu einer Oberfläche der Membran 110, auf der die erste Thermoelement-Struktur 130, die zweite Thermoelement-Struktur 140 und das Heizelement 120 angeordnet sind) in einem Bereich von 1 $\mu$m bis 50 $\mu$m, 2 $\mu$m bis 25 $\mu$m oder 3 $\mu$m bis 10 $\mu$m, wie z. B. 8 $\mu$m, aufweisen. Gemäß einem Ausführungsbeispiel kann die Membran 110 durch eine Ausnehmung 190 aus dem Rahmen 150 realisiert werden. So kann beispielsweise die Ausnehmung 190 so gewählt werden, dass eine Membran 110 mit der gewünschten Dicke 111 realisiert werden kann.

Gemäß dem Ausführungsbeispiel in Fig. 2a und 2b kann die Ausnehmung 190 beispielsweise so gestaltet sein, dass nur das Heizelement 120, die erste Thermoelement-Struktur 130 und die zweite Thermoelement-Struktur 140 zwischen dem Rahmen 150 aufgespannt bleiben. Gemäß einem Ausführungsbeispiel kann eine Oberfläche der Membran 110, auf der die erste Thermoelement-Struktur 130, die zweite Thermoelement-Struktur 140 und das Heizelement 120 angeordnet sind, eine Ausdehnung in einem Bereich von 200x200 $\mu$m$^2$ bis 5x5 mm$^2$, 500x500 $\mu$m$^2$ bis 2000x2000 $\mu$m$^2$ oder 800x800 $\mu$m$^2$ bis 1200x1200 $\mu$m$^2$ aufweisen, wobei es sich bei der Ausdehnung um eine quadratische oder rechteckige Ausdehnung handeln kann. Der Gassensor 100 kann eine Dicke 101 (beispielsweise parallel zu der Dicke 111 der Membran 110) in einem Bereich von 500 nm bis 5 mm, 1 $\mu$m bis 1 mm oder von 200 $\mu$m bis 600 $\mu$m, wie z. B. 400 $\mu$m, aufweisen. Eine Ausdehnung des Gassensors 100, parallel zu der Oberfläche der Membran 110 auf der das Heizelement 120 angeordnet ist, kann in einem Bereich von 1x1 mm$^2$ bis 1x1 cm$^2$, 1.5x1.5 mm$^2$ bis 9x9 mm$^2$ oder von 2x2 mm$^2$ bis 8x8 mm$^2$, wie z. B 6.5x2.5 mm$^2$, liegen.

Gemäß einem Ausführungsbeispiel kann die erste Thermoelement-Struktur 130, die zweite Thermoelement-Struktur 140 und/oder das Heizelement 120 Teil der Membran 110 sein.

Zur Messung eines von der Gasart bzw. vom Gasgemisch abhängigen Wärmetransports kann ein Mikrochip (Beispiels für den thermischen Gassensor 100) mit drei zwischen einem Rahmen freitragend gespannten filigranen Brückenstrukturen (z. B. dem Heizelement 120, der ersten Thermoelement-Struktur 130 und der zweiten Thermoelement-Struktur 140) verwendet werden, die als Mikrodrähte vom zu analysierenden Gas umgeben sein können. Das zu analysierende Gas kann beispielsweise in der ersten Unterbrechung 162, der zweiten Unterbrechung 172, der ersten äußeren Unterbrechung 192 und/oder in der zweiten äußeren Unterbrechung 194 angeordnet sein. Eine mittlere Brückenstruktur kann als Heizer 120 ausgelegt sein und zwei beidseitig in unterschiedlichen Abständen zum Heizer 120 liegende Detektor-Strukturen (z. B. die erste Thermoelement-Struktur 130 und die zweite Thermoelement-Struktur 140) können als Temperatursensoren zur Messung einer Übertragungsantwort aus dem Gasgemisch verwendet werden.

Der mittlere Draht (das Heizelement 120) wird mit einem beispielsweise periodischen Heizsignal beaufschlagt, wodurch beispielsweise Wärme von dem Heizelement ausgestrahlt wird. Ein Wärmeübertrag kann über unbekannte Wärmeübergänge vom Heizer 120 ins zu analysierende Gas und vom Gas in den Sensor-Draht (z. B. in die erste Thermoelement-Struktur 130 und/oder die zweite Thermoelement-Struktur 140) erfolgen. Der so von der ersten Thermoelement-Struktur 130 und/oder der zweiten Thermoelement-Struktur 140 detektierte Wärmeübertrag kann als Übertragungsantwort oder als Sensorsignal verstanden werden (beispielsweise ein erstes Sensorsignal detektiert von der ersten Thermoelement-Struktur 130 und ein zweites Sensorsignal detektiert von der zweiten Thermoelement-Struktur 140). Durch eine Messung einer Temperatur-Antwort (beispielsweise die Übertragungsantwort) mit beispielsweise zwei identischen Sensoren (z. B. die erste Thermoelement-Struktur 130 und/oder die zweite Thermoelement-Struktur 140) in unterschiedlichen Abständen zum Heizer 120 lassen sich die unbekannten Wärmeübergänge in der Messanordnung eliminieren. Phase und Amplitude der beiden Sensorsignale können im Wesentlichen von der Wärmeübertragung durch das Gas abhängig sein.

**1.1.1 Der Gassensor 100 beispielsweise als MEMS Draht-Sensor (Auswertung eines TKR (Temperaturkoeffizient eines Widerstandes) an Detektor-Widerständen (z. B. ein Widerstand der ersten Thermoelement-Struktur 130 und/oder der zweiten Thermoelement-Struktur 140)) in Kombination mit dem Auswertealgorithmus gemäß Abschnitt 1.3 (Alternatives Ausführungsbeispiel, optional verwendbar in Kombination mit der Signalgenerierung und Auswertung gemäß Abschnitt 1.2)**

Eine erste Variante des thermischen Gassensors 100 kann auf Basis eines Silicon-On-Insulator (SOI) Wafer-Substrates aufgebaut sein. Sie besteht beispielsweise aus einem Mikrochip mit freitragenden filigranen Brückenstrukturen aus Silizium-Mikrodrähten (z. B. die erste Temperatursensor-Struktur 130 und die zweite Temperatursensor-Struktur 140), die im zu analysierenden Gasraum aufgespannt sind. Ein mittlerer Draht kann als Heizer 120 ausgelegt sein und zwei Detektor-Drähte (z. B. die erste Temperatursensor-Struktur 130 und die zweite Temperatursensor-Struktur 140) können beidseits zum Heizer in unterschiedlichen Abständen davon als Temperatursensoren dienen (siehe Fig. 2a, Fig. 2b). Fig. 2a zeigt beispielsweise ein Foto des MEMS Draht-Sensor Chips (des Gassensors 100) an einem Lichtmikroskop (links) und Fig. 2b zeigt beispielsweise eine Nah-Aufnahme von Strukturen in einem Raster-Elektronen-Mikroskop (rechts).

Fig. 3 zeigt eine schematische Darstellung einer Silizium-Brücke 120/130/140, die beispielsweise für ein Heizelement, eine erste Thermoelement-Struktur und/oder eine zweite Thermoelement-Struktur eines Gassensors verwendet werden kann. In anderen Worten zeigt Fig. 3 ein Detail einer Mikro-Brücke (REM, Raster-Elektronen-Mikroskop) eines thermischen MEMS-Draht-Sensors (z. B. eines Gassensors). Die dargestellte Silizium-Brücke 120/130/140 kann beispielsweise durch SOI-Technologie hergestellt werden. So kann beispielsweise ein Substrat oder Trägermaterial eines Rahmens 150 Oxidmaterial 152, Siliziummaterial 154 und Aluminiummaterial 156 aufweisen. Um die Silizium-Brücke zu realisieren kann beispielsweise Siliziummaterial 154 zumindest teilweise entfernt werden, um Einschnitte 158 (beispielsweise Trenches) in dem Trägermaterial des Rahmens 150 zu realisieren und dadurch die Silizium-Brücke 120/130/140 zu realisieren. Die Silizium-Brücke 120/130/140 kann auf der Membran 110 (beispielsweise bestehend aus OxidMaterial 152) angeordnet sein.

Die Membran 110 kann beispielsweise einen ersten Unterbrechungsbereich 160/162 und einen zweiten Unterbrechungsbereich 170/172 aufweisen. Sowohl der erste Unterbrechungsbereich 160/162 als auch der zweite Unterbrechungsbereich 170/172 weisen beispielsweise eine Unterbrechung auf, die beispielsweise eine Kavität sein kann. Somit kann die Membran 110 eine erste Unterbrechung 162 und eine zweite Unterbrechung 172 aufweisen, in der das zu analysierende Gas angeordnet sein kann und beispielsweise von der Silizium-Brücke 120/130/140 Wärme übertragen bekommt, wenn die Silizium-Brücke ein Heizelement 120 darstellt bzw. an die Silizium-Brücke 120/130/140 übertragen kann, wenn die Silizium-Brücke 120/130/140 die erste Thermoelement-Struktur 130 und/oder die zweite Thermoelement-Struktur 140 darstellt. Die Silizium-Brücke 120/130/140 kann durch das Aluminiummaterial 156 kontaktiert werden, wodurch das Aluminiummaterial 156 beispielsweise als Bond-Pad dienen kann. Durch das Bond-Pad kann beispielsweise an das Heizelement 120 ein anregendes Heizer-Signal angelegt werden bzw. die erste Thermoelement-Struktur 130 und/oder die zweite Thermoelement-Struktur 140 ausgelesen werden (beispielsweise ein erstes oder zweites Sensorsignal).

Vorteile der SOI-Technologie:

- kristalline Widerstandbahnen, der Temperaturkoeffizient des Widerstandes (TKR) für die Detektoren (z. B. für die erste Thermoelement-Struktur 130 und die zweite Thermoelement-Struktur 140) kann allein von der Grunddotierung des Wafer-Materials (im Active Layer) abhängig sein
- ähnlich hoher TKR wie bei Platin bei hohem Grundwiderstand der Widerstände der Temperatur-Detektoren (z. B. die erste Thermoelement-Struktur 130 und die zweite Thermoelement-Struktur 140) ermöglicht miniaturisierte Sensor-Dimensionen (z. B. Dimensionen der ersten Thermoelement-Struktur 130 und der zweiten Thermoelement-Struktur 140) aufgrund kurzer (Beispielsweise von einer Rahmenseite des Rahmens 150 zu einer gegenüberliegenden Rahmenseite des Rahmens 150) Widerstandsbahnen der Brückenstrukturen 120, 130, 140 (kleiner als 1 mm) sowie für den Bereich der Resistance Temperatur Detektoren (RTD) (z. B. die erste Thermoelement-Struktur 130 und die zweite Thermoelement-Struktur 140) vergleichbar geringen Temperatur-Mess-Fehlern aufgrund von Eigenerwärmung, da z. B. mit Grundwiderstandswerten größer 8 kOhm gearbeitet werden kann, die im Messbetrieb weniger als 360 $\mu$W Leistungsaufnahme benötigen können.
- Heizer-Widerstand (z. B. des Heizers 120) durch Implantation auf niedrige Betriebsspannung anpassbar (vorzugsweise 3,3V)
- sehr homogene Verteilung der ohmschen Sensor-Widerstände, z. B. der Widerstand der ersten Thermoelement-Struktur 130 und/oder der zweiten Thermoelement-Struktur 140, über dem Wafer (z. B. dem Rahmen 150) in einem engen Prozessfeld, insbesondere die Toleranzen der Detektorwiderstände (z. B. Sensor-Widerstände) sind z. B. durch Toleranzen des SOI-Materials in einer aktiven Schicht (Active Layer, Grunddotierung und Materialdicke) sowie durch die laterale Struktur-Genauigkeit der Trockenätzung (Deep-RIE) bestimmt.

Nachteile der SOI-Technologie:

$\mu$ vergleichbar teures SOI-Substrat Material beim Wafer Einkauf

μ Verfügbarkeit meist nicht in den Wunsch-Spezifikationen vorrätig (Wafer Durchmesser, Materialdicken von Handle und Active Layer, Dotierung des Active Layer)

μ derzeit keine Passivierung der Strukturen, eine Passivierung führt u. U. zu Bimetall-Effekten aufgrund der unterschiedlichen Materialdehnung der Schichten bei Wärmeeintrag, Veränderung der Kennlinie des TKR

**1.1.2 Der Gassensor 100 beispielsweise als MEMS Thermopile-Sensor auf Dünnschicht-Membran in Kombination mit dem Auswertealgorithmus gemäß Abschnitt 1.3 (optional verwendbar in Kombination mit der Signalgenerierung und Auswertung gemäß Abschnitt 1.2)**

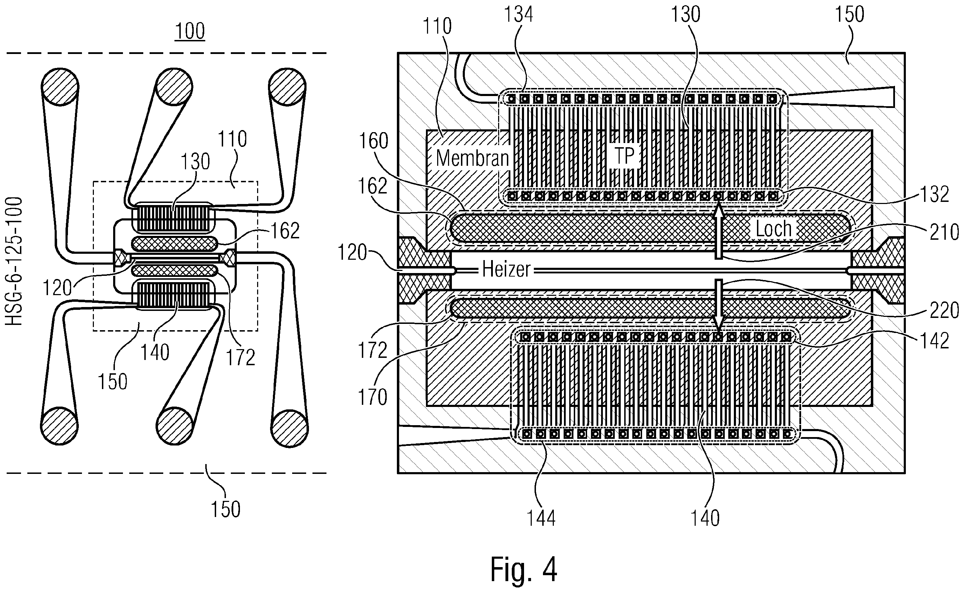

Fig. 4 zeigt eine schematische Darstellung eines Gassensors 100 auf der linken Seite und eine Detailansicht des Gassensors 100 auf der rechten Seite.

Gemäß einem Ausführungsbeispiel kann der Gassensor 100 eine Membran 110 und ein Heizelement 120 aufweisen, das auf der Membran 110 zwischen einem ersten Unterbrechungsbereich 160 der Membran 110 und einem zweiten Unterbrechungsbereich 170 der Membran 110 angeordnet sein kann. Der erste Unterbrechungsbereich 160 kann eine Unterbrechung 162 aufweisen und der zweite Unterbrechungsbereich 170 kann eine Unterbrechung 172 aufweisen.

Die erste Unterbrechung 162 und/oder die zweite Unterbrechung 172 können eine Längsausdehnung parallel zu einer Richtung maximaler Ausdehnung des Heizelements 120 (das beispielsweise als Heizer bezeichnet werden kann) aufweisen und eine Querausdehnung, beispielsweise in einer Richtung senkrecht zu einer Richtung maximaler Ausdehnung des Heizelements 120, aufweisen. Gemäß Fig. 4 kann somit die erste Unterbrechung 162 eine größere Querausdehnung aufweisen als die zweite Unterbrechung 172. Des Weiteren können die erste Unterbrechung 162 und die zweite Unterbrechung 172 gemäß Fig. 4 die gleiche Längsausdehnung aufweisen. Die erste Unterbrechung 162 und die zweite Unterbrechung 172 weisen beispielsweise die Längsausdehnung auf, die so groß ist, dass die erste Unterbrechung 162 als auch die zweite Unterbrechung 172 den Bereich zwischen der ersten Thermoelement-Struktur 130 bzw. der zweiten Thermoelement-Struktur 140 und dem Heizelement 120 vollständig abdecken. So ist beispielsweise die Längsausdehnung sowohl der ersten Unterbrechung 162 als auch der zweiten Unterbrechung 172 entlang der kompletten Länge des Heizelements 120. Dadurch kann vermieden werden, dass ein Großteil der Wärme abgestrahlt von dem Heizelement 120 über die Membran 110 transportiert wird. Somit kann erreicht werden, dass ein Großteil der Wärme über das Gas, angeordnet in der ersten Unterbrechung 162 und in der zweiten Unterbrechung 172, zu der jeweiligen Thermoelement-Struktur 130, 140 übertragen wird.

Die erste Thermoelement-Struktur 130 kann beispielsweise einen anderen Abstand zu dem Heizelement 120 aufweisen, als die zweite Thermoelement-Struktur 140. So weist gemäß Fig. 4 beispielsweise die erste Thermoelement-Struktur 130 einen größeren Abstand zu dem Heizelement 120 auf, als die zweite Thermoelement-Struktur 140. Die erste Thermoelement-Struktur 130 kann beispielsweise einen ersten Wärmeübertrag 210, von dem Heizelement 120 zu dem Gas in der ersten Unterbrechung 162 und von dem Gas zu der ersten Thermoelement-Struktur 130, detektieren und als erstes Sensorsignal erfassen. Die zweite Thermoelement-Struktur 140 kann beispielsweise einen zweiten Wärmeübertrag 220, von dem Heizelement 120 zu dem Gas in der zweiten Unterbrechung 172 und von dem Gas zu der zweiten Thermoelement-Struktur 140, detektieren und als zweites Sensorsignal bereitstellen. Durch den unterschiedlichen Abstand der ersten Thermoelement-Struktur 130 und der zweiten Thermoelement-Struktur 140 von dem Heizelement 120 kann ein Differenzsignal aus dem ersten Sensorsignal und dem zweiten Sensorsignal gebildet werden, wodurch unbekannte Übergänge (wie z. B ein Übergang von dem Heizelement zu dem Gas bzw. von dem Gas zu der jeweiligen Thermoelement-Struktur) herausgerechnet werden können und somit z. B. hauptsächlich bzw. nur der Wärmeübertrag über das Gas in der ersten Unterbrechung 162 oder der zweiten Unterbrechung 172 durch den Gassensor 100 betrachtet wird.

Gemäß einem Ausführungsbeispiel kann der Gassensor 100 außerdem einen Rahmen 150 aufweisen, der die Membran 110 aufspannen kann. Die erste Thermoelement-Struktur 130 und die zweite Thermoelement-Struktur 140 können zumindest teilweise auf der Membran 110 und zumindest teilweise auf dem Rahmen 150 angeordnet sein. Dabei können die erste Thermoelement-Struktur 130 und die zweite Thermoelement-Struktur 140 heiße Enden 132, 142 aufweisen, die dem Heizelement 120 zugewandt angeordnet sind. Zudem können die erste Thermoelement-Struktur 130 und die zweite Thermoelement-Struktur 140 kalte Enden 134, 144 aufweisen, die auf einer Seite der ersten Thermoelement-Struktur 130 bzw. der zweiten Thermoelement-Struktur 140 gegenüber der Seite mit den heißen Enden 132, 142 angeordnet sein können und somit dem Heizelement 120 abgewandt angeordnet sind. So können beispielsweise die heißen Enden 132, 142 auf der Membran 110 angeordnet sein und die kalten Enden 134, 144 auf dem Rahmen 150 angeordnet sein. Dabei kann beispielsweise der Rahmen 150 ein anderes Material aufweisen als die Membran 110. Dadurch können beispielsweise die kalten Enden 134, 144 mit einer Referenztemperatur, durch das Rahmenmaterial des Rahmens 150, beaufschlagt werden, in Bezug zu einer Temperatur gemessen mittels der heißen Enden 130, 142, übertragen von dem Heizelement 120.

In anderen Worten kann die linke Darstellung des Gassensors 100 ein Layout und die rechte Seite der Fig. 4 ein Foto des Gassensors 100 (z. B. ein MEMS-Membran-Sensor) zur Messung beispielsweise eines gasartabhängigen Wärmetransports (Ausführungsbeispiel gemäß Aspekt 1) darstellen. In Fig. 4 ist beispielsweise eine Variante des Gassensors 100 mit konstanter Unterbrechung (z. B. einer ersten Unterbrechung 162 und einer zweiten Unterbrechung 172) einer Membran 110 zu sehen. Die konstante Unterbrechung 162, 172 bewirkt z. B., dass ein Hauptanteil eines Wärmetransports zwischen einem Heizer 120 und den Detektoren (z. B. der ersten Thermoelement-Struktur 130 und der zweiten Thermoelement-Struktur 140) z. B. zwingend über das zwischen beiden Elementen eingeschlossene Messgasvolumen erfolgt, beispielsweise über das Messgas angeordnet in der ersten Unterbrechung 162 und in der zweiten Unterbrechung 172.

Zur Reduzierung des Prozessaufwandes bei der technologischen Herstellung z. B. des Gassensors 100 und zur Erhöhung der Sensitivität bei der Messung des gasartabhängigen Wärmetransports 210, 220 kann ein Mikrochip auf Basis einer Dünnschicht-Membran 110 mit Heizer- 120 und Thermopile-Strukturen 130, 140 (Detektoren) realisiert werden, bei dem die Dünnschicht-Membran 110 in einem lateralen Gebiet zwischen Heizer 120 und Detektoren 130, 140 herausgeätzt werden können.

In einem Vergleich zu einem Drahtsensor (wie z. B. in Abschnitt 1.1.1 beschrieben) benötigt der Membran-Sensor (z. B. der Gassensor 100) nur 1/3 der Heizenergie bei identischer Sensitivität zur Gaskonzentration eines binären Gemisches. Wie bei dem Draht-Sensor liegt die Heizerstruktur (z. B. das Heizelement 120) beispielsweise mittig als freitragende filigrane Brückenstruktur aufgespannt in einem Messraum des zu detektierenden Gases. Die zwei zum Heizer 120 beidseitig (beispielsweise) in unterschiedlichen Abständen angeordneten Detektor-Drähte können durch "Thermopile" (Thermokette) Strukturen (z. B. der ersten Thermoelement-Struktur 130 und/oder der zweiten Thermoelement-Struktur 140) ersetzt werden, die auf den seitlich aufgespannten Membranflächen (der Membran 110) liegen können und die beispielsweise bis an den Grubenrand (z. B. ein Rand der ersten Unterbrechung 162 bzw. der zweiten Unterbrechung 172) reichen können.

Beispielsweise sollten die kalten Enden 134, 144 der Thermoketten (Thermopiles) 130, 140 das Trägermaterial (z. B. des Rahmens 150), das eine hohe Wärmeleitfähigkeit besitzen kann (beispielsweise Silizium ca. 150 W/(m*K)) und als Wärmesenke dienen kann (Kühlkörper nahe Raumtemperatur), unmittelbar kontaktieren. Zwischen den kalten Enden 134, 144 der Thermopiles und dem Silizium liegt z. B. das Grund-Membran-Material (Material der Membran 110), das die Kontakte vom Silizium elektrisch isoliert. Da diese Schicht aber sehr dünn ist, kann die Wärme aus den Thermopiles gut ins Silizium über-tragen werden. So kann die Übertemperatur (z. B. gemessen mittels der heißen Enden 132, 142) als direkte Differenz zur Raumtemperatur (z. B. gemessen mittels der kalten Enden 134, 144) gemessen werden. Eine Mess-Stelle zur Temperatur-Kompensation ist beispielsweise direkt auf oder mit dem Siliziumchip (z. B. der Rahmen 150) mechanisch verbunden.

Um einen parasitären Effekt des Wärmetransports 210, 220 zwischen Heizer 120 und Detektor-Strukturen 130, 140 aufgrund einer Wärmeleitung in dem Membranmaterial der Membran 110 zu reduzieren, kann die Membran 110 konsequent unterbrochen werden, so dass der Wärmetransport 210, 220 von dem Heizer 120 zu den Detektoren 130, 140 hauptsächlich über eine kürzeste laterale Distanz erfolgen kann und somit z. B. eine Strecke über ein dazwischen liegendes Volumen des Messgases (z. B. angeordnet in der ersten Unterbrechung 162 und der zweiten Unterbrechung 172) durchläuft. Dadurch kann die gasartabhängige Übertragungsantwort (z. B. das erste Sensorsignal und das zweite Sensorsignal) des Sensors 130, 140 auf periodische Wärmepulse des Heizers 120 entscheidend erhöht werden.

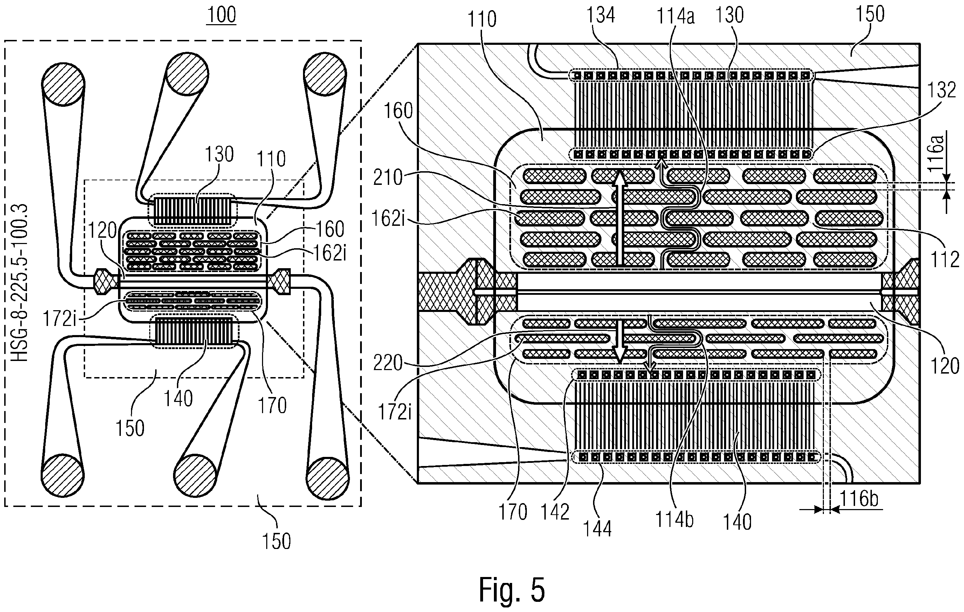

Gemäß einem Ausführungsbeispiel zeigt Fig. 5 auf der linken Seite eine schematische Darstellung des Gassensors 100 und auf der rechten Seite eine vergrößerte Detailansicht des Gassensors 100. Der Gassensor 100 aus Fig. 5 kann dieselben Merkmale und Funktionalitäten wie der Gassensor 100 aus Fig. 4 aufweisen, wobei sich der Gassensor 100 aus Fig. 5 von dem Gassensor 100 auf Fig. 4 in einer Ausgestaltung des ersten Unterbrechungsbereichs 160 bzw. des zweiten Unterbrechungsbereichs 170 unterscheiden kann. So kann beispielsweise der erste Unterbrechungsbereich 160 des Gassensors 100 aus Fig. 5 eine Vielzahl an Unterbrechungen $162_i$ aufweisen und der zweite Unterbrechungsbereich 170 kann ebenfalls eine Vielzahl an Unterbrechungen $172_i$ aufweisen. So kann beispielsweise der Index i der Unterbrechungen $162_i$ des ersten Unterbrechungsbereichs 160 des Gassensors 100 von 1 bis 23 gehen, da der erste Unterbrechungsbereich 160 gemäß dem Ausführungsbeispiel in Fig. 5 23 Unterbrechungen aufweisen kann. Der Index i der Unterbrechungen $172_i$ des zweiten Unterbrechungsbereichs 170 des Gassensors 100 kann beispielsweise von 1 bis 14 gehen, da der zweite Unterbrechungsbereich 170 gemäß einem Ausführungsbeispiel der Fig. 5 14 Unterbrechungen aufweisen kann. Optional kann der Index i sowohl der Unterbrechungen $162_i$ als auch der Unterbrechungen $172_i$ eine natürliche Zahl definieren, wobei der Index i z. B. angibt, wie viele Unterbrechungen $162_i$, $172_i$ in einen Unterbrechungsbereich 160, 170 vorliegen.

Die Unterbrechungen $162_i$, $172_i$ können in dem ersten Unterbrechungsbereich 160 bzw. in dem zweiten Unterbrechungsbereich 170 in Reihen parallel zu einer Richtung maximaler Ausdehnung des Heizelements 120 angeordnet sein und zusätzlich können die Reihen gegeneinander versetzt angeordnet sein. Das bedeutet beispielsweise, dass durch Membranmaterial gebildete Querstege 112 (verlaufen beispielsweise in einer Richtung senkrecht zu einer Richtung maximaler Ausdehnung des Heizelements 120, von dem Heizelement 120 zu der jeweiligen Thermoelement-Struktur 130, 140) aufeinanderfolgender Reihen versetzt zueinander angeordnet sind. Dies bewirkt beispielsweise, dass eine parasitäre Wärmeleitung 114a, 114b in der Membran 110 einen möglichst langen Weg durchläuft.

Die Unterbrechungen $162_i$, $172_i$ sind beispielsweise so angeordnet, dass eine Gitterstruktur in der Membran 110 entsteht, bei der ein Weg einer parasitären Wärmeleitung 114a, 114b durch die Membran 110 länger ist als ein direkter Weg 210, 220. Ein direkter Weg 210, 220 kann beispielsweise ein gerader Weg senkrecht zu dem Heizelement 120, von dem Heizelement 120 zu der jeweiligen Thermoelement-Struktur 130, 140 sein, wobei der direkte Weg 210, 220 ein zu analysierendes Gas, angeordnet in den Unterbrechungen $162_i$, $172_i$, durchlaufen kann. Der Weg der parasitären Wärmeleitung 114a, 114b durch die Membran 110 sollte beispielsweise nicht geradlinig verlaufen, sondern einen gewundenen Weg, wie dargestellt in Fig. 5, bilden. Es sollte beispielsweise keinen direkten Wärmepfad rein über die Membran 110 geben. Dadurch kann ermöglicht werden, dass die erste Thermoelement-Struktur 130 und die zweite Thermoelement-Struktur 140 eine Wärmeübertragung von dem Heizelement 120 über den direkten Weg 210 bzw. 220 detektieren können und Einflüsse von parasitärer Wärmeleitung 114a, 114b bei der Detektion minimiert werden können, wodurch das Gas sehr genau analysiert werden kann.

Bei den Unterbrechungen $162_i$, $172_i$ kann es sich beispielsweise um längliche Unterbrechungen handeln, die beispielsweise mit einer Toleranz von +/- 20° senkrecht zu einer Hauptrichtung der Wärmeleitung (beispielsweise der direkter Weg 210, 220 von dem Heizelement 120 zu den Thermoelement-Strukturen 130, 140) liegen können.

Gemäß einem Ausführungsbeispiel kann es sich bei den Unterbrechungen $162_i$, $172_i$ um rechteckige Ausschnitte mit abgerundeten Ecken handeln. Diese können beispielsweise auch als Langloch bezeichnet werden und es kann sich hierbei beispielsweise auch um ovale Löcher handeln. Dabei können die Unterbrechungen $162_i$, $172_i$ mindestens dreimal länger als breit sein. Die Länge kann beispielsweise definiert sein, als eine Richtung parallel zu einer maximalen Ausdehnung des Heizelements 120 und die Breite, als eine Richtung senkrecht zu der maximalen Ausdehnung des Heizelements 120. Durch dieses Merkmal kann der Weg der parasitären Wärmeleitung 114a, 114b sehr lang realisiert werden, wodurch eine Qualität der Gasanalyse durch den Gassensor 100 gesteigert werden kann.

Gemäß einem Ausführungsbeispiel können die Unterbrechungen $162_i$, $172_i$ in dem ersten Unterbrechungsbereich 160 bzw. dem zweiten Unterbrechungsbereich 170 so angeordnet sein, dass ein Abstand 116a, 116b zwischen den Unterbrechungen $162_i$, $172_i$ einer kleinsten zu realisierenden Strukturbreite, die eine mechanisch haltbare Gitterstruktur ergibt, entspricht. Der Abstand 116a, 116b ist beispielsweise eine Breite von Stegen aus Membranmaterial der Membran 110. Je kleiner der Abstand 116a, 116b realisiert wird, desto geringer kann eine parasitäre Wärmeleitung 114a, 114b sein, wodurch eine Qualität einer Analyse eines Gases durch den Gassensor 100 gesteigert werden kann. Dabei sollte der Abstand 116a, 116b so gewählt sein, dass die durch die Unterbrechungen $162_i$, $172_i$ entstehende Gitterstruktur in der Membran 110 mechanisch haltbar ist, um eine hohe Qualität der Gasanalyse durch den Gassensor 100 zu gewährleisten.

In anderen Worten kann Fig. 5 ein Layout eines MEMS-Membran-Sensors (z. B. dem Gassensor 100) beispielsweise zur Messung des gasartabhängigen Wärmetransports (beispielsweise über den direkten Weg 210, 220) (Ausführungsbeispiel gemäß Aspekt 1) darstellen. Somit kann der Gassensor 100 aus Fig. 5 eine Variante mit einer Gitterstruktur aus dem Membranmaterial der Membran 110 zur Erhöhung der mechanischen Stabilität des Gassensors 100 darstellen. Die geometrische Form des Gitters kann so gewählt sein, dass die parasitäre Wärmeleitung 114a, 114b im Membranmaterial einen möglichst langen Weg durchlaufen muss.

Fig. 5 zeigt eine weitere Ausführungsform des Gassensors 100, die eine Gitterstruktur zwischen Heizer- 120 und Detektor-Elementen (z. B. der ersten Thermoelement-Struktur 130 bzw. der zweiten Thermoelement-Struktur 140) zeigt, die die mechanische Stabilität des Gassensors 100 im Langzeitbetrieb verbessern soll. Eine solche Anordnung kann die gasartabhängige Sensitivität des thermischen Gassensors 100 verringern, da die Wärmeleitung nun auch parasitär 114a, 114b über die Gitterstege des Membranmaterials erfolgen kann. Somit kann ein Teil der periodisch in den Heizer 120 eingetragenen Wärmeenergie zeitlich früher zu den Detektorstrukturen (z. B. der ersten Thermoelement-Struktur 130 bzw. der zweiten Thermoelement-Struktur 140), als der Teil der Wärmeenergie, der über die lateral kürzeste Distanz 210, 220 durch das Messgas hindurch transportiert wird. Aufgrund der thermischen Masse der Detektoren (z. B. der ersten Thermoelement-Struktur 130 bzw. der zweiten Thermoelement-Struktur 140), die auf eine periodische Anregung als Tiefpass antworten können, verschleifen sich z. B. beide Wärmewellen-Laufzeiten (z. B. die parasitäre Wärmeleitung 114a mit dem Wärmeübertrag über den direkten Weg 210 bzw. die parasitäre Wärmeleitung 114b mit dem Wärmeübertrag über den direkten Weg 220) zu einem einzigen sinusförmigen Detektor-Signal (z. B. zu einem ersten Sensorsignal oder zu einem zweiten Sensorsignal).

Die geometrische Form des Gitters ist beispielsweise so gewählt, dass die parasitäre Wärmeleitung 114a, 114b im Membranmaterial einen möglichst langen Weg durchlaufen muss. Die beispielsweise ovalen Löcher (z. B. die Unterbrechungen $162_i$, $172_i$) liegen beispielsweise quer zur Hauptrichtung der Wärmeleitung. Das Aspektverhältnis der ovalen Löcher ist beispielsweise wenigstens dreimal länger als breit, die Stegbreite (z. B. der Abstand 116a, 116b) entspricht beispielsweise der kleinsten zu realisierenden Strukturbreite, die mit der vorhandenen Schichttechnologie eine mechanisch haltbare Gitterstruktur ergibt.

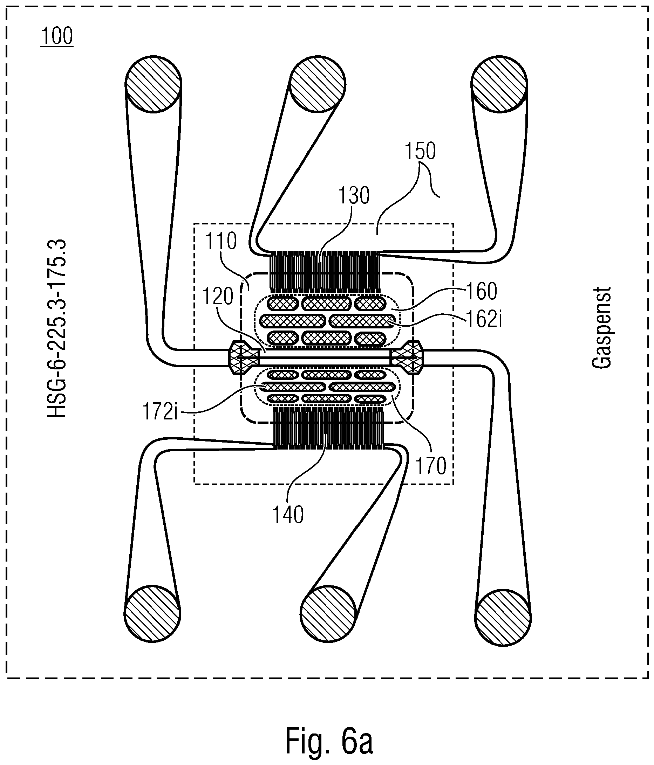

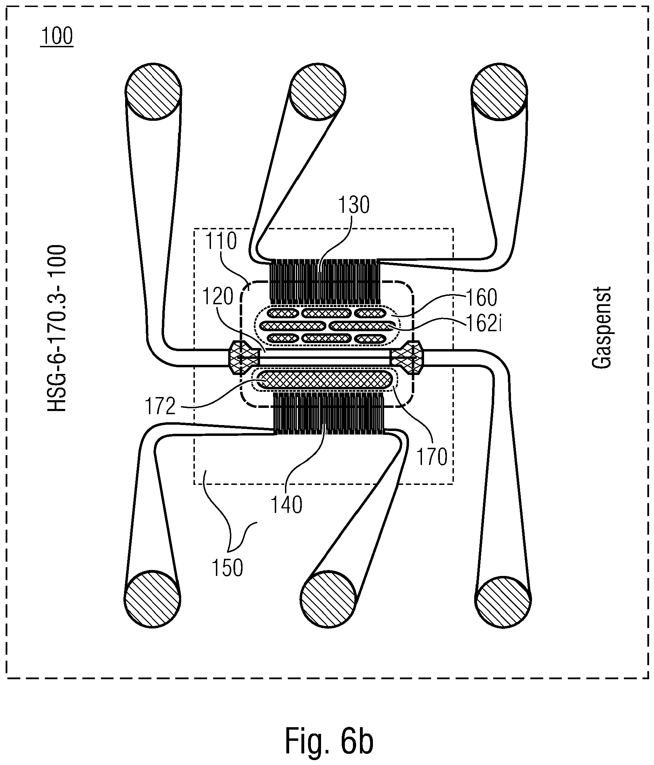

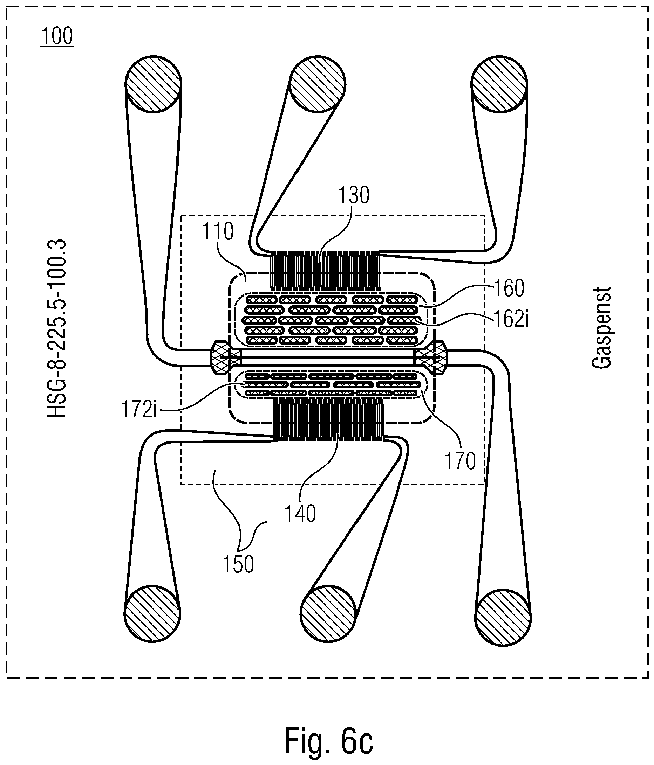

Fig. 6a, Fig. 6b und Fig. 6c zeigen schematische Darstellungen weiterer Ausführungsbeispiele eines Gassensors 100. Dabei kann der Gassensor 100 aus Fig. 6a, Fig. 6b und Fig. 6c die gleichen Merkmale und Funktionalitäten wie der Gassensor 100 aus Fig. 4 und/oder Fig. 5 aufweisen. Unterschiede zwischen den Gassensoren 100 können in dem ersten Unterbrechungsbereich 160 und dem zweiten Unterbrechungsbereich 170 des Gassensors 100 auftreten.

So kann der Gassensor 100 aus Fig. 6a beispielsweise acht Unterbrechungen $162_i$ in dem ersten Unterbrechungsbereich 160 sowie acht Unterbrechungen $172_i$ in dem zweiten Unterbrechungsbereich 170 aufweisen. Dabei können beispielsweise die Unterbrechungen $162_i$ eine größere Querausdehnung als die Unterbrechungen $172_i$ aufweisen. Zusätzlich können die Unterbrechungen $162_i$, $172_i$ innerhalb ihres jeweiligen Unterbrechungsbereichs 160 bzw. 170 unterschiedliche Längsausdehnungen aufweisen.

Der Gassensor 100 aus Fig. 6b weist beispielsweise einen ersten Unterbrechungsbereich 160 mit acht Unterbrechungen $162_i$ auf und einen zweiten Unterbrechungsbereich 170 mit einer durchgehenden Unterbrechung 172. Somit sind in der Variante von Fig. 6b beispielsweise in den Unterbrechungsbereichen 160, 170 die Varianten aus Fig. 6a bzw. Fig. 5 und aus Fig. 4 miteinander kombiniert.

Der Gassensor 100 aus Fig. 6c weist beispielsweise einen ersten Unterbrechungsbereich 160 sowie einen zweiten Unterbrechungsbereich 170 mit mehreren Unterbrechungen $162_i$, $172_i$ auf, wobei der erste Unterbrechungsbereich 160 beispielsweise 23 Unterbrechungen $162_i$ aufweisen kann und der zweite Unterbrechungsbereich 170 beispielsweise 14 Unterbrechungen $172_i$ aufweisen kann. Dabei können beispielsweise die Unterbrechungen $162_i$, $172_i$ eines Unterbrechungsbereichs 160 bzw. 170 dieselbe Querausdehnung und/oder dieselbe Längsausdehnung aufweisen. Optional ist es auch möglich, dass nur reihenweise die Unterbrechungen $162_i$, $172_i$ die gleiche Längsausdehnung und/oder Querausdehnung aufweisen.

In anderen Worten kann somit Fig. 6a, Fig. 6b und 6c weitere Layout-Varianten des MEMS-Membran-Sensors (beispielsweise des Gassensors 100) darstellen, die sich in Anzahl und Größe der Perforationen der Membran (z. B. der Unterbrechungen $162_i$, $172_i$ unterscheiden) (Ausführungsbeispiele gemäß Aspekt 1).

Vorteile der Thermopile-Strukturen (z. B. der ersten Thermoelement-Struktur 130 bzw. der zweiten Thermoelement-Struktur 140) auf Membran-Technologie (Beispiele):

- einfacher 5 Masken MEMS Prozess auf kostengünstigen Substraten möglich, da die Eigenschaften des Wafermaterials z. B. nur in Dicke, Oberflächengüte und für die Strukturierung der Grube in angepasster Grunddotierung spezifiziert sein sollten.
- gegenüber dem Gassensor auf SOI Strukturierung einer Grube (z. B. für die Membran 110) in angepasster Grunddotierung spezifiziert sein müssen.
- Die Strukturen (z. B. das Heizelement 120, die Membran 110, die erste Thermoelement-Struktur 130, die zweite Thermoelement-Struktur 140) sind beispielsweise mit Schutzschichten passiviert und bieten eine bessere Resistenz gegenüber freien Radikalen, die sich im Messgas befinden können und die aktiven Sensor-Strukturen (z. B. die erste Thermoelement-Struktur 130 und/oder die zweite Thermoelement-Struktur 140) anätzen und damit mechanisch schwächen oder thermisch verändern können.
- Substrat benötigt der Gassensor 100 auf Dünnschicht-Membran 110 beispielsweise nur ein Drittel der Heizleistung, um dieselbe Gas-Sensitivität zu erreichen, die Leistungsaufnahme beträgt ca. 12 mW gegenüber 36 mW bei der SOI Technologie.
- Thermopiles (Thermoketten) 130, 140 können statt temperaturveränderliche Widerstandsstrukturen (RTD) als Detektoren eines Wärmeverteilungsfeldes im Messraum realisiert werden: Die elektronische Signalauswertung der Thermopiles 130, 140 ist mit z. B. 0,6 μW nahezu leistungslos, während die Detektoren (z. B. die erste Thermoelement-Struktur 130, die zweite Thermoelement-Struktur 140 aus Fig. 2a, Fig. 2b oder Fig. 3) auf Basis von Widerstandsstrukturen der SOI-Technologie für eine stabile Signalgenerierung einen Stromfluss benötigen aufgrund dessen eine Heizleistung in den Detektor eingetragen wird, die mit ca. 140 μW zwar gering ist, jedoch gegenüber der Thermopile-Technologie 200-fach größer ist und zur Eigenerwärmung der RTD-Detektoren beiträgt und damit parasitär die Gasselektivität verringern kann.

Nachteile der Membran-Technologie:

- filigrane perforierte Membranen 110 können im Produktionsprozess sowie im Dauerbetrieb brechen, ein optimiertes Design (siehe z. B. Fig. 4, Fig. 5, Fig. 6a, Fig. 6b oder Fig. 6c) ist empfehlenswert.

**1.1.3 Sensor Prinzip (Details optional)**

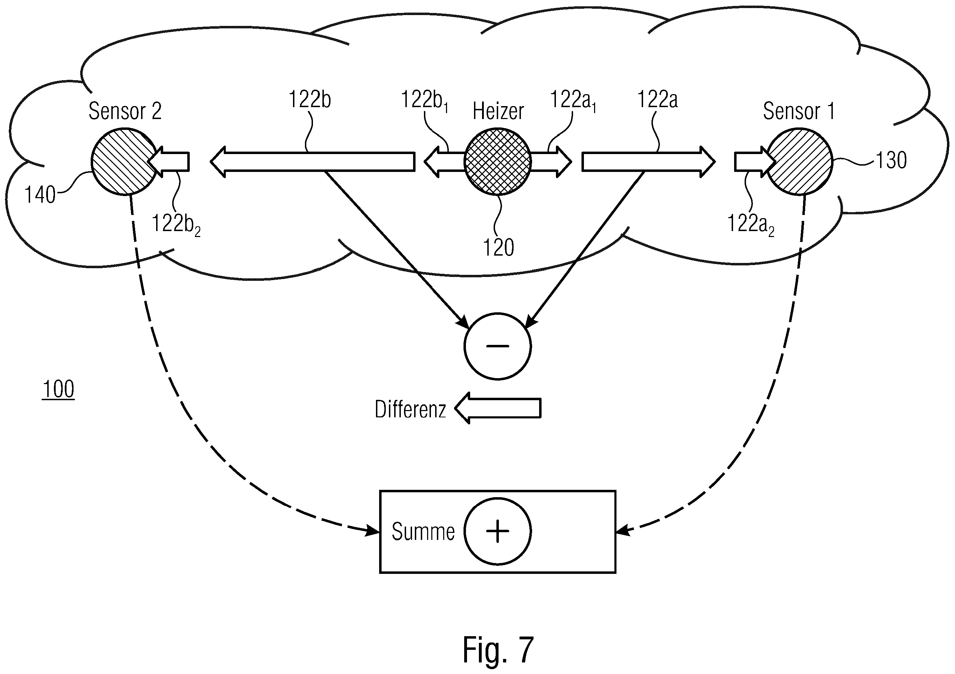

Fig. 7 stellt schematisch ein Grundprinzip des thermischen Sensors 100 (der Gassensor kann hierin auch als thermischer Sensor bezeichnet werden) dar: Deutlich zu sehen ist die räumliche Trennung zwischen Heizer- 120 und Sensor-Strukturen 130, 140 (die erste Temperatursensor-Struktur und die zweite Temperatursensor-Struktur können hierin auch als Sensor-Strukturen, Detektor-Strukturen, Sensoren, Temperatursensoren oder Detektoren bezeichnet werden) mit thermischer Kopplung durch das zu analysierende Gasgemisch; sowie die Messung mit Sensor-Strukturen 130, 140. Dabei können die Sensor-Strukturen 130, 140 in unterschiedlichen Abständen zum Heizer 120 angeordnet sein oder mit gleichem Abstand.

In anderen Worten zeigt Fig. 1 eine schematische Darstellung eines Sensor-Grundprinzips für einen Weg 122a, 122b des Wärmetransports durch das zu messende Gas zwischen Heizer 120 und Detektoren 130, 140.

- Heizer 120 und Sensoren 130, 140 durch Medium getrennt
  Heizer 120 und Sensor(en) 130, 140 sind im Medium getrennt angeordnet und vom zu analysierenden Gas umgeben. Der Wärmestrom 122a, 122b vom Heizer 120 zu den Temperatursensoren 130, 140 findet z. B. nur über das Gas selbst statt.

- Messung in mehreren Abständen
  Der Wärmetransport 122a, 122b findet auch z. B. über unbekannte Wärmeübergänge $122a_1$, $122b_1$ vom Heizer 120 ins zu analysierende Gas und über unbekannte Wärmeübergänge $122a_2$, $122b_2$ vom Gas in die Sensor-Struktur 130, 140 statt. Bei Messung in zwei Abständen $180_1$, $180_2$ sind die Wärmeübergänge $122a_1$, $122b_1$, $122a_2$, $122b_2$ nahezu dieselben. Die Differenz beider Sensorsignale hängt im Wesentlichen von der Wärmeübertragung durch das Medium selbst ab.

- Messung in gleichen Abständen

  Analog zu der Messung mit mehreren Abständen treten hier ebenfalls unbekannte Wärmeübergänge $122a_1$, $122b_1$, $122a_2$, $122b_2$ auf. Mittels einer Auswertung einer Summe der beiden Sensorsignale kann ebenfalls eine sehr genaue Gasanalyse durchgeführt werden und unter Umständen können die unbekannten Wärmeübergänge $122a_1$, $122b_1$, $122a_2$, $122b_2$ ebenfalls in der Analyse beachtet werden.

  Hierbei ist anzumerken, dass bei der Messung mit mehrere Abständen alternativ ebenso eine Auswertung eines Summensignals erfolgen kann.

  Ferner ist anzumerken, dass eine Auswertung eines Summensignal gegenüber einer Auswertung eines Differenzsignals bevorzugt wird, da ein Signal-Rausch-Abstand des Differenzsignals kleiner ist, als bei dem Summensignal.

  Optional kann ein Quotient aus Differenzsignal und Summensignal (das ist eine übliche Normierung) zur Auswertung verwendet werden. Der Messeffekt wird damit z. B. stärker hervorgehoben als wenn nur das Summensignal oder nur das Differenzsignal ausgewertet wird.

- Elektrische Analogie

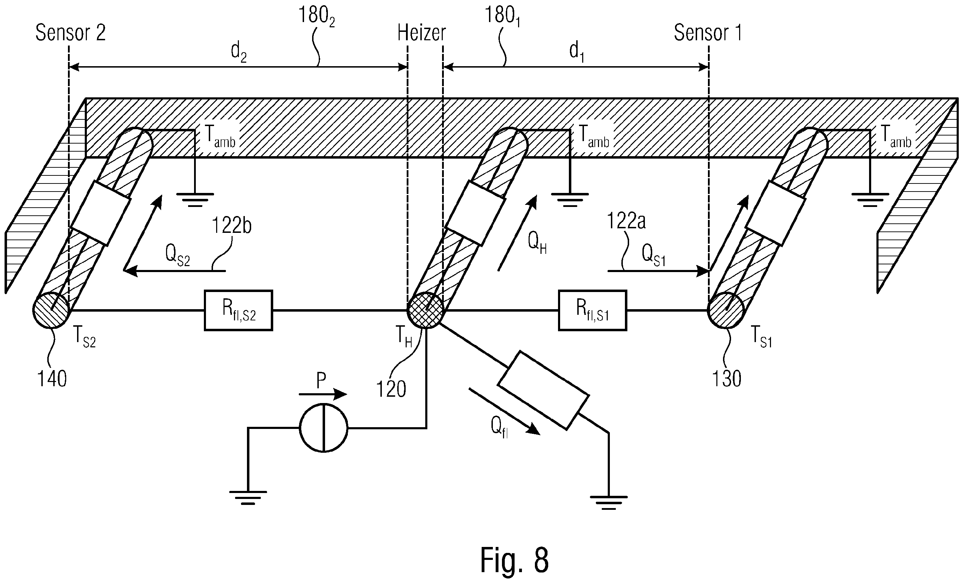

  Um die Wärmeströme zu identifizieren und abzuschätzen, wurde eine elektrische Analogie erstellt (siehe z. B. Fig. 8). Die Optimierung des Wärmeverlusts ist ein wesentlicher Faktor, um die Empfindlichkeit des Sensors 130, 140 zu steigern, ohne eine zu hohe Heizleistung, z. B. über das Heizelement 120, einspeisen zu müssen.

  Gemäß einem Ausführungsbeispiel weist Fig. 8 Merkmale und Funktionalitäten des Gassensors 100 aus Fig. 7 auf. In anderen Worten zeigt Fig. 8 eine schematische Darstellung des Wärmetransports an dem Gassensor 100. Der Wärmetransport vom Heizer 120 (Temperatur $T_H$) zum Sensor 130, 140 (Temperatur $T_S$) findet im Wesentlichen durch das zu messenden Gas statt.

**1.2 Beispiel des Gassensors in Betrieb: Signal-Generierung und -Auswertung auf einem embedded System (eingebettetes System)**

**1.2.1 Funktionsprinzip (Details optional)**

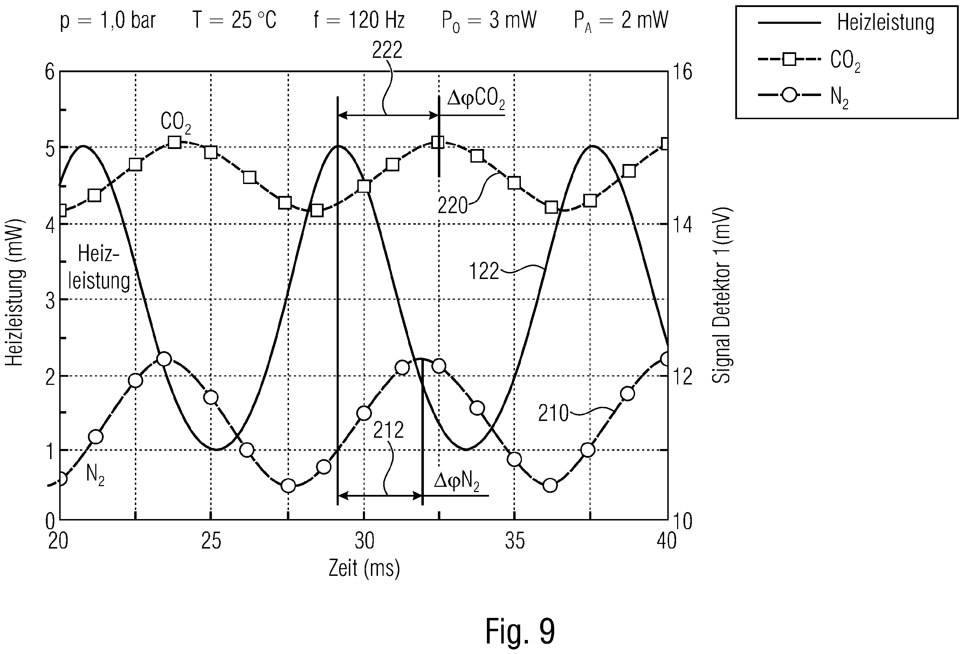

Bei sinusförmiger Heizleistung 122 ergibt sich ein sinusförmiger Verlauf der Sensorsignale 210, 220 (siehe z. B. Fig. 9), der stark von den thermischen Eigenschaften des Gases abhängig ist, welches die Sensor-Strukturen umgibt. Durch die Messung der Temperatur des Heizers 120 mit zwei identischen Sensoren 130, 140 in unterschiedlichen Abständen $180_1$, $180_2$ zum Heizer 120 lassen sich die unbekannten Wärmeübergänge in der Messanordnung eliminieren oder reduzieren.

Zur Auswertung werden ausgesendete und empfangene periodische Temperaturwellen verglichen (siehe Fig. 9). Mit einer Kalibration des Signals 210, 220 über die Phasenverschiebung 212, 222 zwischen Heizer und den Sensoren kann beispielsweise der $CO_2$-Gehalt in Luft mit 0.2 vol%, z. B. mittels des Gassensors, aufgelöst werden. Da Gase kompressibel sind und durch Druck- und Temperatur ihre Dichte ändern, sollten die entsprechenden Driften kompensiert werden.

Fig. 9 zeigt Signale 210, 220 bei Anregung mit sinusförmiger Heizleistung 122 für $CO_2$ und $N_2$ im Vergleich. Bei gleicher Heizleistung 122 unterscheiden sich die empfangenen Sensorsignale 210, 220 in Amplitude, Offset und Phasenlage. Die Signale 210, 220 sind gemäß einem Beispiel Differenzsignale aus einem Signal einer ersten Thermoelement-Struktur und einer zweiten Thermoelement-Struktur des Gassensors.

Über die Auswertung weiterer Messgrößen, die der Sensor liefert, lassen sich Wärmeleitfähigkeit, Temperaturleitfähigkeit und bei bekannter Dichte des Gases auch die spezifische Wärmekapazität bestimmen - ein möglicher Weg, um auch unbekannte Gasgemische zu analysieren.

Durch den konstruktiven Unterschied freitragender Brückenstrukturen gegenüber geschlossener Dünnschicht-Membranen wird weitgehend die parasitäre thermische Entkopplung zwischen Heizer und Detektor-Elementen erreicht und die Signalqualität deutlich erhöht. Aufgrund der geringen thermischen Masse des Heizers ist es möglich, den Heizer mit Frequenzen bis zu 300 Hertz zu modulieren, da die Wärme schnell zu- und abgeführt werden kann.

**1.2.2 Theoretische Betrachtung zur Bestimmung der Temperaturleitfähigkeit (Details optional)**

Zur Bestimmung der Temperaturleitfähigkeit bei sinusförmiger Heizleistung 122 kann zur Beschreibung des sich ausbreitenden Temperaturfeldes ein Modell nach [**Baehr 2008**] herangezogen werden.

Folgende Gleichung beschreibt die zeitabhängige (Zeit t) Temperaturausbreitung entlang der Längenachse x in einen Stab, der an einem Ende mit einer sinusförmigen Temperatur beaufschlagt wird (Mittelwert $T_m$, Amplitude $T_A$, Winkelfrequenz $\omega$):

$$T(x,t) = T_m + T_A \cdot \eta \cdot e^{-k_1 \cdot x} \cdot \sin\left(2\pi f \cdot t - (k_1 \cdot x + \epsilon)\right) \qquad (1)$$

Beim Eintritt vom Heizer ins gasförmige Medium erfährt das Temperaturfeld die Phasenverschiebung $\varepsilon_0$ und die Dämpfung $\eta_0$.

$$\epsilon_0 = arctan\frac{k}{1+k} \quad und \quad \frac{1}{\eta_0} = \sqrt{1 + 2k + 2k^2} \qquad (2)$$

Das Temperaturfeld erfährt abhängig vom durch das Medium zurückgelegten Weg x die Phasenverschiebung $\varepsilon(x) = k_1 \cdot x$ und die Dämpfung $\eta(x) = e^{-k_1 \cdot x}$. Der wesentliche Faktor für die Änderung der wegabhängigen Größen, $k_1$, hängt nach [**Baehr 2008**] von der Temperaturleitfähigkeit a, der Kreisfrequenz $\omega$ und somit von der Anregungsfrequenz $f$ ab:

$$k_1 = \sqrt{\frac{\omega}{2 \cdot a}} = \sqrt{\frac{\pi \cdot f}{a}} \qquad (3)$$

Der Faktor zur Berücksichtigung der Einflüsse beim Wärmeübergang zwischen Festkörper und Gas ergibt sich aus dem Faktor $k_1$, dem Wärmeübergangskoeffizienten $\alpha$ und der Wärmeleitfähigkeit $\lambda$:

$$k = \frac{k_1 \cdot \lambda}{\alpha} = \frac{b}{\alpha} \cdot \sqrt{\pi \cdot f} \quad \text{mit dem Wärmeeindringkoeffizienten b:} \quad b = \sqrt{\lambda \cdot c_p \cdot \rho} = \frac{\lambda}{\sqrt{\alpha}} \quad (4)$$

Um die Temperaturleitfähigkeit nach dem oben genannten Modell zu bestimmen, ist die Auswertung der Phasenverschiebung ausreichend. Die gesamte Phasenverschiebung in *Gleichung (1)* beträgt:

$$\Delta\varphi = k_1 \cdot x + \epsilon_0 \qquad (5)$$

Werden zwei Temperatur-Messungen in zwei verschiedenen Abständen miteinander verglichen, so kürzen sich die konstanten Wärmeübergangseffekte heraus:

$$\Delta\varphi(x_2) - \Delta\varphi(x_1) = (k_1 \cdot x_2 + \epsilon) - (k_1 \cdot x_1 + \epsilon) \qquad (6)$$

Vereinfacht mit den Differenzen $\Delta\varphi_{12} = \Delta\varphi(x_2) - \Delta_\varphi(x_1)$ und $\Delta x_{12} = x_2 - x_1$

$$\Delta\varphi_{12} = k_1 \cdot \Delta x_{12} \qquad (7)$$

und mit *(3)* ergibt sich:

$$\Delta\varphi_{12} = \sqrt{\frac{\pi \cdot f}{a}} \cdot \Delta x_{12} \qquad (8)$$

Für die Temperaturleitfähigkeit $\alpha$ (mit Winkeln im Bogenmaß) gilt:

$$a = \pi \cdot f \cdot \frac{\Delta x_{12}^2}{\Delta\varphi_{12}^2} \qquad (9)$$

Liegen die Phasenverschiebungen im Gradmaß vor, so gilt für die Temperaturleitfähigkeit $\alpha$:

$$a = \frac{180°^2 \cdot f}{\pi} \cdot \frac{\Delta x_{12}^2}{\Delta\varphi_{12}^2} \qquad (10)$$

Die Temperaturwelle schwingt harmonisch mit derselben Kreisfrequenz wie ihre Anregung und klingt rasch mit zunehmender Eindringtiefe im Medium stark gedämpft ab, während die Phasenverschiebung zunimmt. Eindringtiefe und Wellenlänge wachsen mit Erhöhung von Schwingdauer und Temperaturleitfähigkeit des Mediums. Bei Betrachtung der Wellenlänge $\Lambda$ der Temperaturschwingung, die sich aus dem Abstand zweier Messpunkte $x_1$ und $x_2$ ergibt, bei denen sich der Phasenwinkel um $2\pi$ unterscheidet, lässt sich die Eindringtiefe der Temperaturwelle herleiten, bei der die Temperaturamplitude auf den n-ten Teil ihres Wertes an dem Eintrittspunkt ins Medium x=0 gesunken ist. Es gilt:

aus $e^{-2\pi x_n/\Lambda} = 1/n$ ergibt sich:

$$x_n = \frac{\Lambda}{2\pi} \cdot \ln n = \sqrt{\frac{a}{\pi \cdot f}} \ln n \qquad (4)$$

Die Dämpfung der Amplitude ist somit ebenfalls ein Maß für die Temperaturleitfähigkeit des Mediums.

**1.2.3 Theoretische Betrachtung zur Bestimmung der Wärmeleitfähigkeit (Details optional)**

Die Wärmeleitfähigkeit $\lambda$ des Mediums wird durch die mittlere Temperaturverteilung im Messraum abgebildet. In Abhängigkeit der mittleren Heizertemperatur und der Gasart bzw. Gemisch-Konzentration im Volumen des Messraumes stellt sich an den Temperatur-Detektoren eine mittlere Temperatur ein, die in einem Verhältnis zum Wärmestrom steht, der durch das gasförmige Medium vom Heizer über die Detektoren bis zur Gehäusewandung fließt. Zur Bestimmung der Wärmeleitfähigkeit müssen die Temperaturen des Heizers und der Detektoren bekannt sein, bei entsprechender Kalibrierung genügt z. B. die Regelung eines Detektors (bevorzugt des zum Heizer näher liegenden Detektors) auf eine konstante (Über-)Temperatur, wenn als Maß für die Wärmeleitfähigkeit die benötigte mittlere Heizenergie ermittelt wird.

Nach **[Simon 2002]** und **[Baar 2001]** besteht das grundlegende Prinzip zur Messung der Wärmeleitfähigkeit von Gasen darin, dass in einem strömungsfreien Messraum mit einem im Gas freistehenden Heizer-Element (z. B. ein Hitzdraht oder eine "Hot Plate" - heiße Platte) eine Übertemperatur über der Umgebungstemperatur erzeugt wird. Die benötigte Heizleistung, um diese Übertemperatur $\Delta T$ aufrechtzuerhalten, ist das direkte Maß der Wärmeleitfähigkeit $\lambda$ und kann mit folgendem Zusammenhang beschrieben werden:

$$P = \lambda \cdot \Delta T \cdot G \qquad\qquad (5)$$

wobei G die geometrische Konstante der Anordnung darstellt. Bedingung zu korrekten Messung ist ein ruhendes Gas im Messraum, z. B. in einem Totvolumen oder hinter einer Diffusionsbarriere, da konvektive Wärmeströmung zu einem Messfehler führt **[Baar 2001]**. In der Literatur werden sowohl diese Messfehler diskutiert, als auch Verfahren vorgeschlagen, die die Wärmeleitfähigkeit in Gegenwart einer konvektiven Wärmeströmung messen können [**IST AG 2011, 2013, 2015**]. Weiterhin sind Verfahren mit periodischer Anregung des Heizers bekannt, über Fourier-Analyse nicht nur die Konzentration binärer Gasgemische, sondern auch mehrere Komponenten Gemisch bestimmen können [Grien 2012].

**1.2.4 Embedded μController (Eingebetteter Mikrocontroller) Elektronik und Software eines Gassensors (Details optional)**

Aufgabe der Elektronik und Signalauswertung ist, mit einem möglichst preisgünstigen miniaturisierten System z. B. ein zuverlässiges von der Gaskonzentration direkt abhängiges Messergebnis zu generieren. Zudem soll der erfindungsgemäße Gassensor in einem Atemgasmonitor einsetzbar sein, bei dem sich mit hoher Dynamik die Kohlenstoffkonzentration im Luftgemisch ändern kann. So soll der Gassensor Änderungen der Gaszusammensetzung im Beatmungszyklus der Inspiration und Exspiration bis zu einer Rate von 60 Hüben pro Minute auflösen können. Es ist somit eine schnelle Auswertung der Sensorsignale wünschenswert.

1.2.4.1 Hardware

*1.2.4.1.1 Beispielhafte Heizer-Ansteuerung des Gassensors*

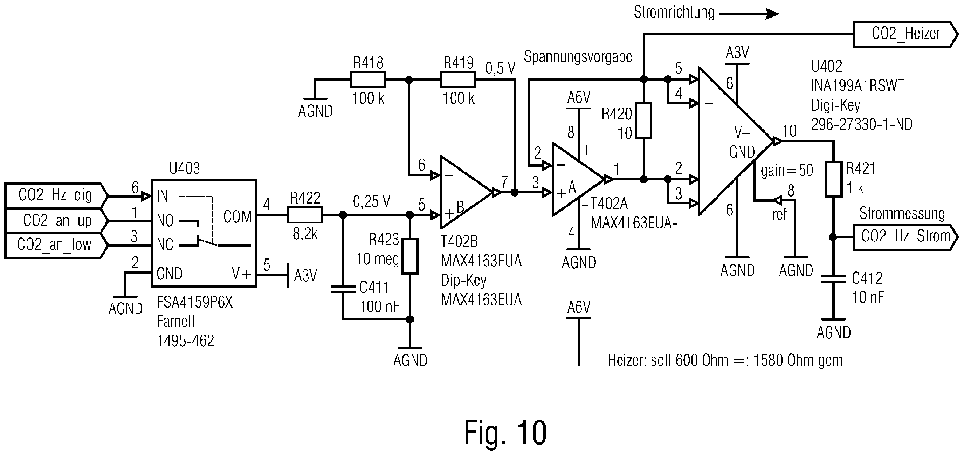

Fig. 10 zeigt ein elektrisches Schaltbild einer Heizer-Ansteuerung für einen thermischen Gassensor. Eine CPU gibt z. B. eine untere und obere Heizerspannung vor und schaltet Timer gesteuert zwischen diesen beiden Werten hin und her. Eine CPU kann zu bestimmten Zeitpunkten den aktuellen Heizstrom messen, um die Heizleistung zu berechnen. In anderen Worten stellt Fig. 10 eine Heizer-Versorgung mit Spannungs-Vorgabe und Strom-Messung dar.

Gegenüber der in der weiter oben theoretisch betrachteten Analogie bei der Übertragung der Gesetzmäßigkeiten für eine gedämpfte Schwingung auf ein Wärmetransport-Phänomen am Beispiel einer sinusförmigen Heizer-Anregung wird auf der entwickelten μController Elektronik (beispielsweise) ein Rechtecksignal erzeugt. Dieses lässt sich aufgrund der im Prozessor vorhandenen Timer-Strukturen zeitlich wesentlich präziser erzeugen, als ein synthetisches Sinussignal, dass vom Prozessor auf dessen Digital/Analog (DA) Port ausgegeben würde.

Über einen DA-Wandler werden beispielsweise 2 Heizer-Spannungen vorgegeben. Dies ist der Tatsache geschuldet, dass der DA-Wandler über SPI angesteuert wird, und der Zeitpunkt, ab dem ein neuer DA-Wert übernommen wird, nicht exakt mit dem gewählten Prozessor-Baustein (CPU) bestimmbar ist. Das ist aber Voraussetzung um die Phasenlage der Sensor-Antwort bestimmen zu können. Deshalb wird beispielsweise über einen Analog-Schalter abwechselnd eine der beiden Spannungen auf den Heizer-Verstärker gegeben. Damit die steilen Schaltflanken sich weniger im System ausbreiten, werden diese beispielsweise durch einen nachgeschalteten Tiefpass verschliffen. Die Operationsverstärker (OP) Schaltung hebt die Spannung auf den vom Heizer benötigten Spannungspegel an. Ein weiterer OP kompensiert beispielsweise den am Strom-Messwiderstand entstehenden Spannungsabfall. Da der Strom gemessen und die Heizer-Spannung bekannt ist, kann die Heizer-Leistung berechnet werden. Dies ist insofern wichtig, als sich der Heizer-Widerstand mit der Temperatur verändern kann.

Beispielsweise kann ein Heizer-Tastverhältnis von 50% verwendet werden (wobei beispielsweise ein periodisches Rechtecksignal mit einem Tastverhältnis von beispielsweise 50% +/-2% an den Heizer angelegt wird).

Alternativ können aber auch kürzere Tastverhältnisse verwendet werden, zum Beispiel im Bereich 5..50%.

Um eine gleiche Leistung zwischen einem Sinus (Offset bei Upp/2, beide Halbwellen im positiven Bereich) und einem Rechteck zu erhalten, benötigt man beispielsweise für den gleichwertigen Rechteck bzw. für ein "gleichwertiges" oder leistungsgleiches Rechtecksignal ein Tastverhältnis von 42%.

In einigen Beispielen ist die Anpassung der Heizerleistung durch Regelung des Tastverhältnisses nicht realisiert- dies ist schwieriger auf dem MSP430, doch interessant bei Verwendung leistungsfähigerer µController: Man kann mit fester Betriebsspannung arbeiten und ändert nur das Tastverhältnis (eine Art PWM-Ansteuerung).

In anderen Worten, optional ist es möglich, die (mittlere) Heizerleistung durch Änderung des Tastverhältnisses einzustellen. Alternativ dazu kann die Heizerleistung durch Änderung der Spannungspegel (der an den Heizer angelegten Spannung) bzw. der Strompegel (des durch den Heizer bzw. das Heizelement fließenden Stromes) einzustellen. Die beiden Möglichkeiten sind auch kombinierbar.

### 1.2.4.1.2 Detektor-Signalauswertung z. B. des Gassensors (Details optional)

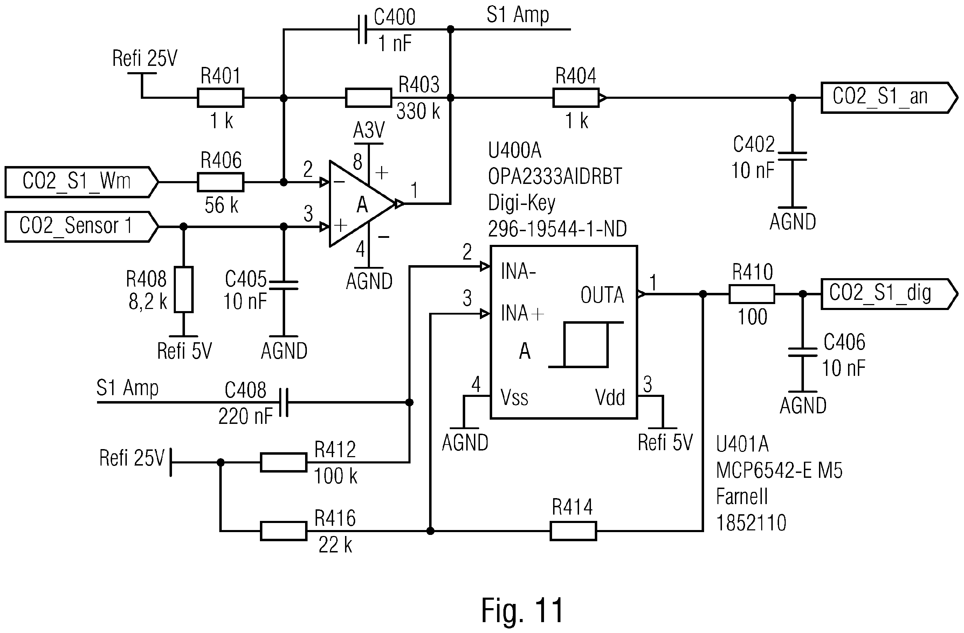

Fig. 11 zeigt ein elektrisches Schaltbild einer Detektor-Signalauswertung eines thermischen Gassensors. Dabei kann sowohl eine erste Thermoelement-Struktur als auch eine zweite Thermoelement-Struktur des Gassensors die in Fig. 11 dargestellte Detektor-Signalauswertung aufweisen, um ein von einem Heizelement des Gassensors an die erste Thermoelement-Struktur und die zweite Thermoelement-Struktur über ein zu analysierendes Gas übertragene Wärme in einem jeweiligen Detektorsignal (z. B. detektiert mittels der ersten Thermoelement-Struktur oder der zweiten Thermoelement-Struktur und kann hierin auch als Sensorsignal bezeichnet werden) auszuwerten. Beispielhaft wird in Fig. 11 die Detektor-Signalauswertung des Sensors 1 (erste Thermoelement-Struktur) dargestellt. Dabei ist die Detektor-Signalauswertung z. B. konfiguriert, um ein erstes Eingangssignal, z. B. ein DAC Signal CO2_S1_Win von einer CPU (Lupenfunktion), und ein zweites Eingangssignal, z. B. ein Detektorsignal CO2_Sensor1, zu empfangen und um ein erstes Ausgangssignal, wie z. B. ein verstärktes Detektorsignal CO2_S1_an, und ein zweites Ausgangssignal, wie z. B. ein Komparator Signal für eine Phasenauswertung CO2_S1_dig, bereitzustellen.

Gemäß einem Beispiel steuert eine CPU einen Heizer, so dass eine Amplitude des Sensorsignals in einem ADC-Bereich bleibt. Über eine Lupenfunktion wird z. B. das Sensorsignal innerhalb der ADC-Grenzen gehalten. Eine Phasenauswertung erfolgt z. B. über den Komparator unter Ausnutzung der MSP430 Timer-Strukturen (Zeitstrukturen).

Eine Widerstandsänderung des Sensor-Drahts (z. B. der Thermoelement-Struktur) ist sehr gering. Aus diesem Grund wird ein Verstärker mit hohem Verstärkungsfaktor bevorzugt oder benötigt. Da ein Absolut-Wert einer Eingangsspannung (z. B. der Sensorsignale) von vielen Faktoren abhängt, ist es empfehlenswert, diesen Wert zu kompensieren.

Eine Möglichkeit wäre einen Wechselstrom (AC) Verstärker zu verwenden. Nachteil ist, dass dieser eine unbekannte Phasenverschiebung verursacht.

Deshalb wurde beispielsweise ein Gleichstrom (DC) Verstärker verwendet, der keine Phasenverschiebung aufweist. Um nun den Gleichanteil des Signals zu kompensieren, wird beispielsweise am Differenzeingang des Operationsverstärkers (OP) der negative Eingangsanschluss auf den Mittelwert des Detektor-Signals angehoben und mithilfe eines Software-Reglers aktiv nachgeführt, der Digital-Analog-Umsetzer (DAC) des

Prozessors gibt diese Spannung direkt aus. Durch die differentielle Arbeitsweise des Differenzeinganges am OP werden die Gleichanteile der Eingangsspannungen voneinander subtrahiert und allein der Wechselanteil des Signals verstärkt. Dazu wird gemäß einem Aspekt das von Analog zu Digital umgesetzte (ADC) Signal gemessen und überprüft, ob dieses innerhalb vernünftiger, vom ADC erfassbaren Grenzen liegt. Stößt das Signal an die obere oder untere Spannungsgrenze des OPs an, so wird der DAC-Wert entsprechend angepasst. Es entsteht also ein Verstärker, bei dem das verstärkte Signal kontinuierlich im optimalen Arbeitsbereich oder Arbeitsfenster gehalten wird, wobei durch Entfernung des Gleichanteils der Verstärkungsfaktor am OP erhöht werden kann, eine Art "Lupenfunktion". Der zur Kompensation notwendige DAC-Wert kann als weiterer Parameter zur Auswertung dienen, mit dem die absolute mittlere Detektor-Temperatur bestimmt werden kann und über den Zusammenhang aus Gleichung (5) die Bestimmung der Wärmeleitfähigkeit des Gasgemisches erfolgen kann.

Um die Phasenlage des Sensor-Signals zu bestimmen, wurde beispielsweise ein Schmitt Trigger verwendet. Er ist so eingestellt, dass er kurz über bzw. unter dem Nulldurchgang des Sensor-Signals schaltet. Hier ist das Signal am steilsten und verursacht damit das kleinste Phasenrauschen. Der DC-Anteil wird beispielsweise über einen Kondensator entfernt. Damit ist eine Phasenbestimmung der Sensor-Antwort möglich.

Durch die Verwendung der internen Timer-Strukturen des Prozessors (MSP430, Texas Instruments) ist eine theoretische Phasenauflösung von 0,009° möglich. Diese wird jedoch durch Rauschen der Schaltung nicht erreicht.

1.2.4.2 Software (Details optional; Funktionalitäten gemäß Aspekte 3 und 4 sind hier zusammmen beschrieben, können aber separat voneinander eingesetzt werden) z. B. für den Gassensor

Die Software hat z. B. verschiedene Aufgaben:

- Setzen der Start-Werte für Heizer-Spannung, Abtastzeitpunkte der Sensor-Signale und des Startwerts für den DC-Arbeitspunkt (Lupen-Funktion).
- Zuerst wird beispielsweise versucht, den DC-Arbeitspunkt zu finden. Dazu werden die DAC-Werte der beiden Sensoren so eingestellt, dass sich das Sensor-Signal beispielsweise mittig im ADC-Range befindet.
- Messen der Sensorspannung zu bestimmten Zeitpunkten. Um die Amplitude zu ermitteln, wird die Spannung im vermuteten Maximum und Minimum erfasst. Um zu erkennen, dass der Abtastzeitpunkt falsch gewählt ist, erfolgt noch eine Messung im vermuteten "Nulldurchgang". Stimmen die Abtastzeitpunkte, so gilt z. B.:

$$\frac{Umax + Umin}{2} = U0$$

Sind die Abtast-Zeitpunkte falsch, so stimmt obige Formel nicht mehr. Daran kann die Software beispielsweise erkennen, dass die Abtastzeitpunkte angepasst werden müssen. Die Nachregelung kann beispielsweise über Software deaktiviert werden. Sie erfolgt auch nur, wenn das Signal innerhalb der ADC-Grenzen liegt.

- Ist der Amplitudenregler aktiv, so wird beispielsweise versucht, die Amplitude vom Sensor1-Signal auf einem gewissen Sollwert zu halten. Die Heizer-Energie wird beispielsweise derart geregelt, dass die S1-Amplitude den ADC-Range um wenigstens 3/4 ausfüllt. Der Regler kann optional per Software abgeschaltet werden. Zudem ist er beispielsweise nur aktiv, wenn keine Abtastzeitpunkte oder DC-Offsets geändert wurden. Dies stellt optional sicher, dass dieser Regelkreis nur im eingeschwungenen Zustand aktiv ist.
- Bestimmung der Phasenlage des Sensorsignals mit Hilfe der Schmitt-TriggerSchaltung (Optional). Je nach Einstellung erfolgt dabei auch die Berechnung der 3 Abtastzeitpunkte des Sensorsignals für die nächste Abtast-Periode.
- Über weitere Sensoren wird der Umgebungsdruck und die Temperatur erfasst (optional)

1.2.4.3 Beispielhafter Software-Regler z. B. für den Gassensor

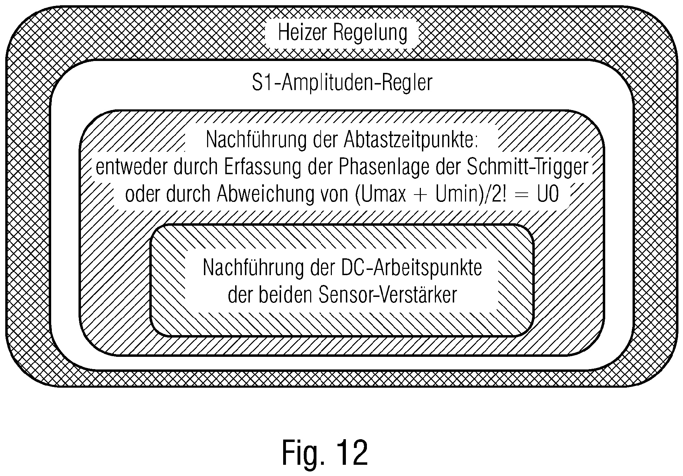

Fig. 12 zeigt eine schematische Darstellung ineinander geschachtelter Regler der Software für einen thermischen Gassensor.
In der Software arbeiten mehrere ineinander geschachtelte Regler. Der innerste ist der DC-Arbeitspunkt-Regler. Beispielsweise nur wenn dieser eingeschwungen ist (keine Anpassung des DC-Offsets notwendig war), dann erfolgt die Nachführung der Abtastzeitpunkte. Im Amplituden-Regelkreis wird die Amplitude von S1 beispielsweise konstant gehalten - aber beispielsweise nur wenn keine Anpassung des DC-Offsets und Abtastzeitpunkts notwendig war. Im äußeren Regelkreis kann (optional) die benötigte Heizenergie zur Ausregelung der S1-Amplitude derart eingeregelt werden, dass sich das thermische System auf eine große Bandbreite verschiedener Gasgemische dynamisch adaptieren kann.
Für die Amplituden-Bestimmung werden beispielsweise 3 A/D-Samples pro Sensor-Draht benötigt: Minimum im unteren Scheitelpunkt, Nulldurchgang und Maximum im oberen Scheitelpunkt, Der Ablauf ist beispielsweise folgendermaßen:

- Alle AD-Werte werden beispielsweise zunächst mit der aktuellen Einstellung gemessen.
- Nun wird beispielsweise geprüft, ob die Min/Max-A/D-Werte für S1 und S2 im gültigen Bereich liegen. Falls nicht, erfolgt eine Nachstellung des DC-Arbeitspunktes des Verstärkers (über DAC) und alle weiteren Regler werden vorübergehend abgeschaltet. Erst wenn beide Sensor-Kanäle im erlaubten Arbeitsbereich liegen (A/Dmax < 3900 bzw. A/D$_{min}$ > 200, d. h. im Bereich von 5..95% des A/D-Ranges von 4096 digits), werden die weiteren Regler wieder aktiv.
- Damit die Amplitude richtig gemessen werden kann, sollte die A/D-Wandlung zur richtigen Zeit erfolgen (oberer/unterer Scheitelpunkt und zur Überprüfung noch den Nulldurchgang). Dazu gibt es derzeit beispielsweise zwei Wege:

  ◦ Über die A/D-Wandlung selbst: Der Zeitpunkt des Nulldurchganges wird in der halben Zeit zwischen den beiden gemessenen Zeitpunkten für die minimalen und maximalen Scheitelpunkt der A/D-Werte erwartet, d. h., (Min+Max)/2 sollte gleich dem A/D-Wert beim Nulldurchgang entsprechen. Bei Abweichungen wird der Sample-Zeitpunkt für die nächste Messung angepasst. Eine Abweichung von beispielsweise rund 0,625° (Grad) oder 14,47 µs wird toleriert.

◦ Über das Komparator-Signal: Da der Komparator zum Zeitpunkt des Nulldurchgangs des Sensor-Signals schaltet, kann beispielsweise der Zeitpunkt, an der die A/D-Messungen stattfinden sollen, ermittelt werden: Zum Messwert des Umschaltzeitpunktes der positiven Flanke werden 90° (bzw. 2,0833 ms für den oberen Scheitelpunkt), 180° (4,1666 ms für den Nulldurchgang der negativen Flanke) und 270° (6,2499 ms für den unteren Scheitelpunkt) addiert. Auch hier wird eine Abweichung von 0,625° toleriert.

• Beispielsweise nur wenn beide Regler (DC-Arbeitspunkt und Phase) keine Stellwert-Änderung benötigt hatten, sich demnach im eingeschwungen Zustand befanden, dann erst greift der Amplituden-Regler. Dieser stellt den Heizer-Wert so nach, dass die gewünschte Amplitude von S1 erreicht wird.

In Fig. 12a wird ein Blockdiagramm gezeigt, das eine beispielhafte Regelung und Nachführung der DC-Arbeitspunkte der beiden Detektor-Verstärker darstellt.

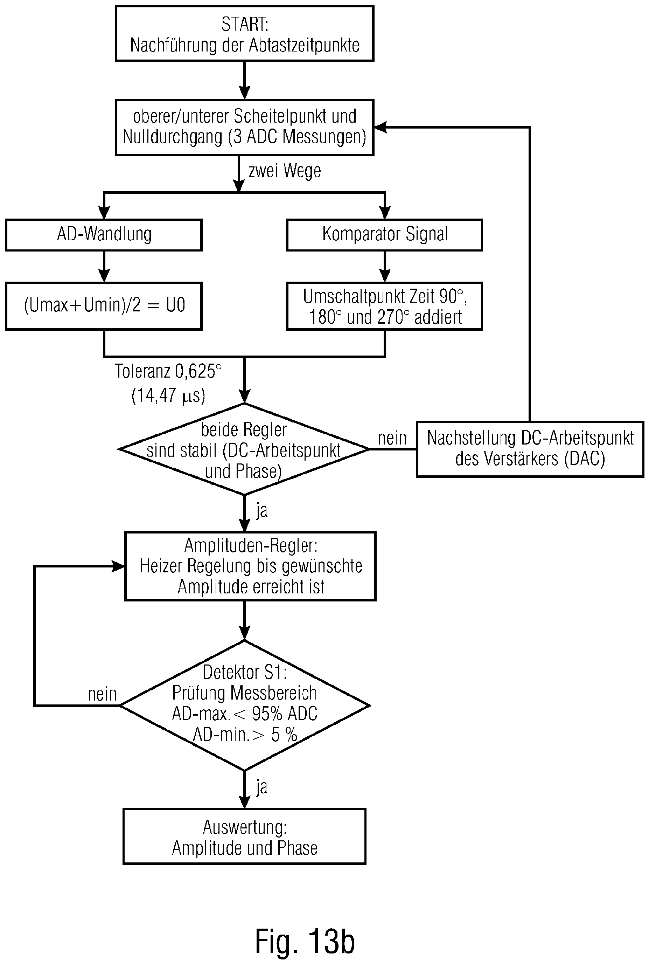

In Fig. 13b wird ein Blockdiagramm gezeigt, das eine beispielhafte Nachführung der Abtastzeitpunkte für die Amplituden-Messung der Detektor-Signale und S1-Amplituden-Regler darstellt. Wenn alle Regler eingeregelt sind, erfolgt z. B. die Auswertung des Gasgemisches mit den gemessenen Werten für Amplitude und Phase der Detektoren.

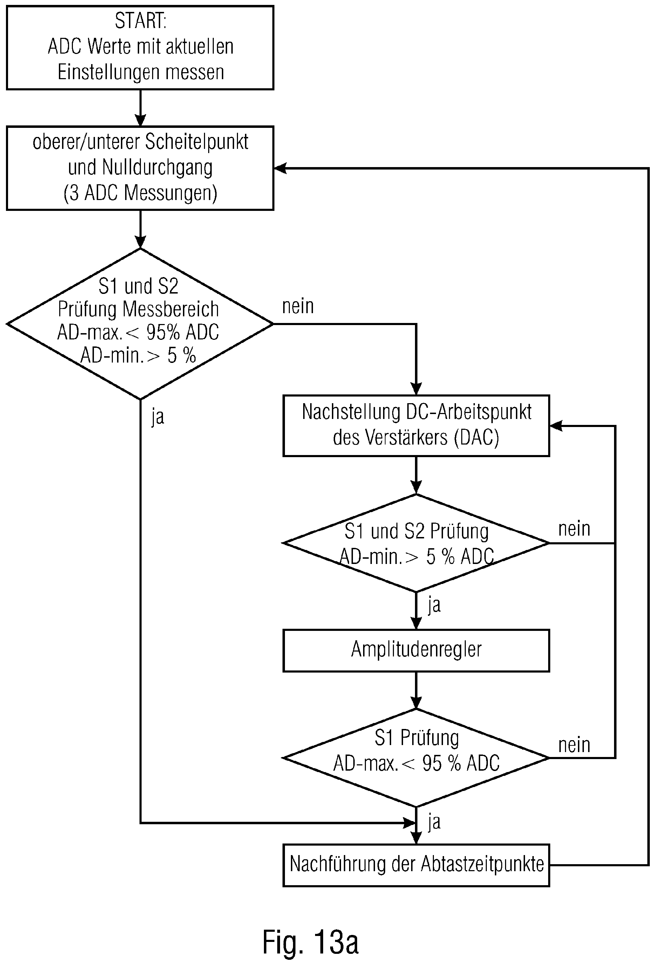

Gemäß einem Beispiel können die Figuren 13a und 13b als ein Blockdiagramm angesehen werden, wobei Fig. 13b mit Fig. 13a über den Block "Nachführung der Abtastzeitpunkte" verbunden ist.

1.2.4.4 Beispielhafte Timing-Tabelle (Zeitgebungs-Tabelle) z. B. für den Gassensor

Die ADC-Messzeitpunkte, an denen der Analog-Digital-Wandler des $\mu$Controllers die Stromaufnahme des Heizers und die Detektor-Spannungen (ein Beispiel für die Sensorsignale) misst, sind z. B. in einer Timing-Tabelle der Software definiert, die sich über zwei Heizer-Puls-Perioden erstreckt. Diese zwei Perioden werden deshalb zum Beispiel benötigt, weil auf dem verwendeten Prozessor z. B. nur ein Timer (Zeitgeber) zur variablen ADC-Steuerung zur Verfügung steht. Bei Betrieb des Heizers mit 120 Hz erhält man nach Ablauf von 2 Perioden alle zur Gasgemisch-Auswertung relevanten Messwerte, d. h. mit einer Frequenz von 60Hz. Da die Pulsform des Heizers über die Periode stabil ist, kann der aufgenommene Heizer-Strom zu festen Zeitpunkten gemessen werden: Bei 45° für den Spitzen-Wert und bei 170° für den unteren Heizstrom-Wert (im allgemeinen Null). Die jeweils 3 ADC-Messwerte pro Detektor (oberer und unterer Scheitelpunkt sowie der Nulldurchgang) werden als variable Messwerte in Zeitfenstern erwartet, die in der Timing-Tabelle definiert sind:

• ADC_SENSOR1:

 ◦ CO2-S1-min: 33,6° .. 123,6° (778 $\mu$s .. 2861 $\mu$s)
 ◦ CO2-S1-Null: 123,6° .. 213,6° (2861 $\mu$s .. 4944 $\mu$s)
 ◦ CO2-S1-max: 213,6° .. 303,6° (4944 $\mu$s .. 7028 $\mu$s)

• ADC_SENSOR2:

 ◦ CO2-S2-min: 68,6°-141,4° (1588 $\mu$s .. 3273 $\mu$s)
 ◦ CO2-S2-Null: 158,6°-231,4° (3671 $\mu$s .. 5356 $\mu$s)
 ◦ CO2-S2-max: 248,6° .. 321,4° (5755 $\mu$s .. 7440 $\mu$s)

**1.3 Auswertealgorithmus zur Kalibration auf ein Gasgemisch mit Drift-Korrektur gegenüber Gas-Druck und Gas-Temperatur z. B. eines Gassensors**

**1.3.1 Messungen in Gasgemischen**

1.3.1.1 Binäres Gemisch

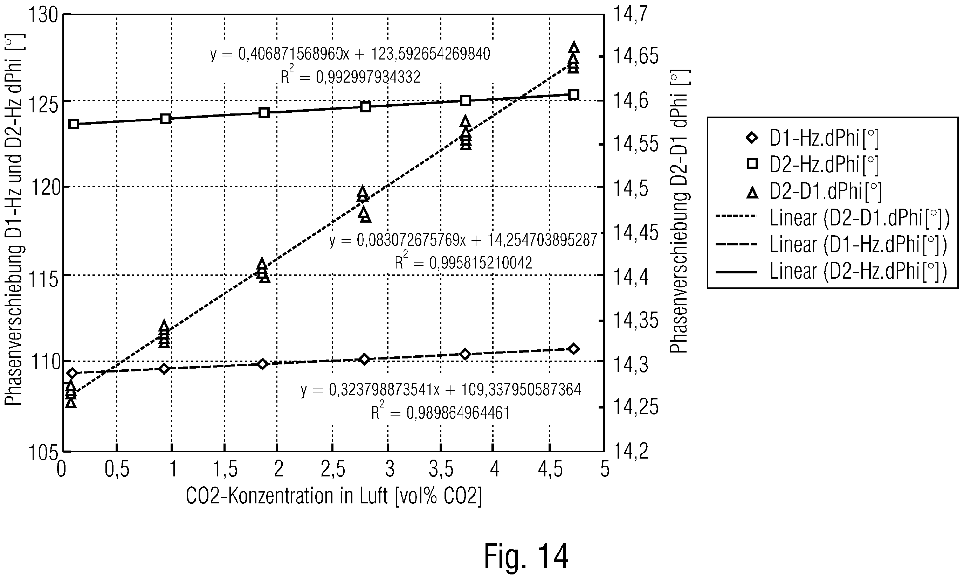

In Fig. 14 ist beispielhaft eine $CO_2$ Abhängigkeit des Sensors im Phasensignal bei konstanter Temperatur und konstantem Druck dargestellt. Dabei werden beispielsweise drei Phasenverschiebungen dargestellt: Eine Phasendifferenz D1-Hz.dPhi (rot) zwischen Heizer und Detektor 1, mit einem 200 $\mu$m Abstand, eine Phasendifferenz D2-Hz.dPhi (blau) zwischen Heizer und Detektor 2, mit einem 300 $\mu$m Abstand und eine Phasendifferenz D2-D1.dPhi (grün, rechte y-Achse) zwischen Detektor 2 und Detektor 1. Gemäß einem Ausführungsbeispiel stellt Fig. 14 Phasenverschiebungen Heizer-Detektoren für (0...5)vol% $CO_2$ in Luft bei einem Druck von p=1010 mbar, einer Temperatur von $T_{amp}$=24°C und einer Heizleistung von P=(15±12,5) mW bei einer Frequenz von f=120 Hz dar.

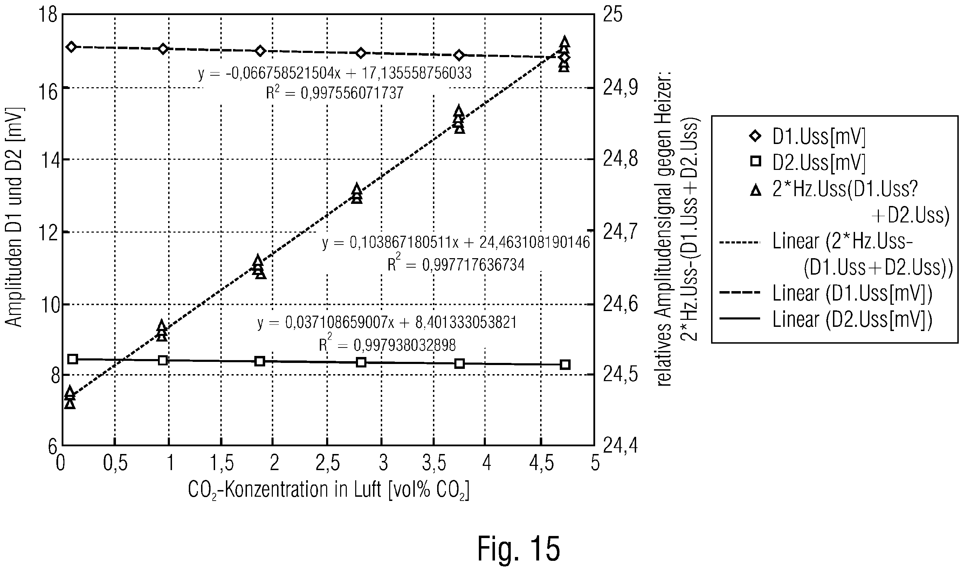

In Fig. 15 sind beispielhaft gemessene Amplituden an den Detektoren D1 und D2 sowie ein relativ zur Heizer-

EP 3 735 580 B1

Amplitude gebildetes Summensignal der Amplituden über die $CO_2$ Abhängigkeit des Sensors dargestellt. Dabei werden beispielsweise die Amplitude D1.Uss (rot) an Detektor 1 und die Amplitude D2.Uss (blau) an Detektor 2 dargestellt. Beide Amplituden-Signale fallen z. B. bei einer Erhöhung der CO2-Konzentration, d. h. bei Erhöhung der Temperaturleitfähigkeit im Gasgemisch. Durch Differenzbildung aus Heizer-Amplitude und der Summe der Detektor-Amplituden wird das relative Amplituden-Signal sigUss=2*Hz.Uss-(D1.Uss+D2.Uss) (grün, rechte y-Achse) mit Erhöhung des CO2-Gehalts im Gasgemisch z. B. steigen. Gemäß einem Ausführungsbeispiel stellt Fig. 15 die Amplituden der Detektoren für (0...5) vol% $CO_2$ in Luft bei einem Druck von p=1010 mbar, einer Temperatur von $T_{amp}$=24°C und einer Heizleistung von P=(15±12,5) mW bei einer Frequenz von f=120 Hz dar.

1.3.1.2 Druck Abhängigkeit

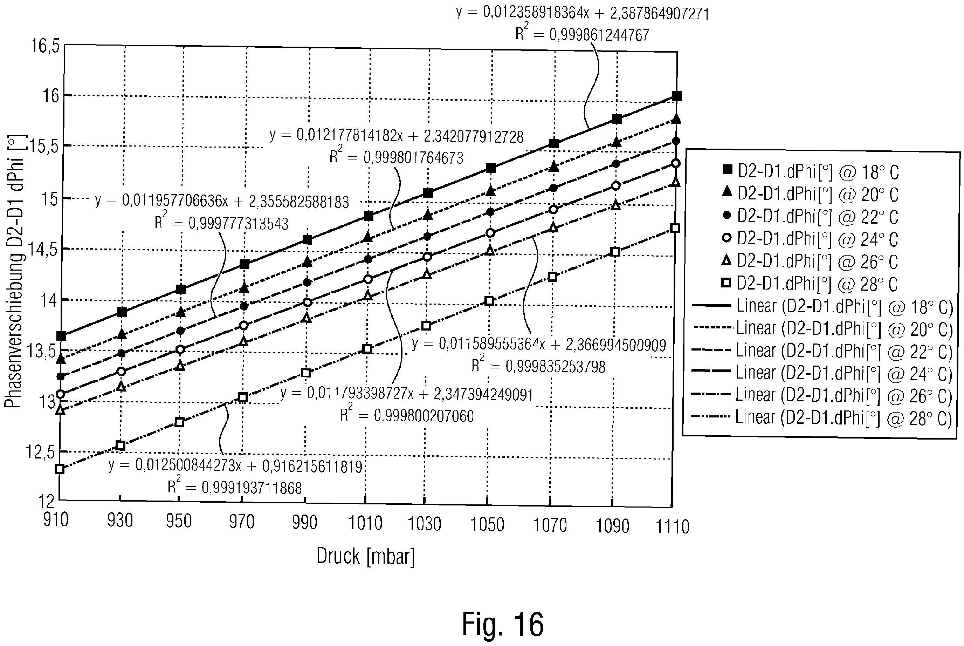

Ein Sensorsignal kann stark von Druck und Temperatur abhängig sein. Zur korrekten Bestimmung der Gaseigenschaften sollten deshalb die Quereffekte bekannt sein und von den Algorithmen korrigiert werden. In Fig. 16 ist beispielsweise die Querempfindlichkeit des Sensorsignals in Luft gegenüber dem Absolutdruck und für unterschiedliche Temperaturen dargestellt. Es ist beispielsweise die Querempfindlichkeit einer PhasenVerschiebung D2-D1 zwischen den Detektoren D2-D1 (z. B. zwischen der ersten Thermoelement-Struktur D1 und der zweiten Thermoelement-Struktur D2) für Luft gegenüber Druck p=(910...1110) mbar über unterschiedliche Temperaturen $T_{amp}$=(18...28)°C in Luft bei einer Heizleistung von P=(15±12,5) mW mit einer Frequenz von f=120 Hz dargestellt.
Der Druck-Einfluss zeigt einen linearen Zusammenhang, der Temperatur-Einfluss einen quadratischen wie theoretisch berechnet. Beide Querempfindlichkeiten liegen in der Größenordnung des Signals für die Gaskonzentration selbst.

1.3.1.3 Heizleistung und Frequenz-Abhängigkeit

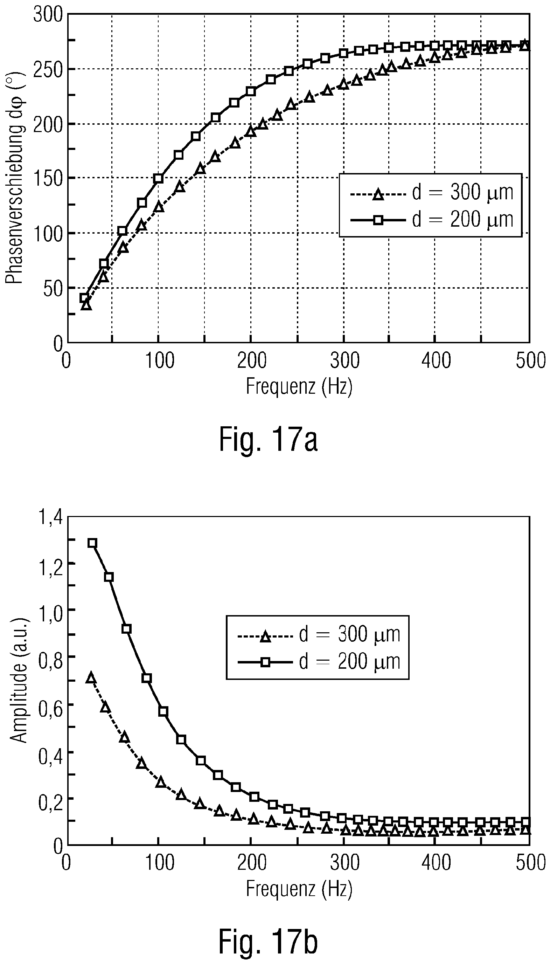

Fig. 17a zeigt eine Darstellung eines Sensorsignals für Phase über der Frequenz bei Messung in $CO_2$. In anderen Worten zeigt Fig. 17a ein Diagramm einer Phasenverschiebung in 100% $CO_2$ in Abhängigkeit der Frequenz. Die Phase geht in Sättigung.
Fig. 17b zeigt eine Darstellung eines Sensorsignals für Amplitude über der Frequenz bei Messung in $CO_2$. In anderen Worten zeigt Fig. 17b ein Diagramm der Amplitude in 100% $CO_2$ in Abhängigkeit der Frequenz. Die Amplitude sinkt gegen Null.
Verglichen mit Luft sollte bei Messungen in Brenngasen die Heizleistung etwas reduziert werden, damit das System nicht seinen A/D Bereich überschreitet. Bei der Heizleistungs-Variation hat sich herausgestellt, dass es in der Praxis sinnvoll ist, das System mit größtmöglichen Sensor-Amplituden zu betreiben und somit stabilere Signale zu erhalten, als die Heizleistung auf ein Minimum einzustellen, bei der das Probegas zwar thermisch weniger beeinflusst wird, jedoch auch der Signal-Rausch-Abstand sinkt. Die in den Sensor periodisch eingetragene Heizenergie muss das Probevolumen innerhalb dieser Periode z. B. verlassen können, damit es sich nicht stetig aufheizt. Bei drei aufgebauten Mess-Systemen wurde beispielsweise eine Spitzen-Heizleistung von ca. 26 mW bei 120 Hz fest eingestellt.
Das Sensorverhalten stellt einen idealen Tiefpass 1. Ordnung dar, es existieren keine oberwelligen Spektralanteile im Sensorsignal. Aus diesem Grund ergibt ein aktives Wobbeln durch ein Frequenzspektrum keine zusätzliche Information. So wurde beschlossen, den Sensor bei einer fixen Frequenz zu betreiben, der Elektronik-Aufwand für das System konnte reduziert werden, die notwendige Messzeit bis zum Erhalt eines gesicherten Wertes ist deutlich kürzer. (alles optional)
Je höher die Anregungsfrequenz am Heizer wird, desto weniger Energie kann zwischen Heizer und Detektor durch das Gas übertragen werden, weil die thermischen Massen des Sensors selbst die Übertragungsgeschwindigkeiten zwischen Festkörper und Gas begrenzen. Die Amplitude sinkt mit zunehmender Frequenz zu einem verschwindenden Signal gegen Null (siehe Fig. 17b), die Phasenverschiebung sättigt sich auf ein Maximum (siehe Fig. 17a).
Die Bildung eines Optimums aus Phasenauflösung, Phasendifferenz und Amplitude für verschiedene Gasgemische ergab die beste Phasenantwort bei einer Frequenz von z. B. 120 Hz bei einer Heizleistung von 26 mW für den Mikrodraht-Sensor und von 160 Hz bei ca. 8 mW für den MEMS Thermopile-Sensor auf Dünnschicht-Membran. (Details optional)

1.3.1.4 Brenngas-Gemische

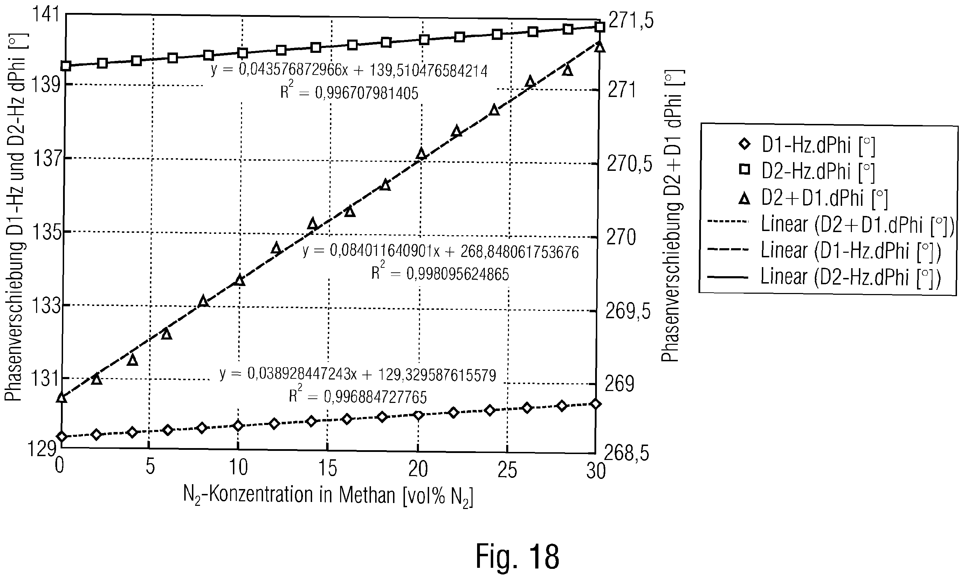

Auf einem Messplatz wurden unterschiedliche Gas-Zusammensetzungen untersucht. Fig. 18 zeigt eine Veränderung eines Phasen-Signals eines Sensors für Methan bei zunehmender Zumischung von Stickstoff als nahezu lineares Verhalten. Es ist beispielsweise das Phasen-Signal in Abhängigkeit der Stickstoff-Konzentration in Methan als Phasendifferenz D1-Hz.dPhi (rot) zwischen Heizer und Detektor 1, mit einem 200$\mu$m Abstand, eine Phasendifferenz D2-Hz.dPhi (blau) zwischen Heizer und Detektor 2, mit einem 300$\mu$m Abstand, und eine Phasendifferenz D2-D1.dPhi (grün, rechte y-Achse) zwischen Detektor 2 und Detektor 1 dargestellt. Dabei ist die Phasenverschiebung Heizer-Detektoren gemäß einem Ausführungsbeispiel für (0...30) vol% $N_2$ in Methan bei einem Druck von p=990 mbar, einer Temperatur $T_{amp}$=21°C und einer Heizleistung von P=(13±12,5)mW mit einer Frequenz von f=120 Hz in Fig. 18 dargestellt.

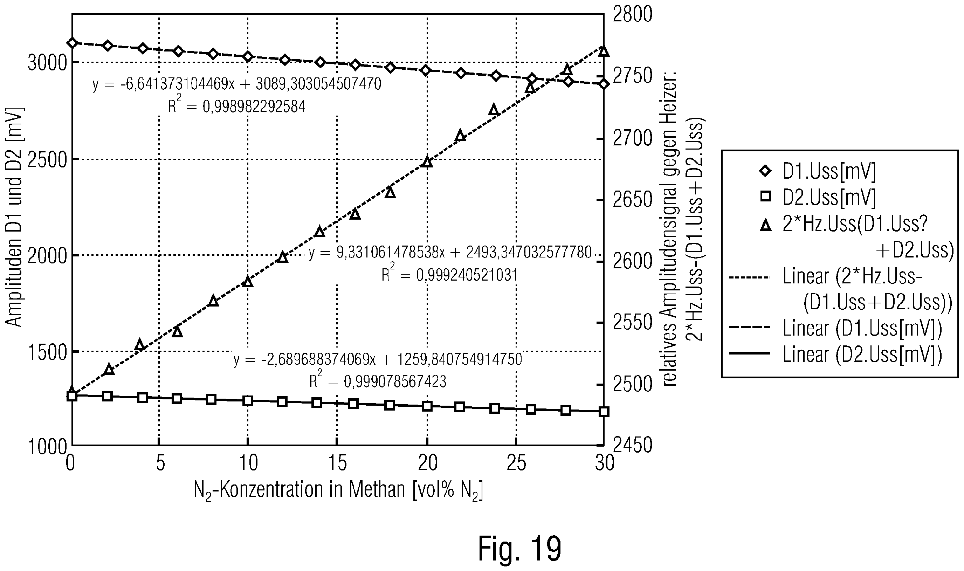

Fig. 19 zeigt ein Diagramm der Amplitude D1.Uss (rot), erfasst mittels des ersten Detektors, und der Amplitude D2.Uss (blau), erfasst mittels des zweiten Detektors. Dabei sind die Amplituden der Detektoren gemäß einem Ausführungsbeispiel für (0...30) vol% $N_2$ in Methan bei einem Druck von p=990 mbar, einer Temperatur $T_{amp}$=21°C und einer Heizleistung von P=(13±12,5)mW mit einer Frequenz von f=120 Hz in Fig. 19 dargestellt. Beide Amplituden-Signale D1.Uss und D2.Uss fallen z. B. bei Erhöhung der $N_2$-Konzentration in Methan, d. h. bei Verringerung der Temperaturleitfähigkeit im Gasgemisch. Durch Differenzbildung aus Heizer-Amplitude und der Summe der Detektor-Amplituden steigt z. B. das relative Amplituden-Signal sigUss =2*Hz.Uss-(D1.Uss+D2.Uss) (grün, rechte y-Achse) mit Erhöhung der $N_2$-Konzentration an.

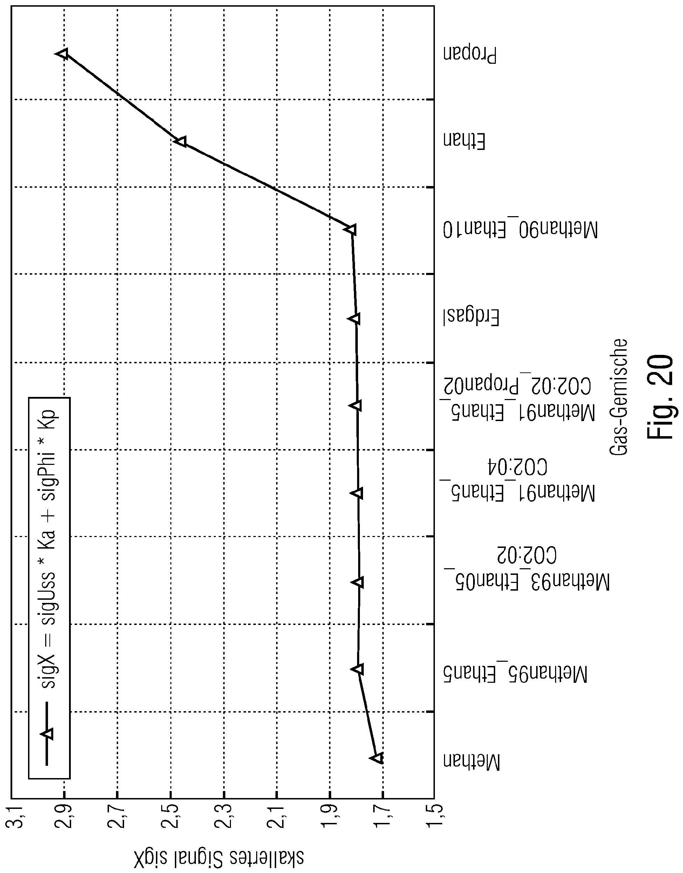

Fig. 20 zeigt ein Diagramm eines berechneten Sensorsignals sigX (ein Beispiel für ein Kombinationssignal des Gassensors) aus Phase und Amplitude für verschiedene Brenngas-Gemische. Fig. 20 zeigt somit das Sensorsignal (ein Beispiel für ein Kombinationssignal des Gassensors) für verschiedene Brenngase und deren Gemische: Methan, Ethan und Propan, sowie die Gemische: Methan95-Ethan05, Methan93-Ethan05-CO202, Methan91-Ethan05-CO204, Methan91-Ethan05-CO202-Propan02, Methan90-Ethan10 und Erdgas-L (die 2-stelligen Ziffern bezeichnen den Anteil der Gaskomponenten in Volumen Prozent). Deutliche Unterschiede zueinander zeigen Methan, Ethan und Propan, jedoch differieren auch die die Methangemische mit Anteilen von 2 vol% bis 10 vol% anderer Gase zueinander. Dabei ist das Sensorsignal in Fig. 20 gemäß einem Ausführungsbeispiel für verschiedene Brenngase bei einem Druck von p=1001 mbar, einer Temperatur $T_{amp}$=26°C und einer Heizleistung von P=(13±12,5)mW mit einer Frequenz von f=120 Hz in Fig. 20 dargestellt.

1.3.1.5 Erkenntnisse aus den Messungen in Gasgemischen

Das Sensorsignal zeigt starke Druck- und Temperatur-Abhängigkeiten. Zur korrekten Bestimmung der Gaseigenschaften eines bekannten Gemisches mit Rückführung auf Standardbedingungen und dem Vergleich aus Tabellenwerken sollten deshalb z. B. die Quereffekte bekannt sein und korrigiert werden. Der Druck-Einfluss zeigt einen linearen Zusammenhang, der Temperatur-Einfluss einen quadratischen. Beide Querempfindlichkeiten liegen in der Größenordnung des Signals für die Gaskonzentration selbst.

**1.3.2 Verfahren zur Kalibration auf ein Gasgemisch mit Drift-Korrektur gegenüber Gas-Druck und Gas-Temperatur für z. B. einen Gassensor**

1.3.2.1 Summensignal aus Phase und Amplitude (Beispiel)

Als besonders stabiles Sensor-Signal hat sich eine Kombination aus Phasen- und Amplituden-Messung erwiesen (Kombinationssignal). Beide Signale werden beispielsweise mithilfe separater Konstanten gewichtet und beispielsweise miteinander addiert und somit zu einem einzigen Sensor-Signal zusammengeführt:

$$sigX = sigUss * Ka + sigPhi * Kp \qquad (6)$$

wobei *sigX* das berechnete Summensignal, *sigUss* das relative Amplituden-Signal und *sigPhi* das addierte Phasensignal beider Detektoren darstellt. Die Faktoren *Ka* und *Kp* sind Konstanten, mit denen beide Teilsignale multipliziert werden. Bei Umrechnung des Amplituden-Signals in mV beträgt beispielsweise *Ka* = 1/3500 und bei Umrechnun des Phasensignals in Grad beträgt beispielsweise *Kp* = 1/276 für $CO_2$-Luftgemische bis 30 vol% $CO_2$.

Das addierte Phasensignal *sigPhi* wird beispielsweise aus der Summe der beiden Phasendifferenzen für die Laufzeiten zwischen der steigenden Flanke des Heizer-Impulses und den steigenden Flanken an den Detektoren gebildet. Es gilt beispielsweise:

$$sigPhi = (D1 - Hz).phi + (D2 - Hz).phi \qquad (7)$$

wobei *(D1-Hz).phi* und *(D2-Hz).phi* die Phasendifferenzen zwischen Heizer und den Detektoren darstellen sollen.

Wie in Fig. 14 zu erkennen, steigt die Phasendifferenz zwischen Heizer und Detektoren mit zunehmender $CO_2$-Konzentration, d. h. mit zunehmender Temperaturleitfähigkeit an, jedoch fallen die beiden Amplituden an den Detektoren mit zunehmender Temperaturleitfähigkeit (Fig. 15).

Beispielsweise durch Differenzbildung aus Heizer-Amplitude und der Summe der Detektor-Amplituden wird das relative Amplituden-Signal mit Erhöhung des CO2-Gehalts im Gasgemisch steigend:

$$sigUss = 2 * Hz.Uss - (D1.Uss + D2.Uss) \qquad (8)$$

Das aus Phase und Amplitude berechnete Signal *sigX* bewegt sich beispielsweise im Bereich zwischen (1,7..2,0) für (0..6) vol% $CO_2$. Das Device (z. B. der Gassensor) wurde in einem Temperatur-Bereich zwischen (16..28)°C und im barometrischen Druckfeld zwischen (900..1200) mbar vermessen.

1.3.2.2 Drift-Korrektur über Polynom-Kompensation (Details optional)

Bei der Kalibration des Sensors auf ein bekanntes Gasgemisch sollte die starke Druck- und Temperaturabhängigkeit des Sensor-Signals kompensiert werden, um vom Messwert auf eine Gas-Konzentration schließen zu können.

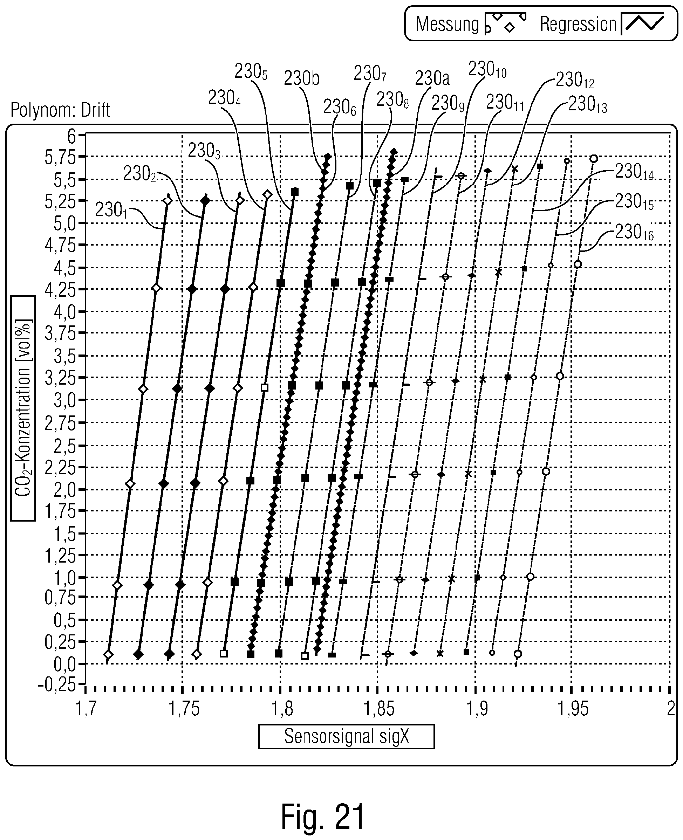

Es ergibt sich daraus beispielsweise ein 4-dimensionales Vektorfeld (Matrix), das sich aus Gas-Konzentration (CO2 [vol%]), dem Sensorsignal sigX (Summensignal aus Phase und Amplitude), der Druckdrift und der Temperatur-Drift zusammensetzt. Auffällig ist, dass die einzelnen Graphen im Diagramm aus Fig. 21 der Abhängigkeit zwischen der Gas-Konzentration und dem Sensor-Signal, die je für einen konstanten Umgebungsdruck oder eine konstante Temperatur stehen, zueinander parallel verschoben sind. Bildet man nun einen mittleren Graphen aus allen zueinander parallel verschobenen Kennlinien, erhält man einen normierten Zusammenhang des Signals für eine mittlere Temperatur und einen mittleren Druck (siehe rote Linie 230a in Fig. 21).

Fig. 21 zeigt die Matrix der Messdaten einer Variation aus Gas-Konzentration von (0..5) vol% $CO_2$ in Stickstoff im Druckbereich (900..1200) mbar und in einem Temperatur-bereich von (16..28)°C. Mithilfe einer druckabhängigen Polynomfunktion lässt sich die grüne Linie 230b der Kalibrierkurve hin zu einem aktuellen Arbeitsdruck verschieben. Die rote Linie 230a entspricht dem Mittel aller blauen Linien $230_1$ bis $230_{16}$ und ist eine auf eine mittlere Temperatur und einen mittleren Druck normierte Kennlinie des Sensorsignals zur Gas-Konzentration.

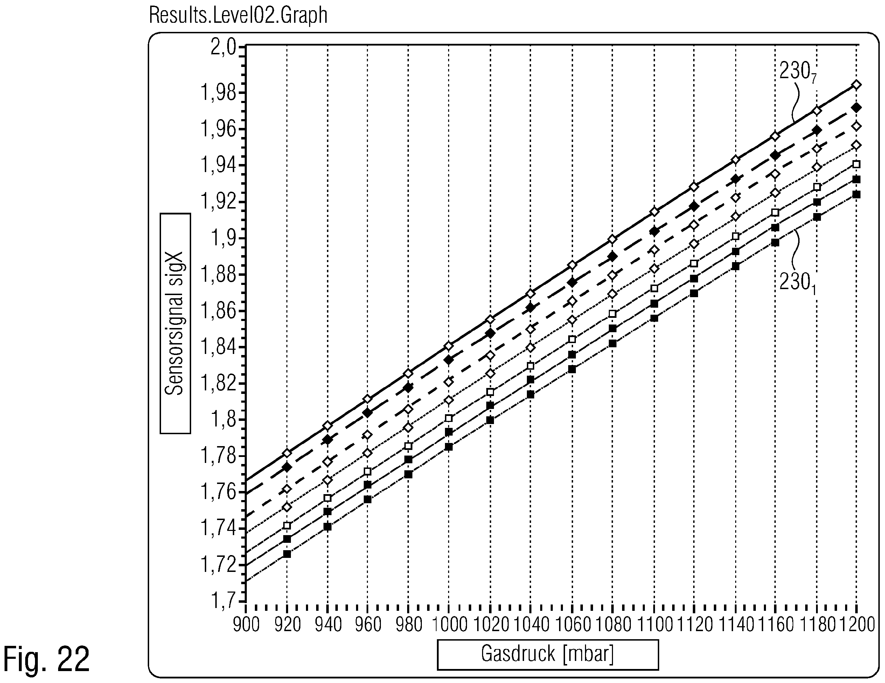

Werden die Kennlinien des Sensorsignals *sigX* aus der gemessenen Variation für jede Temperatur und eine mittlere Gaskonzentration über dem Druck aufgetragen (siehe Fig. 22), so erhält man ebenfalls eine zueinander parallel verschobene Kurvenschar aus Geraden. Höhere Drücke und kaltes Gas, d. h. dichter zueinander liegende Gasmoleküle führen zu einem höheren Sensorsignal, niedrige Drücke und warmes Gas ergibt ein niedriges Signal *sigX*.

Fig. 22 zeigt somit eine Druckabhängigkeit des Sensorsignals sigX für eine mittlere feste Gas-Konzentration, Kurvenschar aus verschiedenen Temperaturen. Die unterste Linie $230_1$ beschreibt den Zusammenhang bei der höchsten Temperatur von 28°C in der Variation und die oberste Linie $230_7$ stellt die Druckabhängigkeit des Signals bei 16°C dar.

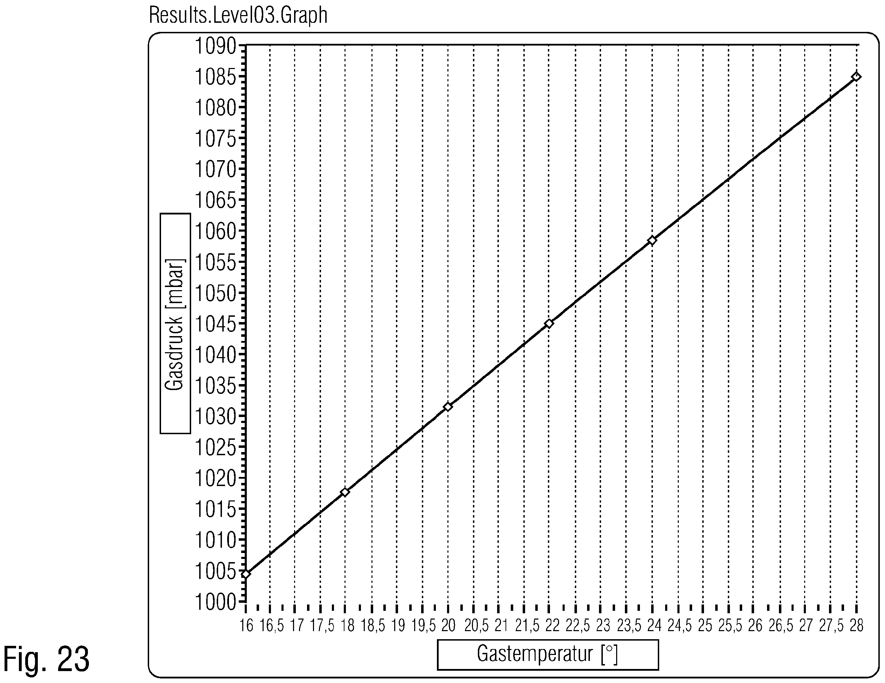

Legt man in die parallele Geradenschar in Fig. 22 für ein festes mittleres Sensorsignal sigX eine waagerechte Gerade, die alle Linien der Kurvenschar schneidet, so erhält man den Zusammenhang in Fig. 23 zwischen Gasdruck und Gastemperatur.

Fig. 23 zeigt einen leicht quadratischen Zusammenhang zwischen Gasdruck und Gastemperatur (für eine mittlere Gas-Konzentration und ein mittleres Sensorsignal sigX).

1.3.2.3 Ermittlung einer Regressionskonstanten (Details optional)

Bei der Kalibration des Gassensors auf ein bestimmtes Gasgemisch werden aus der Variationsmatrix nacheinander Regressionen über die oben beschriebenen Zusammenhänge gebildet. Regressions-Ebene **A** beschreibt den Zusammenhang zwischen der Gaskonzentration der Kalibrier-Referenz und dem Sensorsignal *sigX*. Die einzelnen Kurven pro Druck- und Temperatur werden jeweils in einer quadratischen Regression nach der Form: $y = A.c0 + A.c1*sigX + A.c2*sigX^2$ angenähert. Da der Anstieg aller Kurven nahezu konstant ist und der quadratische Koeffizient c2 gegen Null geht, wird aus allen Werten für die Koeffizienten A.c0, A.c1 und A.c2 jeweils der Mittelwert gebildet, man erhält die in Fig. 21 rot dargestellt mittlere Kennlinie 230a über die gesamte Messwert-Variation $230_1$ bis $230_{16}$. Diese muss entsprechend dem Drift-Einfluss des Drucks auf der x-Achse verschoben werden. Gesucht wird aufgrund des druckabhängigen $sigX_0 = f(p)$ der zugehörige Offset A.c0, der in die Gleichung der Regressions-Ebene **A** eingesetzt wird.

Die Regressions-Ebene **B** beschreibt die Druck-Drift des Sensorsignals *sigX*. Der Offset A.c0 wird in Abhängigkeit der Druck-Drift neu berechnet: $A.c0 = sigX.y_0 - B.c1*Druck.x_0 - B.c2*Druck.x_0^2$. Kann $sigX.y_0 = 0$ gesetzt werden, vereinfacht sich die Gleichung zu: $A.c0 = -(B.c1*Druck.x_0 + B.c2*Druck.x_0^2)$. Der (nun) druckabhängige Polynom-Koeffizient A.c0=f(p) wird z. B. in der Regressionsgleichung der Ebene A ersetzt (substituiert).

Der ermittelte druckabhängige Offset für das Polynom der Regressions-Ebene A errechnet sich z. B. aus dem Cosinus Zusammenhang der Winkel-Beziehung zwischen Offset und Anstieg mit: $A.c1 = A.c0/sigX_0$; $sigXo = f(p)$ und $A.c0 = (-1)* sigX * A.c1$. Bei Polynomen mit höherer Ordnung sollte die 1. Ableitung der Kurve gebildet und daraus der Anstieg im Referenz-Punkt berechnet werden.

| Regressions-Ebene | | Polynom-Koeffizienten | | | Bestimmtheitsmaß | Referenz zur vorhergehenden Ebene (Mitte des Variationsbereiches) |
|---|---|---|---|---|---|---|
| | | c0 | c1 | c2 | | |
| A | Signal zu CO2 | -266,153759 | 144,315423 | 0 | 0,999258 | 0 |
| B | Druck zu Signal-Verschiebung | 0,94394 | 0,001017 | -1,50E-07 | 0,999929 | 1,843335 |
| C | Temperatur zur Druck-Verschiebung | 884,519093 | 7,844777 | -0,023415 | 0,9983 | 1050 |
| Tabelle 1: Polynom-Koeffizienten der drei Regressions-Ebenen (Beispiele) | | | | | | |

1.3.2.4 Umrechnung des Signals auf einen CO2-Wert (Beispiel; Details optional)

Der berechnete Wert für die Gas-Konzentration aus dem Polynom der Regressions-Ebene A wird beispielsweise um die Druck- und Temperatur-Drift korrigiert:

$$CO_2[vol\%] = A.y(sigX) \cdot \left(1 - \left[\frac{B.y(p) - B.ref}{sigX - B.ref}\right] \cdot \left(1 - \left[\frac{C.y(T) - C.ref}{p - C.ref}\right]\right)\right) \tag{9}$$

wobei $A.y(sigX)$, $B.y(p)$ und $C.y(T)$ den jeweils vollständigen Polynomen für das Mess-Signal, dem Gasdruck und der Gastemperatur entsprechen.

Werden die festen Referenzen, die die geometrische Mitte des Variationsbereiches darstellen, in die Formel eingesetzt und die Polynome entsprechend aufgelöst, so ergeben sich folgende Gleichungen. Mit $B.ref = B.y( c.ref)$ folgt:

$$CO_2[vol\%] = A.y(sigX) \cdot \left(1 - \left[\frac{B.c1 \cdot (p - C.ref) + B.c2 \cdot (p^2 - C.ref^2)}{sigX - B.y(C.ref)}\right] \cdot \left(1 - \left[\frac{C.y(T) - C.ref}{p - C.ref}\right]\right)\right) \tag{10}$$

Für $C.ref = 1050\ mbar$ eingesetzt ergibt sich:

$$CO_2[vol\%] = A.y(sigX) \cdot \left(1 - \left[\frac{B.c1 \cdot (p - 1050) + B.c2 \cdot (p^2 - 1050^2)}{sigX - B.y(1050)}\right] \cdot \left(1 - \left[\frac{C.y(T) - 1050}{p - 1050}\right]\right)\right) \tag{11}$$

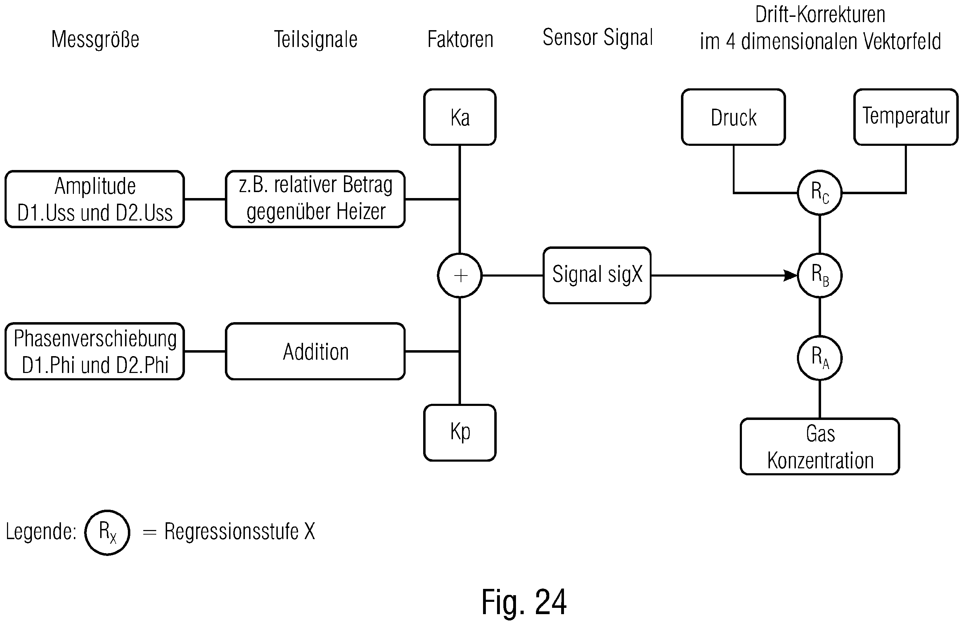

Fig. 24 zeigt ein Blockdiagramm eines schematischen Vorgehens zur Ermittlung einer Gaskonzentration unter Berücksichtigung der Einflüsse von Druck und Temperatur aus dem gebildeten Sensorsignal *sigX*. In anderen Worten zeigt Fig. 24 eine schematische Darstellung zur Bildung des Sensorsignals *sigX* aus Amplituden und Phasen sowie die Ermittlung einer Gaskonzentration aus sigX unter Berücksichtigung von Druck- und Temperatur-Einfluss (Beispiel).

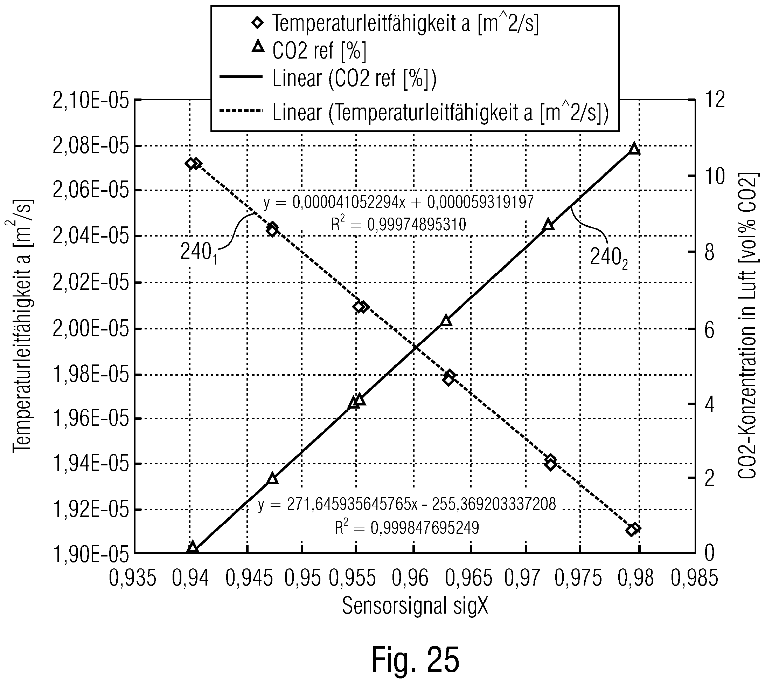

Neben der Kalibration des Sensorsignals auf die Konzentration eines bekannten Gasgemisches, ist es außerdem möglich, die Temperaturleitfähigkeit *a* des Gasgemisches direkt zu bestimmen. In Fig. 25 wurde die theoretisch berechnete Temperaturleitfähigkeit gegenüber dem Sensorsignal sigX aufgetragen. In anderen Worten zeigt Fig. 25 die Temperaturleitfähigkeit gegenüber dem Sensorsignal sigX bei konstantem Druck und konstanter Temperatur in einem Gemisch aus Kohlendioxid $CO_2$ in Stickstoff $N_2$. Die Temperaturleitfähigkeit $240_1$ (rote Linie) fällt mit Erhöhung der $CO_2$-Konzentration $240_2$ (grüne Linie).

Somit wird hierin ein Design uns eine Auswertung eines thermischer Gassensor zur Messung physikalischer Gaseigenschaften beschrieben. Mit dieser Erfindung wird folgendes vorgeschlagen (Aspekte sind unabhängig voneinander und in Kombination einsetzbar):

- Sensor-Design auf Basis zweier Technologie-Varianten: MEMS Draht-Sensor auf SOI-Substrat und Thermopile-Sensor auf Dünnschicht-Membran
- Betrieb des Gassensors: Signal-Generierung und -Auswertung auf einem embedded System
- Auswertealgorithmus zur Kalibration auf ein Gasgemisch mit Drift-Korrektur gegenüber Gas-Druck und -Temperatur

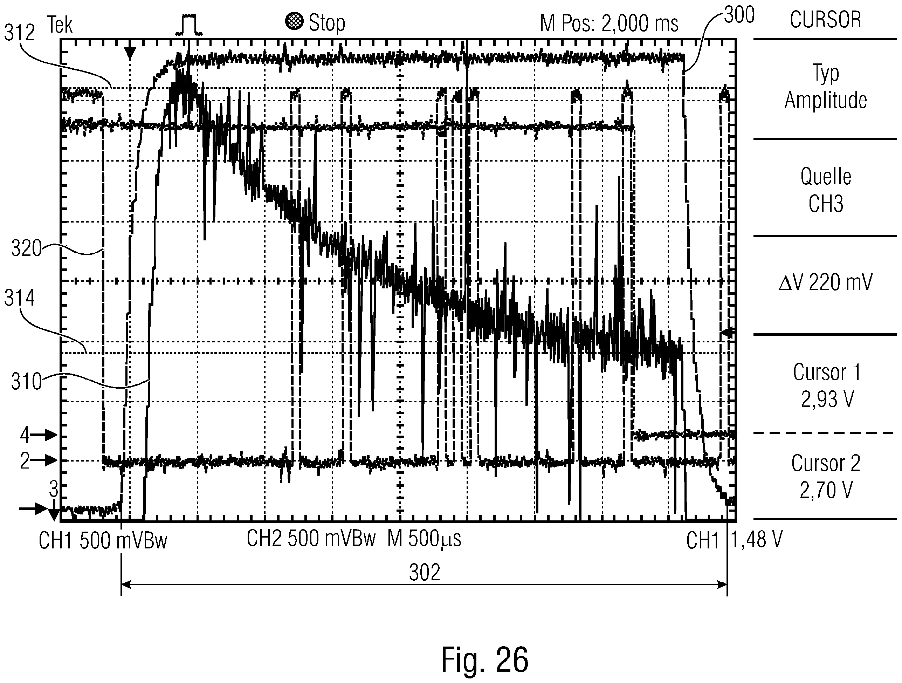

Fig. 26, Fig. 27 und Fig. 28 zeigen ein Diagramm, in dem eine an einen Heizer eines Gassensors angelegte Heizspannung 300, ein Stromfluss 310 während einer Heizperiode 302, Messzeiten 320 für einen optionalen ADC (Analog-Digital-Umsetzer) einer beispielhaften Auswerteanordnung und ein Komparator-Signal für einen Detektor (z. B. für einen ersten Detektor oder für einen zweiten Detektor) dargestellt ist. Die Auswerteanordnung ist z. B. ausgelegt, um die Information, wieviel Wärme von dem Heizer während der Heizperiode 302 abgeführt wird, basierend auf einer Messung des Stromflusses 310 durch den Heizer bei vorgegebener Heizspannung 300 zu erhalten. Gemäß einem Beispiel ist die Auswerteanordnung ausgelegt, um den Stromfluss 310 kurz nach einem Einschalten der vorgegebenen Heizspannung 300 und kurz vor einem Ausschalten der vorgegebenen Heizspannung zu erhalten. Dies bedeutet z. B., dass ein Start-Peak 312 des Heizstromes 310 und ein Heizstrom-Endwert 314 des Stromflusses 310 von der Auswerteanordnung verarbeitet werden können.

Gemäß einem Beispiel weist der Start-Peak 312 für ein und denselben Gassensor immer den gleichen Wert auf (evtl. können geringe Schwankungen von weniger als 1%, 0.5% oder 0.1% auftreten). Gemäß den in den Figuren 26 bis 28 dargestellten Beispielen befindet sich der Start-Peak 312 immer bei 2,93V und ca.400$\mu$s nach dem Einschalten. Dieser Wert des Start-Peaks 312 ist für einen Gassensor in den Figuren 26 bis 28 dargestellt. Für andere Gassensoren kann der Wert des Start-Peaks 312 von dem in den Figuren 26 bis 28 dargestellten Wert abweichen. Hierbei ist anzumerken, dass der Wert des Start-Peaks 312 nicht nur zwischen unterschiedlichen Gassensoren abweichen kann, sondern auch zwischen baugleichen Gassensoren.

Der Heizer erwärmt sich während der Heizperiode 302, wodurch ein Heizer-Widerstand entsprechend einem positiven TKR (Temperaturkoeffizienten) steigt und der Heizer eine Endtemperatur im Abschalt-Moment erreicht ($I_{hz}=U/R_{Hz} \rightarrow I_{hz} \approx 1/R_{Hz}$). Je kleiner der Heiz-Strom-Endwert 314, desto heißer der Heizer, desto weniger Wärme wurde an ein den Heizer umgebendes zu analysierendes Gas oder Gasgemisch abgegeben und desto geringer ist die Wärmeleitfähigkeit des Gases oder Gasgemisches.

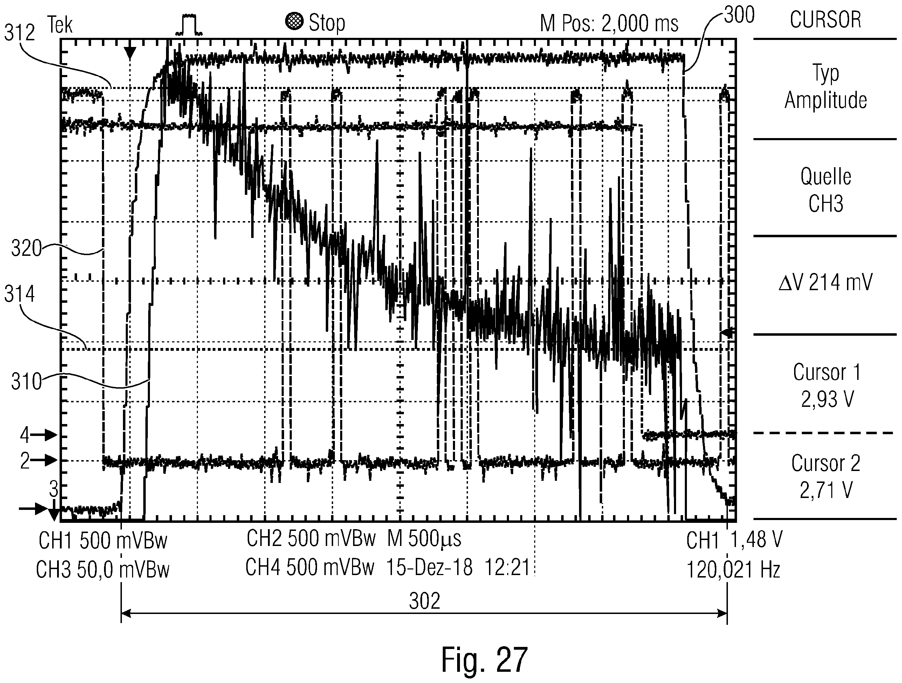

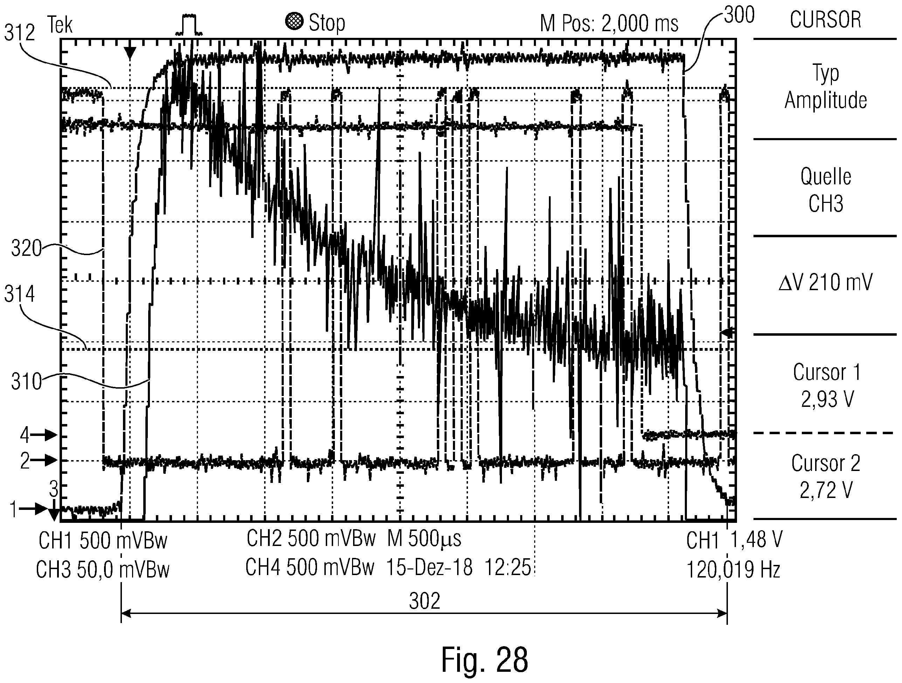

In den Beispielen gemäß den Figuren 26 bis 28 wird ein Gas oder Gasgemisch von einem Gassensor und/oder einer Auswerteanordnung analysiert. Dabei wird gemäß dem Beispiel aus Fig. 26 ein Gasgemisch mit 10% $CO_2$ und 90% $N_2$ (Stickstoff) analysiert, gemäß dem Beispiel aus Fig. 27 wird das Gas Stickstoff (100% $N_2$) analysiert und gemäß dem Beispiel aus Fig. 28 wird das Gas Sauerstoff (100% $O_2$) analysiert. Diese Komponenten weisen z. B. unterschiedliche Wärmeleitfähigkeiten auf, wie z. B. Sauerstoff mit einer Wärmeleitfähigkeit $\lambda$ von 0.0263 W/(m*K), Stickstoff mit einer Wärmeleitfähigkeit $\lambda$ von 0,0260 W/(m*K) und $CO_2$ mit einer Wärmeleitfähigkeit $\lambda$ von 0.0168 W/(m*K).

Gemäß dem Beispiel aus Fig. 26 erreicht der Heiz-Strom-Endwert 314 2.7 V, wodurch eine Differenz zwischen Einschalt- (Start-Peak 312) und Abschalt-Strom (Heiz-Strom-Endwert 314) von 220 mV (deltaU=220 mV) erreicht wird. Gemäß dem Beispiel aus Fig. 27 erreicht der Heiz-Strom-Endwert 314 2.71 V, wodurch eine Differenz zwischen Einschalt- (Start-Peak 312) und Abschalt-Strom (Heiz-Strom-Endwert 314) von 214 mV (deltaU=220 mV) erreicht wird. Gemäß dem Beispiel aus Fig. 28 erreicht der Heiz-Strom-Endwert 314 2.72 V, wodurch eine Differenz zwischen Einschalt- (Start-Peak 312) und Abschalt-Strom (Heiz-Strom-Endwert 314) von 210 mV (deltaU=220 mV) erreicht wird. In anderen Worten verarbeitet (untersucht) die Auswerteanordnung die Differenz des Heizstromes (des Stromflusses 310) zwischen Einschalt- und Abschalt-Moment, also zwischen kaltem und heißem Heizer.

Das $CO_2$-Gemisch aus Fig. 26 weist verglichen mit den Beispielen aus Fig. 27 und Fig. 28 die höchste Heizer-Temperatur auf und somit wird hier der geringste Heizstrom (z. B. der geringste Heiz-Strom-Endwert 314) erreicht, da $CO_2$ nur die Hälfte der Wärmeleitfähigkeit gegenüber $N_2$ und $O_2$ besitzt. Im Vergleich hierzu erreicht der Heizer gemäß Fig. 27 in 100 vol.% $N_2$ nicht die Endtemperatur (z. B. den Heiz-Strom-Endwert 314) wie in dem 10%$CO_2$-90%$N_2$ Gemisch aus Fig. 26. Ebenso erreicht der Heizer gemäß Fig. 28 in 100 vol.% $O_2$ nicht die Endtemperatur (z. B. den Heiz-Strom-Endwert 314) wie in dem 10%$CO_2$-90%$N_2$ Gemisch aus Fig. 26. Die Differenz zwischen Einschalt- und Abschalt-Strom in Fig. 28 ist mit 210 mV am geringsten und dementsprechend weist das hier analysierte Gas auch die höchste Wärmeleitfähigkeit im Vergleich zu dem 10%$CO_2$-90%$N_2$ Gemisch (siehe Fig. 26) und dem 100 vol.% $N_2$ (siehe Fig. 27) auf.

Gemäß einem Beispiel ist ein Messeffekt minimal, da der Heizer z. B. hoch dotiert ist, um mit z. B. 3 V heizen zu können. Ferner kann der TKR (Temperaturkoeffizient) des Heizers niedrig sein (z. B. $\leq 6*10^{-3}$ 1/K, $\leq 4.1*10^{-3}$ 1/K, $\leq 3.9*10^{-3}$ 1/K).

Somit wird mit dem Gassensor und/oder der Auswerteanordnung z. B. ein Sensorsignal (z. B. der Stromfluss 310) genutzt, um die Konzentration eines dritten Gases unterscheiden zu können. Bei einer Notfallbeatmung wird mit erhöhten Sauerstoff-Konzentrationen gearbeitet. So ist es üblich, ein Gemisch aus 50 vol.% $O_2$ in $N_2$ zu verwenden. Ohne Korrektur des Sensor-Signals bei höherer Sauerstoff-Konzentration gegenüber dem Frischgas bei einer Kalibrierung zeigt der Gassensor (z. B. ein $CO_2$-Sensor) z.B. bei 50 vol.% $O_2$ im $N_2$-$O_2$ Gasgemisch eine Konzentration von -5% $CO_2$ an. D.h. die Änderung der dritten Gaskomponente, eine Konzentrations-Änderung von Sauerstoff, im Gemisch führt unter Umständen zu einem Fehlersignal in dem $CO_2$-Sensor. Ein Konzentrationsgemisch von etwa 56% Sauerstoff und 5% $CO_2$ in Stickstoff $N_2$ zeigt am Sensor ebenso z. B. eine $CO_2$-Konzentration von 0 vol.% an, wie bei der Frischgas-Kalibrierung mit 21 vol.% $O_2$ und ca. 78 vol.% $N_2$.

Besteht technisch keine Möglichkeit, die Sauerstoff-Konzentration des Inhalationsgases dem Sensor mitzuteilen, so dass eine Konzentrationsanzeige des Gassensors entsprechend korrigiert werden kann, so würde der Sensor z. B. fehlerhafte Werte anzeigen.

Wie in den Figuren 26 bis 28 zuerkennen, steckt eine Information der Wärmeleitfähigkeit in der Endtemperatur, die der Heizer bis zu seinem Abschalten erreicht. Vergleicht man nun die Temperaturdifferenz (deltaU) zwischen dem Strom-Peak 312 zu Beginn der Aufheizung und dem Endwert 314 kurz vorm Abschalten, so könnte man bei unterschiedlichen deltaU auf unterschiedliche Wärmeleitfähigkeiten im Gasgemisch schließen.

Gemäß einem Beispiel wird der Heizer mit einer konstanten Spannung betrieben (die gemäß den Figuren 26 bis 28 einem Wert von 3000 digits an einem D/A Ausgang entspricht, aber hier nur ein Beispiel darstellt und frei gewählt werden kann). Mit der Strom-Messung über z. B. einen Shunt-Widerstand und einem CURRENT SHUNT MONITOR, z. B. dem INA199A, Baustein U402, wie z. B. in Fig.10, wird ein Spannungssignal erhalten, das sich proportional zum Heizstrom verhält. In den Figuren 26 bis 28 sind Screen-Shots der am Oszilloskop aufgezeichneten Signale dargestellt, wobei der Heizerstrom-Verlauf (der Stromfluss 310) stark vergrößert ist (Das Rauschen kann durch Abschirmung von Messleitungen reduziert werden). Ein Produkt aus Heizspannung 300 und gemessenem Heizstrom 310 ermöglicht z. B. eine Aussage über die Heizleistung. Der Heizstrom 310 sinkt mit der Zeit etwas, da sich der Heizer erwärmt und wegen seines Temperaturgradienten des Widerstandes (TKR) seinen Innenwiderstand erhöht. Aufgrund des höheren Innenwiderstandes kann bei gleicher Heizspannung nun weniger Strom fließen ($I_{Hz} \approx 1/R_{Hz}$).

Wieviel Energie der Heizer in das ihn umgebende Gas abgeben kann, ist unter anderem auch abhängig von der Wärmeleitfähigkeit des Gases. Bei 10% $CO_2$ in $N_2$ zum Beispiel kann der Heizer nicht so viel Wärme abgeben, weil $CO_2$ eine Wärmeleitfähigkeit von λ=0,0168 W/(m*K) besitzt, der Heizer erreicht demnach einer höhere Endtemperatur vorm Abschalten gegenüber einem 100% $N_2$ Gas. Bei der Messung wurde eine Differenz von deltaU = 220mV ermittelt. Stickstoff $N_2$ besitzt eine Wärmeleitfähigkeit von λ=0,0260 W/(m*K), der Heizer könnte gegenüber einem 100% $CO_2$ Gas fast doppelt so viel Wärme abgeben. Bei der Messung wurde ein deltaU von 214mV ermittelt. Der Unterschied der Wärmeleitfähigkeiten zwischen Stickstoff und Sauerstoff $O_2$ ist nicht sehr groß. Dennoch kann mit dem hierin beschriebenen Gassensor und/oder der Auswerteanordnung ein deltaU von 210mV ermittelt werden, das etwas kleiner ist als bei der Messung in $N_2$.

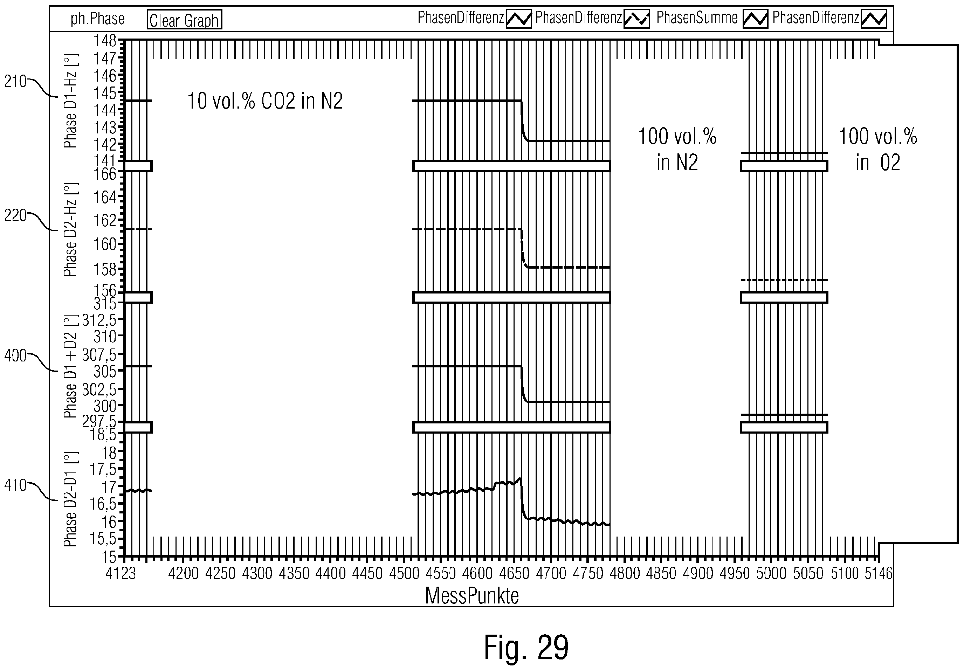

In Fig. 29 werden vier unterschiedliche Phasen-Signale dargestellt, die die Phaseninformation sigPhi für die Auswerteanordnung gemäß einem Ausführungsbeispiel darstellen können. So wird z. B. die Information 210 über die erste Phasendifferenz, die Information 220 über die zweite Phasendifferenz, eine Phasen-Summe 400 (Information 210 + Information 220) und eine Phasen-Differenz 410 (Information 220 - Information 210) dargestellt. Gemäß einem Ausführungsbeispiel liegen bei der Messung der Phaseninformationen 210, 220, 400 und 410 eine Temperatur von T=24°C und ein Druck von p=1013mbar vor. Dies ist aber nur ein Beispiel und die Phaseninformationen 210, 220, 400 und 410 können ebenso bei anderen Umgebungsparametereinstellungen (andere Temperatur, anderer Druck) erfasst werden.

Gemäß dem in Fig. 29 dargestellten Ausführungsbeispiel stellt ein erster Abschnitt (z. B. Messpunkte 4520 bis 4620) die jeweilige Phaseninformation 210, 220, 400 und 410 für ein 10 vol.% $CO_2$ in $N_2$ - Gasgemisch dar, ein zweiter Abschnitt (z. B. Messpunkte 4670 bis 4780) die jeweilige Phaseninformation 210, 220, 400 und 410 für ein 100 vol.% $N_2$ Gas und ein dritter Abschnitt (z. B. Messpunkte 4960 bis 5070) die jeweilige Phaseninformation 210, 220, 400 und 410 für ein 100 vol.% $O_2$ Gas. Zeiten zwischen den einzelnen Abschnitten sind z. B. Wartezeiten bis zu einer Einstellung des jeweiligen Gemisches.

Die Phasen-Summe 400 stellt z. B. ein stabiles Summen-Signal D1+D2 (D1 entspricht Detektor 1 und D2 entspricht Detektor 2) der Phasen-Lagen dar. Eine Unterscheidbarkeit zwischen 100 vol.% $O_2$ und 100 vol.% $N_2$ ist für z. B. alle Phaseninformationen vorhanden. Der größte Signal-Unterschied tritt gemäß dem Ausführungsbeispiel zwischen 10 vol.% $CO_2$ und 0 vol.% $CO_2$ in $N_2$ auf. Da das Phasen-Differenz-Signal 410, derzeit stark verrauscht ist wird als Phaseninformation die Phasen-Summe 400 bevorzugt.

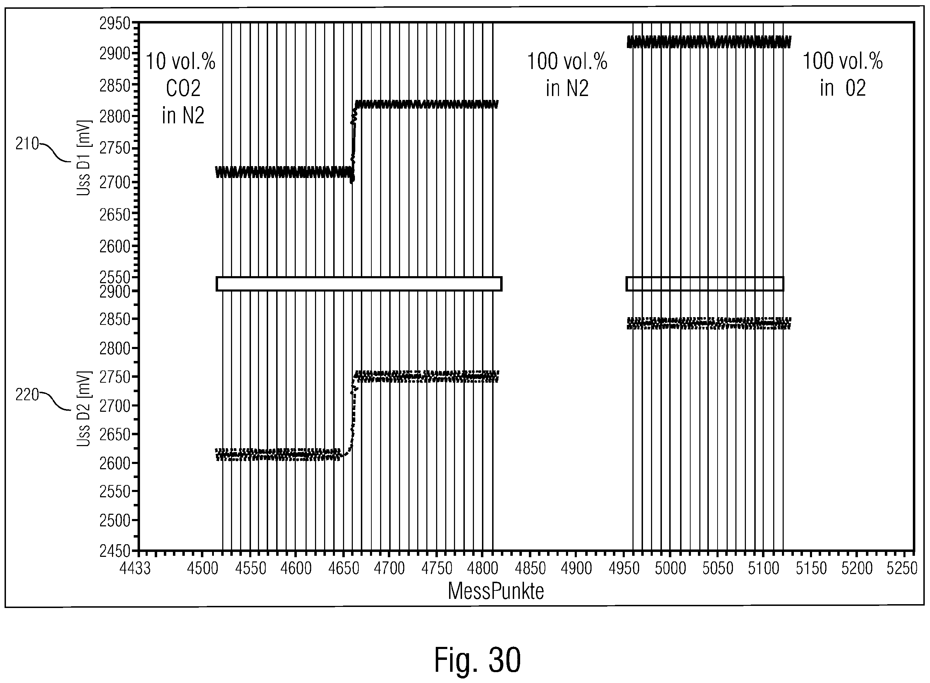

In Fig. 30 werden eine Information 210 über eine Amplitude eines Detektorsignals eines ersten Detektors und eine Information 220 über eine Amplitude eines Detektorsignals eines zweiten Detektors dargestellt. Gemäß einem Ausführungsbeispiel liegen bei der Messung der Amplitudeninformationen 210 und 220 eine Temperatur von T=24°C und ein Druck von p=1013mbar vor. Dies ist aber nur ein Beispiel und die Amplitudeninformationen 210 und 220 können ebenso bei anderen Umgebungsparametereinstellungen (andere Temperatur, anderer Druck) erfasst werden.

Gemäß dem in Fig. 30 dargestellten Ausführungsbeispiel stellt ein erster Abschnitt (z. B. Messpunkte 4520 bis 4650) die jeweilige Amplitudeninformation 210, 220 für ein 10 vol.% $CO_2$ in $N_2$ - Gasgemisch dar, ein zweiter Abschnitt (z. B. Messpunkte 4670 bis 4810) die jeweilige Amplitudeninformation 210, 220 für ein 100 vol.% $N_2$ Gas und ein dritter Abschnitt (z. B. Messpunkte 4960 bis 5120) die jeweilige Amplitudeninformation 210, 220 für ein 100 vol.% $O_2$ Gas. Zeiten zwischen den einzelnen Abschnitten sind z. B. Wartezeiten bis zu einer Einstellung des jeweiligen Gemisches.

Die Information 210 über die Amplitude des Detektorsignals des ersten Detektors und die Information 220 über die Amplitude des Detektorsignals des zweiten Detektors stellen z. B. stabile Amplituden-Signale dar, wobei D2 (der zweite Detektor) gemäß diesem Ausführungsbeispiel ein größeres Rauschen aufweist, da dessen Amplitude geringer ist als die Amplitude an D1 (der erste Detektor) (ca. 5mV zu 1,8mV in Luft). Bei beiden Amplitudeninformationen 210, 220 tritt eine klarere Unterscheidbarkeit zwischen 100 vol.% $O_2$ und 100 vol.% $N_2$ als bei den Phasen-Signalen (siehe 210, 220, 400 und 410 in Fig. 29) auf. Somit ist evtl. ein Vergleich der Verhältnisse zwischen Phase und Amplitude sinnvoll, um auf eine Konzentration des dritten Gases $O_2$ mittels der Auswerteanordnung zu schließen (falls das System z. B. zu weit von der Frischluft Kalibrierung gedriftet ist).

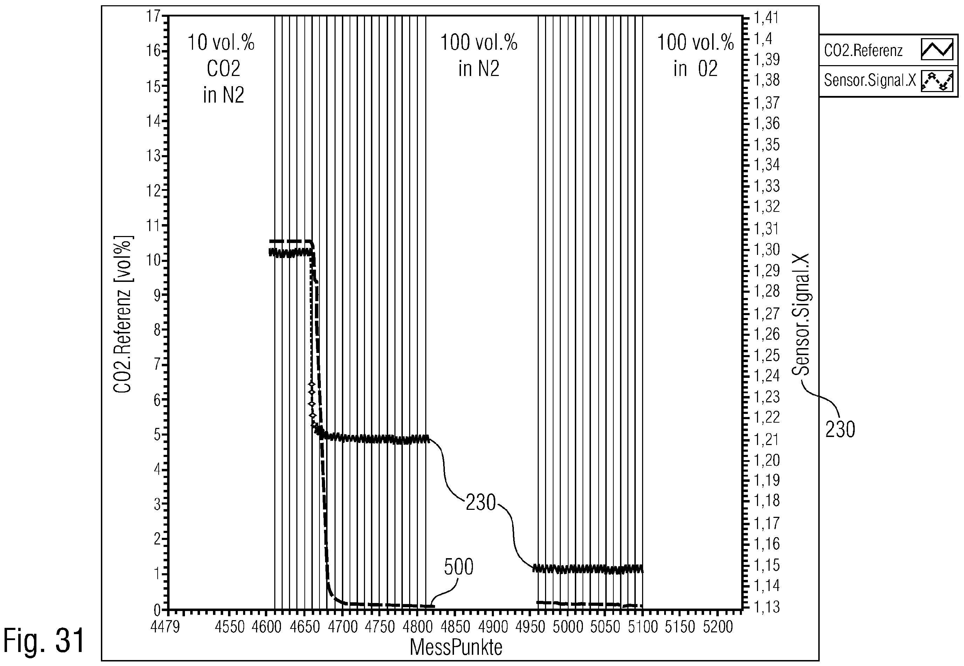

Fig. 31 zeigt ein Kombinationssignal sigX 230 (mit der rechten y-Achse) und ein $CO_2$-Referenz-Signal 500 (mit der linken y-Achse). Gemäß einem Ausführungsbeispiel liegen bei der Messung des Kombinationssignals sigX 230 eine Temperatur von T=24°C und ein Druck von p=1013mbar vor. Dies ist aber nur ein Beispiel und das Kombinationssignal sigX 230 kann ebenso bei anderen Umgebungsparametereinstellungen (andere Temperatur, anderer Druck) erfasst werden.

Gemäß dem in Fig. 31 dargestellten Ausführungsbeispiel stellt ein erster Abschnitt (z. B. Messpunkte 4610 bis 4650) das Kombinationssignal sigX 230 für ein 10 vol.% $CO_2$ in $N_2$-Gasgemisch dar, ein zweiter Abschnitt (z. B. Messpunkte 4700 bis 4810) das Kombinationssignal sigX 230 für ein 100 vol.% $N_2$ Gas und ein dritter Abschnitt (z. B. Messpunkte 4960 bis 5100) das Kombinationssignal sigX 230 für ein 100 vol.% $O_2$ Gas. Zeiten zwischen den einzelnen Abschnitten sind z. B. Wartezeiten bis zu einer Einstellung des jeweiligen Gemisches. Ein NDIR Referenz-Detektor (siehe Referenz-Signal 500) detektiert z. B. nur eine $CO_2$-Konzentration und kann zwischen $N_2$ und $O_2$ nicht unterscheiden.

Alle 3 Gasgemische sind mit dem Kombinationssignal 230 deutlich voneinander unterscheidbar. Somit wird vorgeschlagen Verhältnisse zwischen Phase und Amplitude zu vergleichen, um auf eine Konzentration des dritten Gases $O_2$ zu schließen.

Während sich der Unterschied zwischen 100% $N_2$ und 100% $O_2$ nur gering auf das Phasen-Signal auswirkt (Fig. 29), ist der Unterschied der Amplituden-Signale zwischen einem 10%CO2-90%N2 und einem 100%N2 Gemisch nahezu genauso groß wie der Unterschied zwischen 100%N2 und 100%O2 (Fig. 30). Das Kombinationssignal sigX (Fig. 31) verwischt dieses Verhalten wieder etwas.

Im Folgenden werden weitere Beispiele dargestellt, die Merkmale und Funktionalitäten des Gassensors in anderen Worten beschreiben. Diese Beispiele können mit den oben beschriebenen Ausführungsbeispielen kombiniert werden oder Alternativen darstellen.

Gemäß einem Beispiel handelt es sich bei dem Gassensor um einen Membran-Sensor. Der thermische Gassensor auf Basis von Membran- und Thermopile-Technologie mit perforierter Membran, kann ausgelegt sein um den parasitären Wärmetransport über die Membran oder die Aufhängungen der Strukturen zu minimieren, um ein höheres gassensitives Signal zu erhalten.

Gemäß einem Ausführungsbeispiel kann der Gassensor Elektronik aufweisen, wobei die Elektronik einen oder mehrere der folgenden Aspekte, einzeln oder in Kombination aufweisen kann. Die Elektronik kann einen DC-Sensorverstärker mit über Software nachgeführtem Arbeitspunkt aufweisen. Ferner kann die Elektronik ausgelegt sein, um eine Messung der Phasenlage über die interne Timer-Struktur des μControllers (MSP430) durchzuführen, wobei dazu z. B. die genaue Erzeugung des Heizer-Anregungssignals über den Analog-Schalter über die interne Timer-Struktur des μControllers (MSP430) verwendet wird. Des Weiteren kann die Elektronik ausgelegt sein, um eine Messung der Phasenlage der Sensorsignale über einen Schmitt-Trigger, der die vom DC-Offset befreiten Sensorsignale im Nullpunktdurchgang misst durchzuführen, da dort die Signale am steilsten sind und somit das Phasenrauschen minimiert ist. Optional umfasst die Elektronik eine Regelung der Heizleistung über S1-Amplitudenregler und/oder eine Regelung von Timing der Abtastung.

Gemäß einem Beispiel kann der Gassensor eine Kalibration aufweisen. Die Kalibration kann hierbei ausgebildet sein, um ein Pseudo-Signal aus Phase und Amplitude zu bilden, wobei bei Signalbildung und Formel der Schwerpunkt auf einem Pseudo-Signal liegen kann.

Es sei darauf hingewiesen, dass die Ausführungsbeispiele gemäß den Ansprüchen mit allen Merkmalen, Funktionalitäten und Details ergänzt werden können, die hierin beschrieben sind (soweit dies nicht zu irgendwelchen Widersprüchen führt).

Merkmale, Funktionalitäten und Details der Ansprüche können auch mit den hierin beschriebenen Ausführungsbeispielen kombiniert werden, um zusätzliche Ausführungsbeispiele zu erhalten.

Es sei darauf hingewiesen, dass Merkmale und Funktionalitäten, die in einzelnen oder einigen der Ausführungsbeispielen gezeigt sind, auch in anderen der Ausführungsbeispiele eingesetzt werden können, soweit dem keine gravierenden technischen Gründe entgegenstehen.

Weiterhin sei darauf hingewiesen, dass auch Teil-Funktionalitäten der hierin beschriebenen Ausführungsbeispiele eingesetzt werden können, sofern dem keine wesentlichen technischen Gründe entgegenstehen.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist. Der Datenträger, das digitale Speichermedium oder das computerlesbare Medium sind typischerweise gegenständlich und/oder nichtvergänglich bzw. nichtvorübergehend.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die hierin beschriebenen Vorrichtungen können beispielsweise unter Verwendung eines Hardware-Apparats, oder unter Verwendung eines Computers, oder unter Verwendung einer Kombination eines Hardware-Apparats und eines Computers implementiert werden.

Die hierin beschriebenen Vorrichtungen, oder jedwede Komponenten der hierin beschriebenen Vorrichtungen können zumindest teilweise in Hardware und/oder in Software (Computerprogramm) implementiert sein.

Die hierin beschriebenen Verfahren können beispielsweise unter Verwendung eines Hardware-Apparats, oder unter Verwendung eines Computers, oder unter Verwendung einer Kombination eines Hardware-Apparats und eines Computers implementiert werden.

Die hierin beschriebenen Verfahren, oder jedwede Komponenten der hierin beschriebenen Verfahren können zumindest teilweise durch Hardware und/oder durch Software ausgeführt werden.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

**1.5 Literatur**

[Baehr 2008] H. D. Baehr and K. Stephan, Wärme- und Stoffübertragung, 6. neu bearbeitete Auflage ed Springer-Verlag, 2008.

[Simon 2002] I. Simon and M. Arndt. Thermal and gas-sensing properties of a micromachined thermal conductivity sensor for the detection of hydrogen in automotive applications. Sensors and Actuators A: Physical, 97-98, pp. 104-108, April 2002. doi: 10.1016/S0924-4247(01)00825-1.

[Baar 2001] J. J. van Baar, R. J. Wiegerink, T. S. J. Lammerink, G. J. M. Krijnen, and M. Elwenspoek. Micromachined structures for thermal measurements of fluid and flow parameters. Journal of Micromechanics and Microengineering, 11(4), pp. 311-318, July 2001. doi: 10.1088/0960-1317/11/4/304.

[IST AG 2011] German patent: reference DE 10 2011 075 519 A1; title: Verfahren und Vorrichtung zum thermischen Bestimmen des Massendurchflusses eines Mediums in einer Leitung; inventors: Hepp, Christoph; Krogmann, Florian; Lehmann, Mirko; Polak, Jiri; application date: May 9 2011

[IST AG 2013] Applied for a German patent under reference DE 10 2013 102 2309.3; title: Thermischer Strömungssensor zur Bestimmung eines Gases oder der Zusammensetzung eines Gasgemisches, sowie dessen Strömungsgeschwindigkeit; inventors: Hepp, Christoph, Krogmann, Florian; application date: March 11 2013

[IST AG 2015] Applied for a German patent under reference DE 10 2015 107 584.9, title: Verfahren zur Bestimmung eines Produktes aus Wärmekapazität und Dichte, inventors: Hepp, Christoph, Krogmann, Florian, Reyes, Diego; application date: May 13 2015

[Grien 2012] H. Grienauer - AMS GmbH, Dielheim: Gasanalyse mit thermisch modulierten Wärmeleitfähigkeits-Sensoren mit Fourier-Analyse des Messsignals; 16. GMA/ITG-Fachtagung Sensoren und Messsysteme 2012; 22.-23.05.2012; Nürnberg, Germany; Chapter 1.2 Chemische Sensoren; pp 54-61; DOI: 10.5162/sensoren2012/1.2.2; ISBN: 978-3-9813484-0-8

[2003] A. Al-Salaymeh, M. Alhusein, F. Durst, (2003) "Development of a twowire thermal flow sensor for industrial applications", Journal of Quality in Maintenance Engineering, Vol. 9 Issue: 2, pp. 113-131, https://doi.org/10.1108/13552510310482370

[2009] DE 10 2008 047 511 A1 2009.03.19; Vorrichtung und Verfahren zur Atemgasanalyse; Weinmann Geräte für Medizin GmbH + Co. KG, Florian Dietz

[2011] Kliche, Billat, Messner, Zengerle: Sensorsystem zur thermischen Gasanalyse von Gasgemischen, Konferenzbeitrag in Proc. of Mikrosystemtechnik Kongress 2011, Darmstadt, Deutschland, (Poster), 10. Oktober 2011, Seite 875-878, ISBN: 978-3-8007-3367-5 (2011)

[2011] Sensor for gas analysis based on thermal conductivity, specific heat capacity and thermal diffusivity; K Kliche, S Billat, F Hedrich, C Ziegler, R Zengerle; Micro Electro Mechanical Systems (MEMS), 2011 IEEE 24th International Conference on Micro Electro Mechanical Systems

[2012] DE 10 2011 075519 A1 2012.11.15; Verfahren und Vorrichtung zum thermischen Bestimmen des Massedurchflusses eines Mediums in einer Leitung; Innovative Sensor Technology IST AG, Christoph Hepp, Florian Krogmann, Mirko Lehmann, Jiri Polak

[2013] Sensor for thermal gas analysis based on micromachined siliconmicrowires; K Kliche, G Kattinger, S Billat, L Shen, S Messner, R Zengerle, IEEE Sensors Journal 13 (7), 2626-2635; 2013

**Patentansprüche**

1. Auswerteanordnung (200) für einen thermischen Gassensor (100) mit zumindest einem Heizer (120) und zumindest einem Detektor (130, 140),

   wobei die Auswerteanordnung (200) ausgelegt ist, um

   - eine Information (210) über eine Amplitude eines Detektorsignals eines ersten Detektors (130) und
   - eine Information (210) über eine erste Phasendifferenz zwischen einem Heizer-Signal und dem Detektorsignal des ersten Detektors (130) zu erhalten; und

   wobei die Auswerteanordnung (200) ausgelegt ist, um als Zwischengröße abhängig von der Information (210, 220) über die Amplituden des Detektorsignals und abhängig von der Information (210, 220) über die erste Phasendifferenz ein Kombinationssignal (230) zu bilden, und wobei die Auswerteanordnung (200) ausgelegt ist, um eine Information (240) über eine Gaskonzentration oder eine Information (240) über eine Temperaturleitfähigkeit eines Fluids basierend auf dem Kombinationssignal (230) zu bestimmen.

2. Auswerteanordnung (200) gemäß Anspruch 1, mit einem zweiten Detektor, wobei die zwei Detektoren (130,140) in unterschiedlichen Abständen von dem Heizer (120) angeordnet sind,

wobei die Auswerteanordnung (200) ausgelegt ist, um

- die Information (210) über die Amplitude des Detektorsignals des ersten Detektors (130),
- eine Information (220) über eine Amplitude eines Detektorsignals des zweiten Detektors (140),
- die Information (210) über die erste Phasendifferenz zwischen dem Heizer-Signal und dem Detektorsignal des ersten Detektors (130), und
- eine Information (220) über eine zweite Phasendifferenz zwischen dem Heizer-Signal und dem Detektorsignal des zweiten Detektors (140) zu erhalten; und

wobei die Auswerteanordnung (200) ausgelegt ist, um als Zwischengröße abhängig von der Information (210, 220) über die Amplituden der Detektorsignale und abhängig von der Information (210, 220) über die erste Phasendifferenz und abhängig von der Information über die zweite Phasendifferenz das Kombinationssignal (230) zu bilden, und

wobei die Auswerteanordnung (200) ausgelegt ist, um die Information (240) über die Gaskonzentration oder die Information (240) über die Temperaturleitfähigkeit des Fluids basierend auf dem Kombinationssignal (230) zu bestimmen.

3. Auswerteanordnung (200) gemäß Anspruch 2,

wobei die Auswerteanordnung (200) ausgelegt ist, um eine Information (122) über eine Heizer-Amplitude zu erhalten, und

wobei die Auswerteanordnung (200) ausgelegt ist, um eine Linearkombination der Information (122) über die Heizer-Amplitude, der Information (210, 220) über Amplituden der Detektorsignale, der Information (210) über die erste Phasendifferenz und der Information (220) über die zweite Phasendifferenz zu bilden, um das Kombinationssignal (230) zu bestimmen.

4. Auswerteanordnung (200) gemäß Anspruch 2 oder Anspruch 3,

wobei die Auswerteanordnung (200) ausgelegt ist, um das Kombinationssignal sigX (230) gemäß

$$sigX = sigUss * Ka + sigPhi * Kp$$

zu erhalten,
wobei sigUss eine Amplitudeninformation ist, die von der Information (210) über die Amplitude des Detektorsignals des ersten Detektors (130) und von der Information (220) über die Amplitude des Detektorsignals des zweiten Detektors (140) abhängig ist; und
wobei sigPhi eine Phaseninformation ist, die von der Information (210) über die erste Phasendifferenz und von der Information (220) über die zweite Phasendifferenz abhängig ist; und
wobei Ka und Kp Konstanten sind.

5. Auswerteanordnung (200) gemäß Anspruch 4,

wobei die Auswerteanordnung (200) ausgelegt ist, um die Amplitudeninformation sigUss gemäß

$$sigUss = 2 * Hz.Uss - (D1.Uss + D2.Uss)$$

zu erhalten,
wobei Hz.Uss eine Information (122) über eine Heizer-Amplitude ist,
wobei D1.Uss die Information (210) über die Amplitude des Detektorsignals des ersten Detektors (130) ist, und
wobei D2.Uss die Information (210) über die Amplitude des Detektorsignals des zweiten Detektors (140) ist.

6. Auswerteanordnung (200) gemäß einem der Ansprüche 1 bis 5, wobei die Auswerteanordnung (200) ausgelegt ist, um ein Polynom des Kombinationssignals (230) zu berechnen, um die Information (240) über die Gaskonzentration zu erhalten.

7. Auswerteanordnung (200) gemäß einem der Ansprüche 1 bis 6, wobei die Auswerteanordnung (200) ausgelegt ist, um ein Polynom des Kombinationssignals (230) mit einem Korrekturterm zu multiplizieren, um die Information (240) über die Gaskonzentration zu erhalten, wobei der Korrekturterm von dem Kombinationssignal (230), einer Information über einen Druck und einer Information über eine Temperatur abhängig ist.

8. Auswerteanordnung (200) gemäß einem der Ansprüche 1 bis 7, wobei die Auswerteanordnung (200) ausgelegt ist, um eine Berechnung gemäß

$$C = pol(sigX) \cdot \left(1 - \left[\frac{f(p)}{sigX - const1}\right] \cdot \left(1 - \left[\frac{f(T)}{p - const2}\right]\right)\right)$$

durchzuführen, um die Information C (240) über die Gaskonzentration zu erhalten,

wobei sigX das Kombinationssignal (230) ist,
wobei pol(sigX) ein Polynom des Kombinationssignal sigX (230) ist;
wobei f(p) eine Funktion des Drucks p ist;
wobei const1 eine erste Kontante ist;
wobei f(T) eine Funktion der Temperatur T ist; und
wobei const2 eine zweite Konstante ist.

9. Auswerteanordnung (200) gemäß einem der Ansprüche 1 bis 8, wobei die Auswerteanordnung (200) ausgelegt ist, um eine Berechnung gemäß

$$C[vol\%] = A.y(sigX) \cdot \left(1 - \left[\frac{B.y(p) - B.ref}{sigX - B.ref}\right] \cdot \left(1 - \left[\frac{C.y(T) - C.ref}{p - C.ref}\right]\right)\right)$$

durchzuführen, um die Information C (240) über die Gaskonzentration zu erhalten,

wobei sigX das Kombinationssignal (230) ist,
wobei A.y(sigX) ein Polynom des Kombinationssignal sigX (230) ist;
wobei B.y(p) eine Funktion des Drucks p ist;
wobei B.ref eine Konstante ist;
wobei C.y(T) eine Funktion der Temperatur T ist; und
wobei C.ref eine zweite Konstante ist.

10. Auswerteanordnung (200) gemäß einem der Ansprüche 1 bis 9, wobei die Auswerteanordnung (200) ausgelegt ist, um einen Druck und/oder eine Temperatur in einer Umgebung des thermischen Gassensors (100) bei der Bestimmung der Information (240) über die Gaskonzentration zu berücksichtigen.

11. Auswerteanordnung (200) gemäß einem der Ansprüche 1 bis 10, wobei die Auswerteanordnung (200) ausgelegt ist, um bei der Bestimmung der Information (240) über die Gaskonzentration als Eingangsgrößen einer Drift-Korrektur das Kombinationssignal (230), eine Information über die Temperatur in einer Umgebung des thermischen Gassensors (100) und eine Information über einen Druck in einer Umgebung des thermischen Gassensors (100) zu verwenden, und um die Information (240) über die Gaskonzentration als Ergebnis der Drift-Korrektur zu erhalten.

12. Auswerteanordnung (200) gemäß einem der Ansprüche 1 bis 11,

wobei die Auswerteanordnung (200) ausgelegt ist, um das Kombinationssignal (230) basierend auf einem Quotienten zwischen einer Amplitudeninformation (210, 220), die von der Information (210) über die Amplitude des Detektorsignals zumindest des ersten Detektors (130) abhängig ist; und einer Phaseninformation (210, 220, 400, 410), die von der Information (210) über die erste Phasendifferenz abhängig ist, zu erhalten; und
wobei die Auswerteanordnung (200) ausgelegt ist, um abhängig von dem Kombinationssignal (230) die Information (240) über eine Konzentration einen Gases zu bestimmen.

13. Auswerteanordnung (200) gemäß einem der Ansprüche 1 bis 12, wobei die Auswerteanordnung (200) ausgelegt ist, um das Kombinationssignal sigV (230) gemäß

$$sigV = sigUss * Kav / (sigPhi * Kpv)$$

zu erhalten,

wobei sigUss eine Amplitudeninformation (210, 220) ist, die von der Information (210) über die Amplitude des Detektorsignals des ersten Detektors (130) abhängig ist; und
wobei sigPhi eine Phaseninformation (210, 220, 400, 410) ist, die von der Information (210) über die erste Phasendifferenz abhängig ist; und
wobei Kav und Kpv Konstanten sind.

14. Auswerteanordnung (200) gemäß einem der Ansprüche 1 bis 13,
wobei die Auswerteanordnung (200) ausgelegt ist, um eine Information darüber zu erhalten, wieviel Wärme von dem Heizer während einer Heizperiode (302) abgeführt wird, und um abhängig von der Information darüber, wieviel Energie von dem Heizer während der Heizperiode (302) abgeführt wird, die Information (240) über eine Konzentration eines Gases zu bestimmen.

15. Auswerteanordnung (200) gemäß Anspruch 14, wobei die Auswerteanordnung (200) ausgelegt ist, um die Information, wieviel Wärme von dem Heizer während einer Heizperiode (302) abgeführt wird, basierend auf einer Messung eines Stromflusses (310) durch den Heizer bei vorgegebener Heizspannung (300) zu erhalten.

16. Auswerteanordnung (200) gemäß Anspruch 15, wobei die Auswerteanordnung (200) ausgelegt ist, um den Stromfluss (310) kurz nach einem Einschalten der vorgegebenen Heizspannung (300) und kurz vor einem Ausschalten der vorgegebenen Heizspannung (300) zu erhalten.

17. Verfahren zur Auswertung von Signalen eines thermischen Gassensors mit zumindest einem Heizer und zumindest einem Detektor,

wobei das Verfahren ein Erhalten

- einer Information über eine Amplitude eines Detektorsignals eines ersten Detektors und
- einer Information über eine erste Phasendifferenz zwischen einem Heizer-Signal und dem Detektorsignal des ersten Detektors

umfasst; und
wobei als Zwischengröße abhängig von der Information über die Amplituden des Detektorsignals und abhängig von der Information über die erste Phasendifferenz ein Kombinationssignal gebildet wird, und
wobei eine Information über eine Gaskonzentration oder eine Information über eine Temperaturleitfähigkeit eines Fluids basierend auf dem Kombinationssignal bestimmt wird.

18. Verfahren nach Anspruch 17 zur Auswertung von Signalen eines thermischen Gassensors mit zumindest einem Heizer und zwei in unterschiedlichen Abständen von dem Heizer angeordneten Detektoren,

wobei das Verfahren ein Erhalten

- der Information über die Amplitude des Detektorsignals des ersten Detektors,
- einer Information über eine Amplitude eines Detektorsignals eines zweiten Detektors,
- der Information über die erste Phasendifferenz zwischen dem Heizer-Signal und dem Detektorsignal des ersten Detektors, und
- einer Information über eine zweite Phasendifferenz zwischen dem Heizer-Signal und dem Detektorsignal des zweiten Detektors

umfasst; und
wobei als Zwischengröße abhängig von der Information über die Amplituden der Detektorsignale und abhängig von der Information über die erste Phasendifferenz und abhängig von der Information über die zweite Phasendifferenz das Kombinationssignal gebildet wird, und

wobei die Information über die Gaskonzentration oder die Information über die Temperaturleitfähigkeit des Fluids basierend auf dem Kombinationssignal bestimmt wird.

19. Ein Computerprogramm mit einem Programmcode zur Durchführung des Verfahrens nach Anspruch 17 oder Anspruch 18, wenn das Programm auf einem Computer abläuft.

**Claims**

1. Evaluation arrangement (200) for a thermal gas sensor (100) having at least one heater (120) and at least one detector (130, 140),

   wherein the evaluation arrangement (200) is configured to obtain

   - information (210) about an amplitude of a detector signal of a first detector (130), and
   - information (210) about a first phase difference between a heater signal and the detector signal of the first detector (130); and

   wherein the evaluation arrangement (200) is configured to form a combination signal (230) as an intermediate quantity in dependence on the information (210, 220) about the amplitudes of the detector signal and in dependence on the information (210, 220) about the first phase difference, and
   wherein the evaluation arrangement (200) is configured to determine information (240) about a gas concentration or information (240) about a thermal diffusivity of a fluid on the basis of the combination signal (230).

2. Evaluation arrangement (200) as claimed in claim 1, comprising a second detector, the two detectors (130, 140) being arranged at different distances from the heater (120),

   wherein the evaluation arrangement (200) is configured to obtain

   - the information (210) about the amplitude of the detector signal of the first detector (130),
   - information (220) about an amplitude of a detector signal of the second detector (140),
   - the information (210) about the first phase difference between the heater signal and the detector signal of the first detector (130), and
   - information (220) about a second phase difference between the heater signal and the detector signal of the second detector (140); and

   wherein the evaluation arrangement (200) is configured to form the combination signal (230) as an intermediate quantity in dependence on the information (210, 220) about the amplitudes of the detector signals and in dependence on the information (210, 220) about the first phase difference and in dependence on the information about the second phase difference, and
   wherein the evaluation arrangement (200) is configured to determine the information (240) about the gas concentration or the information (240) about the thermal diffusivity of the fluid on the basis of the combination signal (230).

3. Evaluation arrangement (200) as claimed in claim 2,

   wherein the evaluation arrangement (200) is configured to obtain information (122) about a heater amplitude, and
   wherein the evaluation arrangement (200) is configured to form a linear combination of the information (122) about the heater amplitude, of the information (210, 220) about the amplitudes of the detector signals, of the information (210) about the first phase difference, and of the information (220) about the second phase difference in order to determine the combination signal (230).

4. Evaluation arrangement (200) as claimed in claim 2 or claim 3,

   wherein the evaluation arrangement (200) is configured to obtain the combination signal sigX (230) according to

   $$sigX = sigUss * Ka + sigPhi * Kp$$

wherein sigUss is amplitude information that depends on the information (210) about the amplitude of the detector signal of the first detector (130) and on the information (220) about the amplitude of the detector signal of the second detector (140); and

wherein sigPhi is phase information that depends on the information (210) about the first phase difference and on the information (220) about the second phase difference; and

wherein Ka and Kp are constants.

5. Evaluation arrangement (200) as claimed in claim 4,

wherein the evaluation arrangement (200) is configured to obtain the amplitude information sigUss according to

$$sigUss = 2 * Hz.Uss - (D1.Uss + D2.Uss)$$

wherein Hz.Uss is information (122) about a heater amplitude,
wherein D1.Uss is the information (210) about the amplitude of the detector signal of the first detector (130), and
wherein D2.Uss is the information (210) about the amplitude of the detector signal of the second detector (140).

6. Evaluation arrangement (200) as claimed in any of claims 1 to 5, wherein the evaluation arrangement (200) is configured to calculate a polynomial of the combination signal (230) in order to obtain the information (240) about the gas concentration.

7. Evaluation arrangement (200) as claimed in any of claims 1 to 6, wherein the evaluation arrangement (200) is configured to multiply a polynomial of the combination signal (230) by a correction term in order to obtain the information (240) about the gas concentration, wherein the correction term depends on the combination signal (230), on information about a pressure, and on information about a temperature.

8. Evaluation arrangement (200) as claimed in any of claims 1 to 7, wherein the evaluation arrangement (200) is configured to perform a calculation according to

$$C = pol(sigX) \cdot \left(1 - \left[\frac{f(p)}{sigX - const1}\right] \cdot \left(1 - \left[\frac{f(T)}{p - const2}\right]\right)\right)$$

in order to obtain the information C (240) about the gas concentration,
wherein sigX is the combination signal (230),
wherein pol(sigX) is a polynomial of the combination signal sigX (230);
wherein f(p) is a function of the pressure p;
wherein const1 is a first constant;
wherein f(T) is a function of the temperature T; and
wherein const2 is a second constant.

9. Evaluation arrangement (200) as claimed in any of claims 1 to 8, wherein the evaluation arrangement (200) is configured to perform a calculation according to

$$C[vol\%] = A.y(sigX) \cdot \left(1 - \left[\frac{B.y(p) - B.ref}{sigX - B.ref}\right] \cdot \left(1 - \left[\frac{C.y(T) - C.ref}{p - C.ref}\right]\right)\right)$$

in order to obtain the information C (240) about the gas concentration,
wherein sigX is the combination signal (230),
wherein A.y(sigX) is a polynomial of the combination signal sigX (230);
wherein B.y(p) is a function of the pressure p;
wherein B.ref is a constant;
wherein C.y(T) is a function of the temperature T; and
wherein C.ref is a second constant.

10. Evaluation arrangement (200) as claimed in any of claims 1 to 9, wherein the evaluation arrangement (200) is configured to consider a pressure and/or a temperature in a surrounding area of the gas sensor (100) when determining the information (240) about the gas concentration.

11. Evaluation arrangement (200) as claimed in any of claims 1 to 10, wherein the evaluation arrangement (200) is configured, when determining the information (240) about the gas concentration, to use as input quantities of a drift correction the combination signal (230), information about the temperature in a surrounding area of the thermal gas sensor (100), and information about a pressure in a surrounding area of the thermal gas sensor (100), and to obtain the information (240) about the gas concentration as a result of the drift correction.

12. Evaluation arrangement (200) as claimed in any of claims 1 to 11,

wherein the evaluation arrangement (200) is configured to obtain the combination signal (230) on the basis of a quotient between amplitude information (210, 220) that depends on the information (210) about the amplitude of the detector signal at least of the first detector (130); and phase information (210, 220, 400, 410) that depends on the information (210) about the first phase difference; and
wherein the evaluation arrangement (200) is configured to determine the information (240) about a concentration of a gas in dependence on the combination signal (230).

13. Evaluation arrangement (200) as claimed in any of claims 1 to 12, wherein the evaluation arrangement (200) is configured to obtain the combination signal sigV (230) according to

$$sigV=sigUss*Kav/(sigPhi*Kpv),$$

wherein sigUss is amplitude information (210, 220) that depends on the information (210) about the amplitude of the detector signal of the first detector (130); and
wherein sigPhi is phase information (210, 220, 400, 410) that depends on the information (210) about the first phase difference; and
wherein Kav and Kpv are constants.

14. Evaluation arrangement (200) as claimed in any of claims 1 to 13,
wherein the evaluation arrangement (200) is configured to obtain information as to how much heat is dissipated by the heater during a heating period (302), and to determine, in dependence on the information as to how much energy is dissipated by the heater during the heating period (302), the information (240) about a concentration of a gas.

15. Evaluation arrangement (200) as claimed in claim 14, wherein the evaluation arrangement (200) is configured to obtain the information as to how much heat is dissipated by the heater during a heating period (302) on the basis of a measurement of a current flow (310) through the heater at a specified heating voltage (300).

16. Evaluation arrangement (200) as claimed in claim 15, wherein the evaluation arrangement (200) is configured to obtain the current flow (310) shortly after switching on the specified heating voltage (300) and shortly after switching off the specified heating voltage (300).

17. Method of evaluating signals of a thermal gas sensor having at least one heater and at least one detector,

wherein the method includes obtaining

- information about an amplitude of a detector signal of the first detector, and
- information about a first phase difference between a heater signal and the detector signal of the first detector; and

wherein a combination signal is formed as an intermediate quantity in dependence on the information about the amplitudes of the detector signal and in dependence on the information about the first phase difference, and
wherein information about a gas concentration or information about a thermal diffusivity of a fluid is determined on the basis of the combination signal.

18. Method as claimed in claim 17 of evaluating signals of a thermal gas sensor having at least one heater and two detectors arranged at different distances from the heater,

wherein the method includes obtaining

- the information about the amplitude of the detector signal of the first detector,
- information about an amplitude of a detector signal of a second detector,
- the information about the first phase difference between the heater signal and the detector signal of the first detector, and
- information about a second phase difference between the heater signal and the detector signal of the second detector; and

wherein a combination signal is formed as an intermediate quantity in dependence on the information about the amplitudes of the detector signals and in dependence on the information about the first phase difference and in dependence on the information about the second phase difference, and
wherein the information about the gas concentration or the information about the thermal diffusivity of the fluid is determined on the basis of the combination signal.

19. Computer program having a program code for performing the method as claimed in claim 17 or claim 18 when the program runs on a computer.

**Revendications**

1. Aménagement d'évaluation (200) pour un capteur de gaz thermique (100) avec au moins un élément chauffant (120) et au moins un détecteur (130, 140),

dans lequel l'aménagement d'évaluation (200) est conçu pour obtenir

- une information (210) sur une amplitude d'un signal de détecteur d'un premier détecteur (130), et
- une information (210) sur une première différence de phase entre un signal de l'élément chauffant et le signal de détecteur du premier détecteur (130); et

dans lequel l'aménagement d'évaluation (200) est conçu pour former un signal de combinaison (230) comme grandeur intermédiaire en fonction de l'information (210, 220) sur les amplitudes du signal de détecteur et en fonction de l'information (210, 220) sur la première différence de phase, et
dans lequel l'aménagement d'évaluation (200) est conçu pour déterminer une information (240) sur une concentration de gaz ou une information (240) sur une conductivité thermique d'un fluide sur base du signal de combinaison (230).

2. Aménagement d'évaluation (200) selon la revendication 1, avec un deuxième détecteur, dans lequel les deux détecteurs (130, 140) sont disposés à des distances différentes de l'élément chauffant (120),

dans lequel l'aménagement d'évaluation (200) est conçu pour obtenir

- l'information (210) sur l'amplitude du signal de détecteur du premier détecteur (130),
- une information (220) sur une amplitude d'un signal de détecteur du deuxième détecteur (140),
- l'information (210) sur la première différence de phase entre le signal de l'élément chauffant et le signal de détecteur du premier détecteur (130), et
- une information (220) sur une deuxième différence de phase entre le signal de l'élément chauffant et le signal de détecteur du deuxième détecteur (140); et

dans lequel l'aménagement d'évaluation (200) est conçu pour former le signal de combinaison (230) comme grandeur intermédiaire en fonction de l'information (210, 220) sur les amplitudes des signaux de détecteur et en fonction de l'information (210, 220) sur la première différence de phase et en fonction de l'information sur la deuxième différence de phase, et
dans lequel le l'aménagement d'évaluation (200) est conçu pour déterminer l'information (240) sur la concentration de gaz ou l'information (240) sur la conductivité thermique du fluide sur base du signal de combinaison (230).

3. Aménagement d'évaluation (200) selon la revendication 2,

   dans lequel l'aménagement d'évaluation (200) est conçu pour recevoir une information (122) sur une amplitude de l'élément chauffant, et

   dans lequel l'aménagement d'évaluation (200) est conçu pour former une combinaison linéaire de l'information (122) sur l'amplitude de l'élément chauffant, de l'information (210, 220) sur les amplitudes des signaux de détecteur, de l'information (210) sur la première différence de phase et de l'information (220) sur la deuxième différence de phase, pour déterminer le signal de combinaison (230).

4. Aménagement d'évaluation (200) selon la revendication 2 ou la revendication 3,

   dans lequel l'aménagement d'évaluation (200) est conçu pour obtenir le signal de combinaison sigX (230) selon

$$sigX = sigUss * Ka + sigPhi * Kp,$$

   où sigUss est une information d'amplitude qui dépend de l'information (210) sur l'amplitude du signal de détecteur du premier détecteur (130) et de l'information (220) sur l'amplitude du signal de détecteur du deuxième détecteur (140); et

   où sigPhi est une information de phase qui dépend de l'information sur la première différence de phase (210) et de l'information (220) sur la deuxième différence de phase; et

   où Ka et Kp sont des constantes.

5. Aménagement d'évaluation (200) selon la revendication 4,

   dans lequel l'aménagement d'évaluation (200) est conçu pour obtenir l'information d'amplitude sigUss selon

$$sigUss = 2 * Hz.Uss - (D1.Uss + D2.Uss),$$

   où Hz.Uss est une information (122) sur une amplitude de l'élément chauffant,

   où D1.Uss est l'information (210) sur l'amplitude du signal de détecteur du premier détecteur (130), et

   où D2.Uss est l'information (210) sur l'amplitude du signal de détecteur du deuxième détecteur (140).

6. Aménagement d'évaluation (200) selon l'une des revendications 1 à 5, dans lequel l'aménagement d'évaluation (200) est conçu pour calculer un polynôme du signal de combinaison (230) pour obtenir l'information (240) sur la concentration de gaz.

7. Aménagement d'évaluation (200) selon l'une des revendications 1 à 6, dans lequel l'aménagement d'évaluation (200) est conçu pour multiplier un polynôme du signal de combinaison (230) par un terme de correction pour obtenir l'information (240) sur la concentration de gaz, dans lequel le terme de correction dépend du signal de combinaison (230), d'une information sur une pression et d'une information sur une température.

8. Aménagement d'évaluation (200) selon l'une des revendications 1 à 7, dans lequel l'aménagement d'évaluation (200) est conçu pour effectuer un calcul selon

$$C = pol(sigX) \cdot \left(1 - \left[\frac{f(p)}{sigX - const1}\right] \cdot \left(1 - \left[\frac{f(T)}{p - const2}\right]\right)\right)$$

   pour obtenir l'information C (240) sur la concentration de gaz,

   où sigX est le signal de combinaison (230),

   où pol(sigX) est un polynôme du signal de combinaison sigX (230);

   où f(p) est une fonction de la pression p;

   où const1 est une première constante;

   où f(T) est une fonction de la température T; et

   où const2 est une deuxième constante.

**9.** Aménagement d'évaluation (200) selon l'une des revendications 1 à 8, dans lequel l'aménagement d'évaluation (200) est conçu pour effectuer un calcul selon

$$C[vol\%] = A.\,y(sigX) \cdot \left(1 - \left[\frac{B.\,y(p) - B.ref}{sigX - B.ref}\right] \cdot \left(1 - \left[\frac{C.\,y(T) - C.ref}{p - C.ref}\right]\right)\right)$$

pour obtenir l'information C (240) sur la concentration de gaz,
où sigX est le signal de combinaison (230),
où A.y(sigX) est un polynôme du signal de combinaison sigX (230);
où B.y(p) est une fonction de la pression p;
où B.ref est une constante;
où C.y(T) est une fonction de la température T; et
où C.ref est une deuxième constante.

**10.** Aménagement d'évaluation (200) selon l'une des revendications 1 à 9, dans lequel l'aménagement d'évaluation (200) est conçu pour tenir compte d'une pression et/ou d'une température dans un environnement du capteur de gaz thermique (100) lors de la détermination de l'information (240) sur la concentration de gaz.

**11.** Aménagement d'évaluation (200) selon l'une des revendications 1 à 10, dans lequel l'aménagement d'évaluation (200) est conçu pour utiliser, lors de la détermination de l'information (240) sur la concentration de gaz comme grandeurs d'entrée d'une correction de dérive, le signal de combinaison (230), une information sur la température dans un environnement du capteur de gaz thermique (100) et une information sur une pression dans un environnement du capteur de gaz thermique (100), et pour obtenir l'information (240) sur la concentration de gaz comme résultat de la correction de dérive.

**12.** Aménagement d'évaluation (200) selon l'une des revendications 1 à 11,

dans lequel l'aménagement d'évaluation (200) est conçu pour obtenir le signal de combinaison (230) sur base d'un quotient entre une information d'amplitude (210, 220) qui dépend de l'information (210) sur l'amplitude du signal de détecteur au moins du premier détecteur (130), et d'une information de phase (210, 220, 400, 410) qui dépend de l'information (210) sur la première différence de phase; et
dans lequel l'aménagement d'évaluation (200) est conçu pour déterminer, en fonction du signal de combinaison (230), l'information (240) sur une concentration d'un gaz.

**13.** Aménagement d'évaluation (200) selon l'une des revendications 1 à 12, dans lequel l'aménagement d'évaluation (200) est conçu pour obtenir le signal de combinaison sigV (230) selon

$$sigV=sigUss*Kav/(sigPhi*Kpv),$$

où sigUss est une information d'amplitude (210, 220) qui dépend de l'information (210) sur l'amplitude du signal de détecteur du premier détecteur (130); et
où sigPhi est une information de phase (210, 220, 400, 410) qui dépend de l'information sur la première différence de phase (210); et
où Kav et Kpv sont des constantes.

**14.** Aménagement d'évaluation (200) selon l'une des revendications 1 à 13,
dans lequel l'aménagement d'évaluation (200) est conçu pour obtenir une information sur la quantité de chaleur qui est dissipée par l'élément chauffant pendant une période de chauffage (302), et pour déterminer, en fonction de l'information sur la quantité d'énergie qui est dissipée par l'élément chauffant pendant la période de chauffage (302), l'information (240) sur une concentration d'un gaz.

**15.** Aménagement d'évaluation (200) selon la revendication 14, dans lequel l'aménagement d'évaluation (200) est conçu pour obtenir l'information sur la quantité de chaleur qui est dissipée par l'élément chauffant pendant une période de chauffage (302) sur base d'une mesure d'un flux de courant (310) à travers l'élément chauffant à une tension de chauffage prédéterminée (300).

**16.** Aménagement d'évaluation (200) selon la revendication 15, dans lequel l'aménagement d'évaluation (200) est conçu pour obtenir le flux de courant (310) peu après l'activation de la tension de chauffage prédéterminée (300) et peu avant la désactivation de la tension de chauffage prédéterminée (300).

**17.** Procédé d'évaluation des signaux d'un capteur de gaz thermique avec au moins un élément chauffant et au moins un détecteur,

dans lequel le procédé comporte le fait d'obtenir

- une information sur une amplitude d'un signal de détecteur d'un premier détecteur, et
- une information sur une première différence de phase entre un signal de l'élément chauffant et le signal de détecteur du premier détecteur; et

dans lequel est formé un signal de combinaison comme grandeur intermédiaire en fonction de l'information sur les amplitudes du signal de détecteur et en fonction de l'information sur la première différence de phase, et dans lequel une information sur une concentration de gaz ou une information sur une conductivité thermique d'un fluide est déterminée sur base du signal de combinaison.

**18.** Procédé selon la revendication 17 pour évaluer les signaux d'un capteur de gaz thermique avec au moins un élément chauffant et deux détecteurs disposés à des distances différentes de l'élément chauffant,

dans lequel le procédé comporte le fait d'obtenir

- l'information sur l'amplitude du signal de détecteur du premier détecteur,
- une information sur une amplitude d'un signal de détecteur d'un deuxième détecteur,
- l'information sur la première différence de phase entre le signal de l'élément chauffant et le signal de détecteur du premier détecteur, et
- une information sur une deuxième différence de phase entre le signal de l'élément chauffant et le signal de détecteur du deuxième détecteur; et

dans lequel est formé le signal de combinaison comme grandeur intermédiaire en fonction de l'information sur les amplitudes des signaux de détecteur et en fonction de l'information sur la première différence de phase et en fonction de l'information sur la deuxième différence de phase, et dans lequel l'information sur la concentration de gaz ou l'information sur la conductivité thermique du fluide est déterminée sur base du signal de combinaison.

**19.** Programme d'ordinateur avec un code de programme pour mettre en œuvre le procédé selon la revendication 17 ou la revendication 18 lorsque le programme est exécuté sur un ordinateur.

Fig. 1a

Fig. 1b

EP 3 735 580 B1

$180_1$  $180_2$

100  130  120  140

252

210  220

250  Regelung einer Heizleistung

122

240  Gaskonzentration + Temperaturleitfähigkeit

200

Fig. 1c

$180_1$    $180_2$

100    130    120    140

210    220

200

| periodisches Signal | 260 |

Abtastung zu 3 Zeitpunkten — 270

Ermittlung neuer Zeitpunkte

290

Überprüfung — 280

Gaskonzentration + Temperaturleitfähigkeit

122    240

Fig. 1d

Fig. 2a

EP 3 735 580 B1

Fig. 2b

EP 3 735 580 B1

150
156
154
152
158
160/162
110
120/130/140
170/172

00075851          10 µm          HSG-IMIT

Fig. 3

HSG-6-125-100

Fig. 4

EP 3 735 580 B1

Fig. 5

EP 3 735 580 B1

Fig. 6a

Fig. 6b

Fig. 6c

Fig. 7

Fig. 8

Fig. 9

CO2_Heizer

Stromrichtung →

Spannungsvorgabe

U402
INA199A1RSWT
Digi-Key
296-27330-1-ND

R421
1 k

Strommessung

CO2_Hz_Strom

C412
10 nF

AGND

gain=50

8

ref

AGND

AGND

A3V

6

V−
GND

6

AGND

5

4

2

3

R420
10

−

+

T402A
MAX4163EUA-

A6V

8

+

A

−

2

3

4

AGND

A6V

Heizer: soll 600 Ohm =: 1580 Ohm gem

R419
0,5 V
100 k

R418
100 k

7

−

B

+

T402B
MAX4163EUA
Dip-Key
MAX4163EUA

6

5

0,25 V

R423
10 meg

C411
100 nF

AGND

AGND

R422
8,2k

A3V

4

COM

5

V+

U403

IN

NO

NC

GND

FSA4159P6X
Farnell
1495-462

AGND

6

1

3

2

CO2_Hz_dig

CO2_an_up

CO2_an_low

**Fig. 10**

CO2_S1_an

C402
10 nF
AGND

R404
1 k

S1 Amp

C400
1 nF

R403
330 k

R401
1 k

Refi 25V

R406
56 k

CO2_S1_Wm

CO2_Sensor 1

R408
8,2 k

Refi 5V

C405
10 nF
AGND

A3V
8 +
A
2 −
3 +
4 −
1
AGND

U400A
OPA2333AIDRBT
Digi-Key
296-19544-1-ND

CO2_S1_dig

C406
10 nF
AGND

R410
100

INA−
INA+
A
OUTA
Vdd
Vss
1
2
3
4
3

Refi 5V

AGND

U401A
MCP6542-E M5
Farnell
1852110

R414

R412
100 k

R416
22 k

C408
220 nF

S1 Amp

Refi 25V

Fig. 11

Heizer Regelung

S1-Amplituden-Regler

Nachführung der Abtastzeitpunkte:
entweder durch Erfassung der Phasenlage der Schmitt-Trigger
oder durch Abweichung von (Umax + Umin)/2! = U0

Nachführung der DC-Arbeitspunkte
der beiden Sensor-Verstärker

Fig. 12

START:
ADC Werte mit aktuellen
Einstellungen messen

oberer/unterer Scheitelpunkt
und Nulldurchgang
(3 ADC Messungen)

S1 und S2
Prüfung Messbereich
AD-max.< 95% ADC
AD-min.> 5 %

nein

ja

Nachstellung DC-Arbeitspunkt
des Verstärkers (DAC)

S1 und S2 Prüfung
AD-min.> 5 % ADC

nein

ja

Amplitudenregler

S1 Prüfung
AD-max.< 95 % ADC

nein

ja

Nachführung der Abtastzeitpunkte

Fig. 13a

START:
Nachführung der Abtastzeitpunkte

oberer/unterer Scheitelpunkt und
Nulldurchgang (3 ADC Messungen)

zwei Wege

AD-Wandlung

(Umax+Umin)/2 = U0

Komparator Signal

Umschaltpunkt Zeit 90°,
180° und 270° addiert

Toleranz 0,625°
(14,47 µs)

beide Regler
sind stabil (DC-Arbeitspunkt
und Phase)

nein → Nachstellung DC-Arbeitspunkt
des Verstärkers (DAC)

ja

Amplituden-Regler:
Heizer Regelung bis gewünschte
Amplitude erreicht ist

Detektor S1:
Prüfung Messbereich
AD-max.< 95% ADC
AD-min.> 5 %

nein

ja

Auswertung:
Amplitude und Phase

Fig. 13b

Fig. 14

Fig. 15

Fig. 16

Fig. 17a

Fig. 17b

Fig. 18

Fig. 19

EP 3 735 580 B1

Fig. 20

Fig. 21

Results.Level02.Graph

Fig. 22

EP 3 735 580 B1

Results.Level03.Graph

Fig. 23

Fig. 24

Messgröße — Teilsignale — Faktoren — Sensor Signal — Drift-Korrekturen im 4 dimensionalen Vektorfeld

Amplitude D1.Uss und D2.Uss → z.B. relativer Betrag gegenüber Heizer → Ka

Phasenverschiebung D1.Phi und D2.Phi → Addition → Kp

+ → Signal sigX → R_B → R_C (Druck, Temperatur); R_A → Gas Konzentration

Legende: (R_X) = Regressionsstufe X

Fig. 25

Fig. 26

EP 3 735 580 B1

Fig. 27

Tek                          ⊛ Stop                    M Pos: 2,000 ms                    300

312                                                                                              CURSOR

320                                                                                              Typ
                                                                                                 Amplitude

314                                                                                              Quelle
                                                                                                 CH3

310                                                                                              ΔV 210 mV

4→
2→                                                                                               Cursor 1
                                                                                                 2,93 V
3
1→                                                                                               Cursor 2
                                                                                                 2,72 V

CH1 500 mVBw        CH2 500 mVBw   M 500µs                      CH1 1,48 V
CH3 50,0 mVBw       CH4 500 mVBw   15-Dez-18  12:25             120,019 Hz

                              |←——————————— 302 ———————————→|

EP 3 735 580 B1

Fig. 28

Fig. 29

EP 3 735 580 B1

Fig. 30

Fig. 31

**IN DER BESCHREIBUNG AUFGEFÜHRTE DOKUMENTE**

**In der Beschreibung aufgeführte Patentdokumente**

- DE 102011075519 A1
- DE 1020131022309
- DE 102015107584
- DE 102008047511 A1

**In der Beschreibung aufgeführte Nicht-Patentliteratur**

- **H. D. BAEHR ; K. STEPHAN.** *Wärme- und Stoffübertragung,* 2008, vol. 6
- **I. SIMON ; M. ARNDT.** Thermal and gas-sensing properties of a micromachined thermal conductivity sensor for the detection of hydrogen in automotive applications. *Sensors and Actuators A: Physical,* April 2002, vol. 97-98
- **J. J. VAN BAAR ; R. J. WIEGERINK ; T. S. J. LAMMERINK ; G. J. M. KRIJNEN ; M. ELWENSPOEK.** Micromachined structures for thermal measurements of fluid and flow parameters. *Journal of Micromechanics and Microengineering,* Juli 2001, vol. 11 (4), 311-318
- **H. GRIENAUER.** Gasanalyse mit thermisch modulierten Wärmeleitfähigkeits-Sensoren mit Fourier-Analyse des Messsignals. *GMA/ITG-Fachtagung Sensoren und Messsysteme,* 22. Mai 2012, vol. 16, ISBN 978-3-9813484-0-8, 54-61
- **A. AL-SALAYMEH ; M. ALHUSEIN ; F. DURST.** Development of a twowire thermal flow sensor for industrial applications. *Journal of Quality in Maintenance Engineering,* 2003, vol. 9 (2), 113-131
- **KLICHE ; BILLAT ; MESSNER ; ZENGERLE.** Sensorsystem zur thermischen Gasanalyse von Gasgemischen, Konferenzbeitrag. *Proc. of Mikrosystemtechnik Kongress 2011,* 10. Oktober 2011, ISBN 978-3-8007-3367-5, 875-878
- **K KLICHE ; S BILLAT ; F HEDRICH ; C ZIEGLER ; R ZENGERLE.** Sensor for gas analysis based on thermal conductivity, specific heat capacity and thermal diffusivity. *Micro Electro Mechanical Systems (MEMS), 2011 IEEE 24th International Conference on Micro Electro Mechanical Systems*
- **K KLICHE ; G KATTINGER ; S BILLAT ; L SHEN ; S MESSNER ; R ZENGERLE.** Sensor for thermal gas analysis based on micromachined siliconmicrowires. *IEEE Sensors Journal,* 2013, vol. 13 (7), 2626-2635